# EUROPEAN PATENT APPLICATION

(11) **EP 3 950 006 A1**
(43) Date of publication of application: **09.02.2022**
(21) Application number: 20781989.7
(22) Date of filing: 27.03.2020
(51) Int. Cl.: A61K 48/00, A61P 21/00, A61P 43/00, C12N 15/113, A61K 31/7088

(54) **COMPOUND, METHOD AND PHARMACEUTICAL COMPOSITION FOR DUX4 EXPRESSION ADJUSTMENT**

(30) Priority: 29.03.2019 JP 2019067914
(71) Applicant: Mitsubishi Tanabe Pharma Corporation, Osaka-shi, Osaka 541-8505 (JP)
(72) Inventor: KUMAGAI, Shinji, Osaka-shi, Osaka 541-8505 (JP); YASHIRO, Takashi, Osaka-shi, Osaka 541-8505 (JP); ARAKI, Tomo, Osaka-shi, Osaka 541-8505 (JP); KANAGAWA, Takayuki, Osaka-shi, Osaka 541-8505 (JP); OKAGAKI, Chieko, Osaka-shi, Osaka 541-8505 (JP); FURUKAWA, Hiroyuki, Osaka-shi, Osaka 541-8505 (JP)
(74) Representative: Hoffmann Eitle
(86) International application number: PCT/JP2020/014307
(87) International publication number: WO 2020/203880

(57) **Abstract**

An object of the present invention is to provide a compound, a method and a pharmaceutical composition for normalizing double homeobox 4 (DUX4) of an individual in which the DUX4 gene has abnormally expressed. Provided is a modified oligonucleotide consisting of 12 - 30 residues. The modified oligonucleotide includes a nucleobase sequence that includes at least 8 contiguous nucleobase sequences and is complementary to an equal length portion at positions 126 - 147, 232 - 248, 1306 - 1325 or 1472 - 1495 from a 5' end of a nucleobase of a mature mRNA of DUX4 of SEQ ID NO: 1. The nucleobase sequence of the modified oligonucleotide has at least 90% complementarity to the equal length portion in the nucleobase sequence of the mature mRNA of DUX4 of SEQ ID NO: 1.

SEQUENCE LISTING

<110> Mitsubishi Tanabe Pharma Corporation

<120> Compounds for modulating expression of DUX4, Methods and
Pharmaceutical Composition thereof

<130> A19030-19510

<150> JP2019-67914
<151> 2019-03-29

<160> 109

<170> PatentIn version 3.5

<210> 1
<211> 1574
<212> DNA
<213> Homo sapiens

<400> 1
atggccctcc cgacaccctc ggacagcacc ctccccgcgg aagcccgggg acgaggacgg      60
cgacggagac tcgtttggac cccgagccaa agcgaggccc tgcgagcctg ctttgagcgg     120
aacccgtacc cgggcatcgc caccagagaa cggctggccc aggccatcgg cattccggag     180
cccagggtcc agatttggtt tcagaatgag aggtcacgcc agctgaggca gcaccggcgg     240
gaatctcggc cctggcccgg gagacgcggc ccgccagaag gccggcgaaa gcggaccgcc     300
gtcaccggat cccagaccgc cctgctcctc cgagcctttg agaaggatcg ctttccaggc     360
atcgccgccc gggaggagct ggccagagag acgggcctcc cggagtccag gattcagatc     420
tggtttcaga atcgaagggc caggcacccg ggacagggtg gcagggcgcc cgcgcaggca     480
ggcggcctgt gcagcgcggc ccccggcggg ggtcaccctg ctccctcgtg ggtcgccttc     540
gcccacaccg gcgcgtgggg aacggggctt cccgcacccc acgtgccctg cgcgcctggg     600
gctctcccac agggggcttt cgtgagccag gcagcgaggg ccgcccccgc gctgcagccc     660
agccaggccg cgccggcaga ggggatctcc caacctgccc cggcgcgcgg ggatttcgcc     720
tacgccgccc cggctcctcc ggacggggcg ctctcccacc ctcaggctcc tcgctggcct     780
ccgcacccgg gcaaaagccg ggaggaccgg gacccgcagc gcgacggcct gccgggcccc     840
tgcgcggtgg cacagcctgg gcccgctcaa gcggggccgc agggccaagg ggtgcttgcg     900
ccacccacgt cccaggggag tccgtggtgg ggctggggcc ggggtcccca ggtcgccggg     960
gcggcgtggg aaccccaagc cggggcagct ccacctcccc agcccgcgcc cccggacgcc    1020
tccgcctccg cgcggcaggg gcagatgcaa ggcatcccgg cgccctccca ggcgctccag    1080
gagccggcgc cctggtctgc actcccctgc ggcctgctgc tggatgagct cctggcgagc    1140
ccggagtttc tgcagcaggc gcaacctctc ctagaaacgg aggccccggg ggagctggag    1200
gcctcggaag aggccgcctc gctggaagca cccctcagcg aggaagaata ccgggctctg    1260
ctggaggagc tttaggacgc ggggtctagg cccggtgaga gactccacac cgcggagaac    1320
tgccattctt tcctgggcat cccggggatc ccagagccgg cccaggtacc agcagacctg    1380
cgcgcagtgc gcaccccggc tgacgtgcaa gggagctcgc tggcctctct gtgcccttgt    1440
tcttccgtga aattctggct gaatgtctcc ccccaccttc cgacgctgtc taggcaaacc    1500
tggattagag ttacatctcc tggatgatta gttcagagat atattaaaat gccccctccc    1560
tgtggatcct atag                                                      1574


<210> 2
<211> 16
<212> DNA
<213> Artificial sequence

<220>
<223> Synthetic oligonucleotide

<220>
<221> modified_base
<222> (1)..(1)
<223> m5c

<400> 2
cgagattccc gccggt                                                        16


<210> 3
<211> 15
<212> DNA
<213> Artificial Sequence

<220>
<223> Synthetic oligonucleotide

<220>
<221> modified_base
<222> (2)..(2)
<223> m5c

<400> 3
gcagttctcc gcggt                                                         15


<210> 4
<211> 16
<212> DNA
<213> Artificial Sequence

<220>
<223> Synthetic oligonucleotide

<220>
<221> modified_base
<222> (2)..(3), (14)..(14)
<223> m5c

<400> 4
gcctagacag cgtcgg                                                        16


<210> 5
<211> 1710
<212> DNA
<213> Homo sapiens

<400> 5
atggccctcc cgacaccctc ggacagcacc ctccccgcgg aagcccgggg acgaggacgg      60
cgacggagac tcgtttggac cccgagccaa agcgaggccc tgcgagcctg ctttgagcgg     120
aacccgtacc cgggcatcgc caccagagaa cggctggccc aggccatcgg cattccggag     180
cccagggtcc agatttggtt tcagaatgag aggtcacgcc agctgaggca gcaccggcgg     240
gaatctcggc cctggcccgg gagacgcggc ccgccagaag gccggcgaaa gcggaccgcc     300
gtcaccggat cccagaccgc cctgctcctc cgagcctttg agaaggatcg ctttccaggc     360
atcgccgccc gggaggagct ggccagagag acgggcctcc cggagtccag gattcagatc     420
tggtttcaga atcgaagggc caggcacccg ggacagggtg gcagggcgcc cgcgcaggca     480
ggcggcctgt gcagcgcggc ccccggcggg ggtcaccctg ctccctcgtg ggtcgccttc     540
gcccacaccg gcgcgtgggg aacggggctt cccgcacccc acgtgccctg cgcgcctggg     600
gctctcccac agggggcttt cgtgagccag gcagcgaggg ccgcccccgc gctgcagccc     660
agccaggccg cgccggcaga ggggatctcc caacctgccc cggcgcgcgg ggatttcgcc     720
tacgccgccc cggctcctcc ggacggggcg ctctcccacc ctcaggctcc tcgctggcct     780
ccgcacccgg gcaaaagccg ggaggaccgg gacccgcagc gcgacggcct gccgggcccc     840
tgcgcggtgg cacagcctgg gcccgctcaa gcggggccgc agggccaagg ggtgcttgcg     900
ccacccacgt cccaggggag tccgtggtgg ggctggggcc ggggtcccca ggtcgccggg     960
gcggcgtggg aaccccaagc cggggcagct ccacctcccc agcccgcgcc cccggacgcc    1020
tccgcctccg cgcggcaggg gcagatgcaa ggcatcccgg cgccctccca ggcgctccag    1080

gagccggcgc cctggtctgc actcccctgc ggcctgctgc tggatgagct cctggcgagc 1140
ccggagtttc tgcagcaggc gcaacctctc ctagaaacgg aggccccggg ggagctggag 1200
gcctcggaag aggccgcctc gctggaagca cccctcagcg aggaagaata ccgggctctg 1260
ctggaggagc tttaggacgc ggggttggga cggggtcggg tggttcgggg cagggcggtg 1320
gcctctcttt cgcggggaac acctggctgg ctacggaggg gcgtgtctcc gccccgcccc 1380
ctccaccggg ctgaccggcc tgggattcct gccttctagg tctaggcccg gtgagagact 1440
ccacaccgcg gagaactgcc attctttcct gggcatcccg gggatcccag agccggccca 1500
ggtaccagca gacctgcgcg cagtgcgcac cccggctgac gtgcaaggga gctcgctggc 1560
ctctctgtgc ccttgttctt ccgtgaaatt ctggctgaat gtctcccccc accttccgac 1620
gctgtctagg caaacctgga ttagagttac atctcctgga tgattagttc agagatatat 1680
taaaatgccc cctccctgtg gatcctatag 1710

<210> 6
<211> 768
<212> DNA
<213> Homo sapiens

<220>
<223> Description of Artificial Sequence: oligonucleotide

<400> 6
atggccctcc cgacaccctc ggacagcacc ctccccgcgg aagcccgggg acgaggacgg 60
cgacggagac tcgtttggac cccgagccaa agcgaggccc tgcgagcctg ctttgagcgg 120
aacccgtacc cgggcatcgc caccagagaa cggctggccc aggccatcgg cattccggag 180
cccagggtcc agatttggtt tcagaatgag aggtcacgcc agctgaggca gcaccggcgg 240
gaatctcggc cctggcccgg gagacgcggc ccgccagaag gccggcgaaa gcggaccgcc 300
gtcaccggat cccagaccgc cctgctcctc cgagcctttg agaaggatcg ctttccaggc 360
atcgccgccc gggaggagct ggccagagag acgggcctcc cggagtccag gattcagatc 420
tggtttcaga atcgaagggc caggcacccg ggacagggtg gcagggcgcc cgcgcaggtc 480
taggcccggt gagagactcc acaccgcgga gaactgccat tctttcctgg gcatcccggg 540
gatcccagag ccggcccagg taccagcaga cctgcgcgca gtgcgcaccc cggctgacgt 600
gcaagggagc tcgctggcct ctctgtgccc ttgttcttcc gtgaaattct ggctgaatgt 660
ctccccccac cttccgacgc tgtctaggca aacctggatt agagttacat ctcctggatg 720
attagttcag agatatatta aaatgccccc tccctgtgga tcctatag 768

<210> 7
<211> 14
<212> DNA
<213> Artificial Sequence

<220>
<223> Synthetic oligonucleotide

<400> 7
gattcccgcc ggtg 14

<210> 8
<211> 14
<212> DNA
<213> Artificial Sequence

<220>
<223> Synthetic oligonucleotide

<400> 8
agattcccgc cggt 14

<210> 9
<211> 15

<212> DNA
<213> Artificial Sequence

<220>
<223> Synthetic oligonucleotide

<400> 9
gagattcccg ccggt 15


<210> 10
<211> 16
<212> DNA
<213> Artificial Sequence

<220>
<223> Synthetic oligonucleotide

<220>
<221> modified_base
<222> (1)..(1), (8)..(10), (12)..(13)
<223> m5c

<400> 10
cgagattccc gccggt 16


<210> 11
<211> 14
<212> DNA
<213> Artificial Sequence

<220>
<223> Synthetic oligonucleotide

<220>
<221> modified_base
<222> (12)..(12)
<223> m5c

<400> 11
gagattcccg ccgg 14


<210> 12
<211> 14
<212> DNA
<213> Artificial Sequence

<220>
<223> Synthetic oligonucleotide

<220>
<221> modified_base
<222> (7)..(9), (11)..(12)
<223> m5c

<400> 12
gagattcccg ccgg 14


<210> 13
<211> 15

<212> DNA
<213> Artificial Sequence

<220>
<223> Synthetic oligonucleotide

<220>
<221> modified_base
<222> (1)..(1), (8)..(10), (12)..(13),
<223> m5c

<400> 13
cgagattccc gccgg                                                        15


<210> 14
<211> 15
<212> DNA
<213> Artificial Sequence

<220>
<223> Synthetic oligonucleotide

<220>
<221> modified_base
<222> (1)..(1), (13)..(13)
<223> m5c

<400> 14
cgagattccc gccgg                                                        15


<210> 15
<211> 16
<212> DNA
<213> Artificial Sequence

<220>
<223> Synthetic oligonucleotide

<400> 15
agttctccgc ggtgtg                                                       16


<210> 16
<211> 17
<212> DNA
<213> Artificial Sequence

<220>
<223> Synthetic oligonucleotide

<220>
<221> modified_base
<222> (2)..(2)
<223> m5c

<400> 16
gcagttctcc gcggtgt                                                      17


<210> 17
<211> 18

<212> DNA
<213> Artificial Sequence

<220>
<223> Synthetic oligonucleotide

<220>
<221> modified_base
<222> (3)..(3)
<223> m5c

<400> 17
ggcagttctc cgcggtgt                                                    18


<210> 18
<211> 15
<212> DNA
<213> Artificial Sequence

<220>
<223> Synthetic oligonucleotide

<220>
<221> modified_base
<222> (1)..(1)
<223> m5c

<400> 18
cagttctccg cggtg                                                       15


<210> 19
<211> 15
<212> DNA
<213> Artificial Sequence

<220>
<223> Synthetic oligonucleotide

<220>
<221> modified_base
<222> (1)..(1), (6)..(6), (8)..(9), (11)..(11)
<223> m5c

<400> 19
cagttctccg cggtg                                                       15


<210> 20
<211> 16
<212> DNA
<213> Artificial Sequence

<220>
<223> Synthetic oligonucleotide

<400> 20
gcagttctcc gcggtg                                                      16


<210> 21
<211> 16

<212> DNA
<213> Artificial Sequence

<220>
<223> Synthetic oligonucleotide

<220>
<221> modified_base
<222> (2)..(2)
<223> m5c

<400> 21
gcagttctcc gcggtg                                                       16


<210> 22
<211> 16
<212> DNA
<213> Artificial Sequence

<220>
<223> Synthetic oligonucleotide

<220>
<221> modified_base
<222> (2)..(2), (7)..(7), (9)..(10), (12)..(12)
<223> m5c

<400> 22
gcagttctcc gcggtg                                                       16


<210> 23
<211> 17
<212> DNA
<213> Artificial Sequence

<220>
<223> Synthetic oligonucleotide

<220>
<221> modified_base
<222> (3)..(3)
<223> m5c

<400> 23
ggcagttctc cgcggtg                                                      17


<210> 24
<211> 17
<212> DNA
<213> Artificial Sequence

<220>
<223> Synthetic oligonucleotide

<220>
<221> modified_base
<222> (3)..(3), (8)..(8), (10)..(11), (13)..(13)
<223> m5c

<400> 24

ggcagttctc cgcggtg                                                    17

<210> 25
<211> 14
<212> DNA
<213> Artificial Sequence

<220>
<223> Synthetic oligonucleotide

<220>
<221> modified_base
<222> (1)..(1)
<223> m5c

<400> 25
cagttctccg cggt                                                       14

<210> 26
<211> 15
<212> DNA
<213> Artificial Sequence

<220>
<223> Synthetic oligonucleotide

<220>
<221> modified_base
<222> (12)..(12)
<223> m5c

<400> 26
gcagttctcc gcggt                                                     15

<210> 27
<211> 15
<212> DNA
<213> Artificial Sequence

<220>
<223> Synthetic oligonucleotide

<220>
<221> modified_base
<222> (2)..(2), (12)..(12)
<223> m5c

<400> 27
gcagttctcc gcggt                                                        15

<210> 28
<211> 15
<212> DNA
<213> Artificial Sequence

<220>
<223> Synthetic oligonucleotide

<220>

<221> modified_base
<222> (2)..(2), (7)..(7), (9)..(10), (12)..(12)
<223> m5c

<400> 28
gcagttctcc gcggt                                                        15


<210> 29
<211> 15
<212> DNA
<213> Artificial Sequence

<220>
<223> Synthetic oligonucleotide

<220>
<221> modified_base
<222> (2)..(2), (7)..(7), (9)..(10)
<223> m5c

<400> 29
gcagttctcc gcggt                                                        15


<210> 30
<211> 15
<212> DNA
<213> Artificial Sequence

<220>
<223> Synthetic oligonucleotide

<220>
<221> modified_base
<222> (2)..(2), (7)..(7), (9)..(10), (12)..(12)
<223> m5c

<400> 30
gcagttctcc gcggt                                                        15


<210> 31
<211> 15
<212> DNA
<213> Artificial Sequence

<220>
<223> Synthetic oligonucleotide

<220>
<221> modified_base
<222> (2)..(2), (7)..(7), (9)..(10)
<223> m5c

<400> 31
gcagutctcc gcggt                                                        15


<210> 32
<211> 15
<212> DNA
<213> Artificial Sequence

<220>
<223> Synthetic oligonucleotide

<220>
<221> modified_base
<222> (2)..(2), (7)..(7), (9)..(10), (12)..(12)
<223> m5c

<400> 32
gcagutctcc gcggt                                                        15


<210> 33
<211> 16
<212> DNA
<213> Artificial Sequence

<220>
<223> Synthetic oligonucleotide

<400> 33
ggcagttctc cgcggt                                                       16


<210> 34
<211> 16
<212> DNA
<213> Artificial Sequence

<220>
<223> Synthetic oligonucleotide

<220>
<221> modified_base
<222> (3)..(3)
<223> m5c

<400> 34
ggcagttctc cgcggt                                                       16


<210> 35
<211> 16
<212> DNA
<213> Artificial Sequence

<220>
<223> Synthetic oligonucleotide

<220>
<221> modified_base
<222> (3)..(3), (8)..(8), (10)..(11), (13)..(13)
<223> m5c

<400> 35
ggcagttctc cgcggt                                                       16


<210> 36
<211> 17
<212> DNA
<213> Artificial Sequence

<220>
<223> Synthetic oligonucleotide

<400> 36
tggcagttct ccgcggt                                                    17


<210> 37
<211> 14
<212> DNA
<213> Artificial Sequence

<220>
<223> Synthetic oligonucleotide

<220>
<221> modified_base
<222> (2)..(2)
<223> m5c

<400> 37
gcagttctcc gcgg                                                       14


<210> 38
<211> 15
<212> DNA
<213> Artificial Sequence

<220>
<223> Synthetic oligonucleotide

<220>
<221> modified_base
<222> (13)..(13)
<223> m5c

<400> 38
ggcagttctc cgcgg                                                      15


<210> 39
<211> 15
<212> DNA
<213> Artificial Sequence

<220>
<223> Synthetic oligonucleotide

<220>
<221> modified_base
<222> (3)..(3)
<223> m5c

<400> 39
ggcagttctc cgcgg                                                      15


<210> 40
<211> 15
<212> DNA
<213> Artificial Sequence

<220>
<223> Synthetic oligonucleotide

<220>
<221> modified_base
<222> (3)..(3), (13)..(13)
<223> m5c

<400> 40
ggcagttctc cgcgg                                                        15


<210> 41
<211> 15
<212> DNA
<213> Artificial Sequence

<220>
<223> Synthetic oligonucleotide

<220>
<221> modified_base
<222> (3)..(3), (8)..(8), (10)..(11), (13)..(13)
<223> m5c

<400> 41
ggcagttctc cgcgg                                                        15


<210> 42
<211> 14
<212> DNA
<213> Artificial Sequence

<220>
<223> Synthetic oligonucleotide

<220>
<221> modified_base
<222> (1)..(1)
<223> m5c

<400> 42
cgtcggaagg tggg                                                         14


<210> 43
<211> 15
<212> DNA
<213> Artificial Sequence

<220>
<223> Synthetic oligonucleotide

<220>
<221> modified_base
<222> (2)..(2)
<223> m5c

<400> 43
gcgtcggaag gtggg                                                        15

<210> 44
<211> 16
<212> DNA
<213> Artificial Sequence

<220>
<223> Synthetic oligonucleotide

<220>
<221> modified_base
<222> (3)..(3)
<223> m5c

<400> 44
agcgtcggaa ggtggg                                                        16


<210> 45
<211> 17
<212> DNA
<213> Artificial Sequence

<220>
<223> Synthetic oligonucleotide

<220>
<221> modified_base
<222> (1)..(1)
<223> m5c

<400> 45
cagcgtcgga aggtggg                                                       17


<210> 46
<211> 15
<212> DNA
<213> Artificial Sequence

<220>
<223> Synthetic oligonucleotide

<220>
<221> modified_base
<222> (3)..(3)
<223> m5c

<400> 46
agcgtcggaa ggtgg                                                         15


<210> 47
<211> 15
<212> DNA
<213> Artificial Sequence

<220>
<223> Synthetic oligonucleotide

<220>
<221> modified_base
<222> (3)..(3), (6)..(6)

<223> m5c

<400> 47
agcgtcggaa ggtgg                                                        15


<210> 48
<211> 16
<212> DNA
<213> Artificial Sequence

<220>
<223> Synthetic oligonucleotide

<220>
<221> modified_base
<222> (1)..(1)
<223> m5c

<400> 48
cagcgtcgga aggtgg                                                       16


<210> 49
<211> 16
<212> DNA
<213> Artificial Sequence

<220>
<223> Synthetic oligonucleotide

<220>
<221> modified_base
<222> (1)..(1), (4)..(4), (7)..(7)
<223> m5c

<400> 49
cagcgtcgga aggtgg                                                       16


<210> 50
<211> 17
<212> DNA
<213> Artificial Sequence

<220>
<223> Synthetic oligonucleotide

<220>
<221> modified_base
<222> (2)..(2)
<223> m5c

<400> 50
acagcgtcgg aaggtgg                                                      17


<210> 51
<211> 15
<212> DNA
<213> Artificial Sequence

<220>

<223> Synthetic oligonucleotide

<220>
<221> modified_base
<222> (1)..(1)
<223> m5c

<400> 51
cagcgtcgga aggtg                                                        15


<210> 52
<211> 16
<212> DNA
<213> Artificial Sequence

<220>
<223> Synthetic oligonucleotide

<220>
<221> modified_base
<222> (2)..(2)
<223> m5c

<400> 52
acagcgtcgg aaggtg                                                       16


<210> 53
<211> 16
<212> DNA
<213> Artificial Sequence

<220>
<223> Synthetic oligonucleotide

<220>
<221> modified_base
<222> (3)..(3)
<223> m5c

<400> 53
gacagcgtcg gaaggt                                                       16


<210> 54
<211> 15
<212> DNA
<213> Artificial Sequence

<220>
<223> Synthetic oligonucleotide

<220>
<221> modified_base
<222> (3)..(3)
<223> m5c

<400> 54
gacagcgtcg gaagg                                                        15


<210> 55

<211> 16
<212> DNA
<213> Artificial Sequence

<220>
<223> Synthetic oligonucleotide

<400> 55
agacagcgtc ggaagg                                                        16


<210> 56
<211> 15
<212> DNA
<213> Artificial Sequence

<220>
<223> Synthetic oligonucleotide

<220>
<221> modified_base
<222> (1)..(2), (13)..(13)
<223> m5c

<400> 56
cctagacagc gtcgg                                                         15


<210> 57
<211> 16
<212> DNA
<213> Artificial Sequence

<220>
<223> Synthetic oligonucleotide

<220>
<221> modified_base
<222> (3)..(3), (14)..(14)
<223> m5c

<400> 57
gcctagacag cgtcgg                                                        16


<210> 58
<211> 16
<212> DNA
<213> Artificial Sequence

<220>
<223> Synthetic oligonucleotide

<220>
<221> modified_base
<222> (2)..(2)
<223> m5c

<400> 58
gcctagacag cgtcgg                                                        16


<210> 59

<211> 16
<212> DNA
<213> Artificial Sequence

<220>
<223> Synthetic oligonucleotide

<220>
<221> modified_base
<222> (2)..(2), (14)..(14)
<223> m5c

<400> 59
gcctagacag cgtcgg                                                        16


<210> 60
<211> 16
<212> DNA
<213> Artificial Sequence

<220>
<223> Synthetic oligonucleotide

<220>
<221> modified_base
<222> (2)..(3)
<223> m5c

<400> 60
gcctagacag cgtcgg                                                        16


<210> 61
<211> 16
<212> DNA
<213> Artificial Sequence

<220>
<223> Synthetic oligonucleotide

<220>
<221> modified_base
<222> (2)..(3), (8)..(8), (11)..(11), (14)..(14)
<223> m5c

<400> 61
gcctagacag cgtcgg                                                        16


<210> 62
<211> 16
<212> DNA
<213> Artificial Sequence

<220>
<223> Synthetic oligonucleotide

<220>
<221> modified_base
<222> (2)..(3), (8)..(8), (11)..(11), (14)..(14)
<223> m5c

<400> 62
gcctagacag cgucgg                                                        16


<210> 63
<211> 16
<212> DNA
<213> Artificial Sequence

<220>
<223> Synthetic oligonucleotide

<220>
<221> modified_base
<222> (2)..(3), (8)..(8), (11)..(11), (14)..(14)
<223> m5c

<400> 63
gccuagacag cgtcgg                                                        16


<210> 64
<211> 16
<212> DNA
<213> Artificial Sequence

<220>
<223> Synthetic oligonucleotide

<220>
<221> modified_base
<222> (2)..(3), (8)..(8), (11)..(11), (14)..(14)
<223> m5c

<400> 64
gccuagacag cgucgg                                                        16


<210> 65
<211> 14
<212> DNA
<213> Artificial Sequence

<220>
<223> Synthetic oligonucleotide

<220>
<221> modified_base
<222> (1)..(1), (3)..(3)
<223> m5c

<400> 65
ctcagctggc gtga                                                          14


<210> 66
<211> 14
<212> DNA
<213> Artificial Sequence

<220>
<223> Synthetic oligonucleotide

<220>
<221> modified_base
<222> (1)..(2)
<223> m5c

<400> 66
ccaggaaaga atgg                                                         14


<210> 67
<211> 14
<212> DNA
<213> Artificial Sequence


<220>
<223> Synthetic oligonucleotide

<220>
<221> modified_base
<222> (14)..(14)
<223> m5c

<400> 67
gggagacatt cagc                                                         14


<210> 68
<211> 14
<212> DNA
<213> Artificial Sequence

<220>
<223> Synthetic oligonucleotide

<220>
<221> modified_base
<222> (1)..(1)
<223> m5c

<400> 68
ctaatccagg tttg                                                         14


<210>  69
<211>  16
<212>  DNA
<213>  Artificial Sequence

<220>
<223>  Synthetic oligonucleotide

<220>
<221>  modified_base
<222>  (3)..(3),(16)..(16)
<223>  m5c

<400>  69
ggcgatgccc gggtac                                                         16


<210>  70
<211>  18

<212>  DNA
<213>  Artificial Sequence

<220>
<223>  Synthetic oligonucleotide

<220>
<221>  modified_base
<222>  (18)..(18)
<223>  m5c

<400>  70
gtggcgatgc ccgggtac                                                    18


<210>  71
<211>  18
<212>  DNA
<213>  Artificial Sequence

<220>
<223>  Synthetic oligonucleotide

<400>  71
ggtggcgatg cccgggta                                                    18


<210>  72
<211>  17
<212>  DNA
<213>  Artificial Sequence

<220>
<223>  Synthetic oligonucleotide

<400>  72
gtggcgatgc ccgggta                                                     17


<210>  73
<211>  16
<212>  DNA
<213>  Artificial Sequence

<220>
<223>  Synthetic oligonucleotide

<400>  73
tggcgatgcc cgggta                                                      16


<210>  74
<211>  20
<212>  DNA
<213>  Artificial Sequence

<220>
<223>  Synthetic oligonucleotide

<220>
<221>  modified_base
<222>  (1)..(1)
<223>  m5c

<400>  74
ctggtggcga tgcccgggta                                                   20


<210>  75
<211>  16
<212>  DNA
<213>  Artificial Sequence

<220>
<223>  Synthetic oligonucleotide

<400>  75
gtggcgatgc ccgggt                                                       16


<210>  76
<211>  17
<212>  DNA
<213>  Artificial Sequence

<220>
<223>  Synthetic oligonucleotide

<400>  76
ggtggcgatg cccgggt                                                      17


<210>  77
<211>  20
<212>  DNA
<213>  Artificial Sequence

<220>
<223>  Synthetic oligonucleotide

<220>
<221>  modified_base
<222>  (2)..(2)
<223>  m5c

<400>  77
tctggtggcg atgcccgggt                                                   20


<210>  78
<211>  16
<212>  DNA
<213>  Artificial Sequence

<220>
<223>  Synthetic oligonucleotide

<220>
<221>  modified_base
<222>  (12)..(12)
<223>  m5c

<400>  78
gagattcccg ccggtg                                                       16

<210>  79
<211>  20
<212>  DNA
<213>  Artificial Sequence

<220>
<223>  Synthetic oligonucleotide

<220>
<221>  modified_base
<222>  (2)..(2)
<223>  m5c

<400>  79
gcagttctcc gcggtgtgga                                                    20


<210>  80
<211>  19
<212>  DNA
<213>  Artificial Sequence

<220>
<223>  Synthetic oligonucleotide

<400>  80
ggcagttctc cgcggtgtg                                                     19


<210>  81
<211>  20
<212>  DNA
<213>  Artificial Sequence

<220>
<223>  Synthetic oligonucleotide

<400>  81
atggcagttc tccgcggtgt                                                    20


<210>  82
<211>  19
<212>  DNA
<213>  Artificial Sequence

<220>
<223>  Synthetic oligonucleotide

<400>  82
tggcagttct ccgcggtgt                                                     19


<210>  83
<211>  18
<212>  DNA
<213>  Artificial Sequence

<220>
<223>  Synthetic oligonucleotide

<400>  83
tggcagttct ccgcggtg                                                      18

<210>  84
<211>  20
<212>  DNA
<213>  Artificial Sequence

<220>
<223>  Synthetic oligonucleotide

<220>
<221>  modified_base
<222>  (2)..(2)
<223>  m5c

<400>  84
gcctagacag cgtcggaagg                                                   20


<210>  85
<211>  19
<212>  DNA
<213>  Artificial Sequence

<220>
<223>  Synthetic oligonucleotide

<220>
<221>  modified_base
<222>  (2)..(2)
<223>  m5c

<400>  85
gcctagacag cgtcggaag                                                    19

<210>  86
<211>  19
<212>  DNA
<213>  Artificial Sequence

<220>
<223>  Synthetic oligonucleotide

<400>  86
tgcctagaca gcgtcggaa                                                    19


<210>  87
<211>  20
<212>  DNA
<213>  Artificial Sequence

<220>
<223>  Synthetic oligonucleotide

<400>  87
tttgcctaga cagcgtcgga                                                   20


<210>  88
<211>  18
<212>  DNA
<213>  Artificial Sequence

<220>
<223>  Synthetic oligonucleotide

<400>  88
tgcctagaca gcgtcgga                                                    18


<210>  89
<211>  17
<212>  DNA
<213>  Artificial Sequence

<220>
<223>  Synthetic oligonucleotide

<220>
<221>  modified_base
<222>  (2)..(3)
<223>  m5c

<400>  89
gcctagacag cgtcgga                                                     17


<210>  90
<211>  20
<212>  DNA
<213>  Artificial Sequence

<220>
<223>  Synthetic oligonucleotide

<400>  90
gtttgcctag acagcgtcgg                                                  20


<210>  91
<211>  18
<212>  DNA
<213>  Artificial Sequence

<220>
<223>  Synthetic oligonucleotide

<400>  91
ttgcctagac agcgtcgg                                                    18


<210>  92
<211>  17
<212>  DNA
<213>  Artificial Sequence

<220>
<223>  Synthetic oligonucleotide

<220>
<221>  modified_base
<222>  (3)..(3), (15)..(15)
<223>  m5c

<400>  92

tgcctagaca gcgtcgg                                                    17

<210>  93
<211>  16
<212>  DNA
<213>  Artificial Sequence

<220>
<223>  Synthetic oligonucleotide

<220>
<221>  modified_base
<222>  (2)..(3), (14)..(14)
<223>  m5c

<400>  93
gcctagacag cgtcgg                                                     16

<210>  94
<211>  16
<212>  DNA
<213>  Artificial Sequence

<220>
<223>  Synthetic oligonucleotide

<220>
<221>  modified_base
<222>  (2)..(2), (12)..(12)
<223>  m5c

<400>  94
gcagttctcc gcggtg                                                     16

<210>  95
<211>  18
<212>  DNA
<213>  Artificial Sequence

<220>
<223>  Synthetic oligonucleotide

<400>  95
ggcagttctc cgcggtgt                                                   18

<210>  96
<211>  16
<212>  DNA
<213>  Artificial Sequence

<220>
<223>  Synthetic oligonucleotide

<220>
<221>  modified_base
<222>  (12)..(12)
<223>  m5c

<400>  96
gtggcgatgc ccgggt                                                        16


<210>  97
<211>  16
<212>  DNA
<213>  Artificial Sequence

<220>
<223>  Synthetic oligonucleotide

<220>
<221>  modified_base
<222>  (12)..(12)
<223>  m5c

<400>  97
gcagttctcc gcggtg                                                    16


<210>  98
<211>  16
<212>  DNA
<213>  Artificial Sequence

<220>
<223>  Synthetic oligonucleotide

<220>
<221>  modified_base
<222>  (2)..(2)
<223>  m5c

<400>  98
acgggttccg ctcaaa                                                        16


<210>  99
<211>  16
<212>  DNA
<213>  Artificial Sequence

<220>
<223>  Synthetic oligonucleotide

<220>
<221>  modified_base
<222>  (1)..(1),  (15)..(16)
<223>  m5c

<400>  99
cggaatgccg atggcc                                                        16


<210>  100
<211>  16
<212>  DNA
<213>  Artificial Sequence

<220>
<223>  Synthetic oligonucleotide

<220>
<221>  modified_base
<222>  (3)..(3), (14)..(14)
<223>  m5c

<400>  100
ttctggcggg ccgcgt                                                    16


<210>  101
<211>  16
<212>  DNA
<213>  Artificial Sequence

<220>
<223>  Synthetic oligonucleotide

<220>
<221>  modified_base
<222>  (14)..(14)
<223>  m5c

<400>  101
tagaccccgc gtccta                                                    16


<210>  102
<211>  16
<212>  DNA
<213>  Artificial Sequence

<220>
<223>  Synthetic oligonucleotide

<220>
<221>  modified_base
<222>  (2)..(3), (14)..(14)
<223>  m5c

<400>  102
gccuagacag cgtcgg                                                    16

<210>  103
<211>  16
<212>  DNA
<213>  Artificial Sequence

<220>
<223>  Synthetic oligonucleotide

<220>
<221>  modified_base
<222>  (2)..(3), (14)..(14)
<223>  m5c

<400>  103
gcctagacag cgucgg                                                    16



<210>  104
<211>  16
<212>  DNA

<213>  Artificial Sequence

<220>
<223>  Synthetic oligonucleotide

<220>
<221>  modified_base
<222>  (2)..(3), (14)..(14)
<223>  m5c

<400>  104
gccuagacag cgtcgg                                                       16


<210>  105
<211>  16
<212>  DNA
<213>  Artificial Sequence

<220>
<223>  Synthetic oligonucleotide

<220>
<221>  modified_base
<222>  (2)..(3), (14)..(14)
<223>  m5c

<400>  105
gccuagacag cgucgg                                                       16


<210>  106
<211>  16
<212>  DNA
<213>  Artificial Sequence

<220>
<223>  Synthetic oligonucleotide

<400>  106
gtggcgaugc ccgggt                                                       16


<210> 107
<211> 15
<212> DNA
<213> Artificial Sequence

<220>
<223> Synthetic oligonucleotide

<220>
<221> modified_base
<222> (2)..(2)
<223> m5c

<400> 107
gcagutctcc gcggt                                                        15


<210> 108
<211> 15
<212> DNA

<213> Artificial Sequence

<220>
<223> Synthetic oligonucleotide

<220>
<221> modified_base
<222> (2)..(2)
<223> m5c

<400> 108
gcagtuctcc gcggt                                                        15


<210> 109
<211> 15
<212> DNA
<213> Artificial Sequence

<220>
<223> Synthetic oligonucleotide

<220>
<221> modified_base
<222> (2)..(2)
<223> m5c

<400> 109
gcagttcucc gcggt                                                        15

## Description

### BACKGROUND OF THE INVENTION

### Field of the Invention

The present invention relates to a compound for reducing expression of DUX4 mRNA and protein in animals, a method for using the compound, and a pharmaceutical composition containing the compound. The method of the present invention is useful for therapeutically treating, preventing, or alleviating DUX4-related diseases, for example, facioscapulohumeral muscular dystrophy (FSHD).

### Description of Background

Facioscapulohumeral muscular dystrophy (FSHD) is a muscular dystrophy that occurs with an approximate frequency of 1 in 20,000 worldwide and 1 in 7,500 in Europe, and is the third most common muscular dystrophy after Duchenne muscular dystrophy and myotonic dystrophy. An initial symptom is muscle weakness of the face, upper limbs, scapula, and upper limb girdle. Lower limb girdle and lower limbs are also disturbed as the disease progresses, and about 20% of patients will be in a wheelchair by the age of 40 (even in middle age, there may be only mild facial muscle involvement). Complications of pain, neurological deafness and retinopathy are also common. Approximately 90% of patients develop symptoms by the age of 20. Severely affected patients (about 4%) show muscle weakness from infancy.

FSHD is classified into two types, FSHD1 and FSHD2, according to causative genes. FSHD1 accounts for about 95% of all FSHD patients. In FSHD1 patients, a D4Z4 repeat region of 4q35 is genetically shortened (1 - 10 D4Z4 repeats). As a result, abnormal expression of DUX4 encoded in the D4Z4 repeat region occurs (DUX4 is not expressed in healthy individuals). FSHD2 accounts for about 5% of all FSHD patients, and abnormal expression of DUX4 occurs due to SMCHD1 (DNA methylase) mutation. DUX4 has a transcription factor-like function and expresses a group of genes that are encoded downstream and cause muscle cell apoptosis or muscle atrophy. Abnormal expression of DUX4 is due to possession of an allele called 4qA among two alleles of 4qA and 4qB. A polyadenylation site present in 4qA is required for stabilizing DUX4 mRNA (Non-Patent Document 1, Non-Patent Document 2, Non-Patent Document 3).

An antisense technology is emerging as an effective means for modulating expression of certain gene products, and thus, may prove to be uniquely useful in some therapeutic, diagnostic, and research applications for modulating DUX4.

A method for suppressing DUX4 gene expression using an adeno-associated virus encoding DUX4 miRNA has been reported (Patent Document 1). However, preparation of the adeno-associated virus is cumbersome, and delivery of the adeno-associated virus to required systemic muscles is difficult.

A method for suppressing DUX4 gene expression using a lentivirus encoding DUX4 shRNA has been reported (Patent Document 2). However, preparation of the lentivirus is cumbersome, and delivery of the lentivirus to required systemic muscles is difficult. Further, in vitro gene silencing has 21% and 44% residual activities in quadriceps and trapezius muscle cells, and thus is not sufficient.

A compound in which multiple antisense oligonucleotides targeting DUX4 are linked has been reported (Patent Document 2). However, it is not a modified oligonucleotide and its inhibitory effect is not sufficient.

An antisense oligonucleotide compound that binds to a splicing site of DUX4 mRNA has been reported (Patent Document 3). However, since these compounds are selective for premRNA containing intron, their inhibitory effect with respect to mature mRNA is weak, and, due to knockdown by a Lipofection method, they are difficult to be administered to a living body.

Current treatments for FSHD include rehabilitation (stretching and exercise) as a symptomatic treatment, administration of NSAIDs, respiratory care, and the like. However, effects thereof are insufficient and burden on a patient is large. Therefore, it is an object herein to provide a compound, a composition and a method for treating FSHD

### [Related Art]

### [Patent Document]

[Patent Document 1] International Publication No. 2013/016352.
[Patent Document 2] International Publication No. 2017/007886.
[Patent Document 3] U.S. Patent Application Publication No. 2012/0225034.

### [Non-Patent Documents]

[Non-Patent Document 1] Snider et al., PLoS 2010, Vol.6 (10) p1.
[Non-Patent Document 2] Ferreboeuf et al., Human Molecular Genetics 2013, Vol. 23 (1), p171.
[Non-Patent Document 3] Sacconi et al., Biochim. Biophys. Acta 2015, p607.

The entire contents of these publications are incorporated herein by reference.

### SUMMARY OF THE INVENTION

### [Problems to Be Solved by the Invention]

An object of the present invention is to provide a compound, a method and a pharmaceutical composition for inhibiting expression of DUX4 and for treating, that is, therapeutically treating, preventing, delaying or ameliorating, DUX4-related diseases and/or symptoms thereof. The compound and pharmaceutical composition disclosed herein also inhibit mutant DUX4 such as SNP and DUX4 splicing variants.

Certain embodiments provide a method for reducing expression of DUX4 in animals (including humans), the method including: administering to an animal a compound containing a modified oligonucleotide targeting DUX4, or a pharmaceutical composition containing the compound, as described herein.

Certain embodiments provide a method for knockdown via a nuclear ribonuclease (such as RNase H) by administering to an animal a compound or a pharmaceutical composition containing a modified oligonucleotide that targets DUX4. Further, provided is a method for inhibiting transcription of DUX4 mRNA and translation of a DUX4 protein by administering a compound containing the modified oligonucleotide. The modified oligonucleotide is preferably distributed in muscle, and particularly preferably in skeletal muscle.

Certain embodiments provide a method for treating an animal having FSHD. In certain embodiments, the method of the present invention further includes administering to an animal a therapeutically effective amount of a compound or pharmaceutical composition, including a modified oligonucleotide that targets DUX4, as described herein. In certain embodiments, the method of the present invention includes identifying an animal having FSHD1 and/or FSHD2.

Certain embodiments provide a method of treating, that is, therapeutically treating, preventing, delaying, or ameliorating muscular atrophy and muscle weakness. It is to relieve or delay deterioration of poor facial expression, sleeping with eyes open, difficulty in raising upper limbs, and winged shoulder blades. Further, it is preferable to prevent muscle weakness of lumbar girdle and lower limbs, and also to prevent complications of neurological deafness and retinopathy.

In certain embodiments, DUX4 mRNA has a sequence set forth in GenBank accession number NM_001293798.2 (incorporated herein as SEQ ID NO: 1 in the sequence listing). A splicing variant of DUX4 mRNA of SEQ ID NO: 1 in the sequence listing is also referred to as DUX4-FL1 or mature mRNA of DUX4. In certain embodiments, DUX4 mRNA has a sequence set forth in GenBank accession number NM_001306068.2 (incorporated herein as SEQ ID NO: 5 in the sequence listing). A splicing variant of DUX4 mRNA of SEQ ID NO: 5 in the sequence listing is also referred to as DUX4-FL2. In certain embodiments, DUX4 has a sequence set forth in GenBank accession number NM_001363820.1 (incorporated herein as SEQ ID NO: 6 in the sequence listing). A splicing variant of DUX4 mRNA of SEQ ID NO: 6 in the sequence listing is also referred to as DUX4-s. In certain embodiments, DUX4 refers to SNP of the above splicing variant.

### [Means for Solving the Problems]

The present disclosure relates to, but is not limited to, the non-limiting numbered embodiments described in the following aspects [1] - [27].

Aspect [1] A modified oligonucleotide consisting 12 - 30 residues, comprising a nucleobase sequence that includes at least 8 contiguous nucleobase sequences and is complementary to an equal length portion at positions 126 - 147, 232 - 248, 1306 - 1325 or 1480 - 1495 from a 5' end of a nucleobase sequence of a mature mRNA of DUX4 of SEQ ID NO: 1, wherein the nucleobase sequence of the modified oligonucleotide has at least 90% complementarity to the equal length portion in the nucleobase sequence of the mature mRNA of DUX4 of SEQ ID NO: 1, when the at least 8 contiguous nucleobase sequences include a nucleobase sequence that is complementary to the equal length portion at the positions 1480 - 1495 from the 5' end of the nucleobase sequence of SEQ ID NO: 1, the modified oligonucleotide consists of a nucleobase sequence having at a 3' end a complementary base of a base of the position 1480 from the 5' end of the nucleobase of SEQ ID NO: 1.

Aspect [2] The modified oligonucleotide according to Aspect [1], wherein one or more modified nucleotides of the modified oligonucleotide each include a modified sugar.

Aspect [3] The modified oligonucleotide according to Aspect [2], wherein the modified sugar is selected from a group consisting of a bicyclic sugar, a 2'-O-methoxyethyl modified sugar, and a 2'-O-methyl modified sugar.

Aspect [4] The modified oligonucleotide according to Aspect [3], wherein the bicyclic sugar is selected from a group consisting of LNA, GuNA, ALNA [Ms], ALNA [mU], ALNA [ipU], ALNA [Oxz], and ALNA [Trz].

Aspect [5] A modified oligonucleotide consisting of 12-30 residues, the modified oligonucleotide comprising a nucleobase sequence that includes at least 8 contiguous nucleobase sequences and is complementary to an equal length portion at positions 1472 - 1495 from a 5' end of a nucleobase sequence of a mature mRNA of DUX4 of SEQ ID NO: 1, the nucleobase sequence of the modified oligonucleotide having at least 90% complementarity to the equal length portion in the nucleobase sequence of the mature mRNA of DUX4 of SEQ ID NO: 1, and the modified oligonucleotide comprising at least one nucleoside that includes a modified sugar selected from GuNA, ALNA [Ms], ALNA [mU], ALNA [ipU], ALNA [Oxz], and ALNA [Trz].

Aspect [6] The modified oligonucleotide according to Aspect [5], further comprising a 2'-O-methoxyethyl modified sugar and/or a 2'-O-methyl modified sugar.

Aspect [7] The modified oligonucleotide according to any one of Aspects [1] - [6], wherein at least one modified nucleotide of the modified oligonucleotide includes a modified nucleobase.

Aspect [8] The modified oligonucleotide according to Aspect [7], wherein the modified nucleobase is a 5-methylcytosine.

Aspect [9] The modified oligonucleotide according to any one of Aspects [1] - [8], wherein at least one internucleoside linkage is a modified internucleoside linkage.

Aspect [10] The modified oligonucleotide according to Aspect [9], wherein the modified internucleoside linkage is a phosphorothioate internucleoside linkage.

Aspect [11] The modified oligonucleotide according to any one of Aspects [1] - [10], comprising:
1) a gap segment;
2) a 5' wing segment; and
3) a 3' wing segment,
wherein the gap segment is positioned between the 5' wing segment and the 3' wing segment, all nucleosides of the 5' wing segment and the 3' wing segment each include at least one modified sugar, and the gap segment includes only nucleosides that contain no modified sugar, or includes one or two nucleosides that each contain a modified sugar, and includes other nucleosides that contain no modified sugar.

Aspect [12] The modified oligonucleotide according to any one of Aspects [1] - [11], consisting of a nucleobase sequence that is complementary to
a nucleobase sequence of positions 128 - 143 from the 5' end of the nucleobase sequence of the mature mRNA of DUX4 of SEQ ID NO: 1,
a nucleobase sequence of positions 232 - 247 from the 5 'end,
a nucleobase sequence of positions 233 - 248 from the 5' end,
a nucleobase sequence of positions 1309 - 1323 from the 5' end, or
a nucleobase sequence of positions 1480 - 1495 from the 5' end.

Aspect [13] The modified oligonucleotide according to any one of Aspects [1] - [12], consisting of a base sequence of
gtggcgatgc ccgggt (SEQ ID NO: 75),
gagattcccg cnggtg (SEQ ID NO: 78: n represents a 5-methylcytosine),
ngagattcccgccggt (SEQ ID NO: 2: n represents a 5-methylcytosine),
gnagttctccgcggt (SEQ ID NO: 3: n represents a 5-methylcytosine), or
gnntagacagcgtngg (SEQ ID NO: 4: n represents a 5-methylcytosine).

Aspect [14] The modified oligonucleotide according to Aspect [13] represented by the following formula:

GlsMlsMlsTdsAdsGdsAdsCdsAdsGdsCdsGdsTdsMlsGlsGl,

wherein nucleobases are represented according to the following symbols:
   A = adenine, T = thymine, G = guanine, C = cytosine, and M = 5-methylcytosine;
sugar moieties are represented according to the following symbols:
   l = LNA, and d = 2'-deoxyribose;
and internucleoside linkages are represented according to the following symbol:
   s = phosphorothioate.

Aspect [15] The modified oligonucleotide according to Aspect [13] represented by the following formula:

GmsMmsMmsTdsAdsGdsAdsCdsAdsGdsCdsGdsTdsMmsGmsGm,

wherein nucleobases are represented according to the following symbols:
   A = adenine, T = thymine, G = guanine, C = cytosine, and M = 5-methylcytosine;
sugar moieties are represented according to the following symbols:
   m = ALNA [Ms], and d = 2'-deoxyribose;
and internucleoside linkages are represented according to the following symbol:
   s = phosphorothioate.

Aspect [16] The modified oligonucleotide according to Aspect [13] represented by the following formula:

GmsMmsAmsGdsTdsTdsCdsTdsCdsCdsGdsCdsGmsGmsTm,

wherein nucleobases are represented according to the following symbols:
   A = adenine, T = thymine, G = guanine, C = cytosine, and M = 5-methylcytosine;
sugar moieties are represented according to the following symbols:
   m = ALNA [Ms], and d = 2'-deoxyribose;
and internucleoside linkages are represented according to the following symbol:
   s = phosphorothioate.

Aspect [17] The modified oligonucleotide according to Aspect [13] represented by the following formula:

MlsGlsAlsGdsAdsTdsTdsCdsCdsCdsGdsCdsCdsGlsGlsTl,

wherein nucleobases are represented according to the following symbols:
   A = adenine, T = thymine, G = guanine, C = cytosine, and M = 5-methylcytosine;
sugar moieties are represented according to the following symbols:
   l = LNA, and d = 2'-deoxyribose;
and internucleoside linkages are represented according to the following symbol:
   s = phosphorothioate.

Aspect [18] The modified oligonucleotide according to Aspect [14] represented by the following formula or a salt thereof:

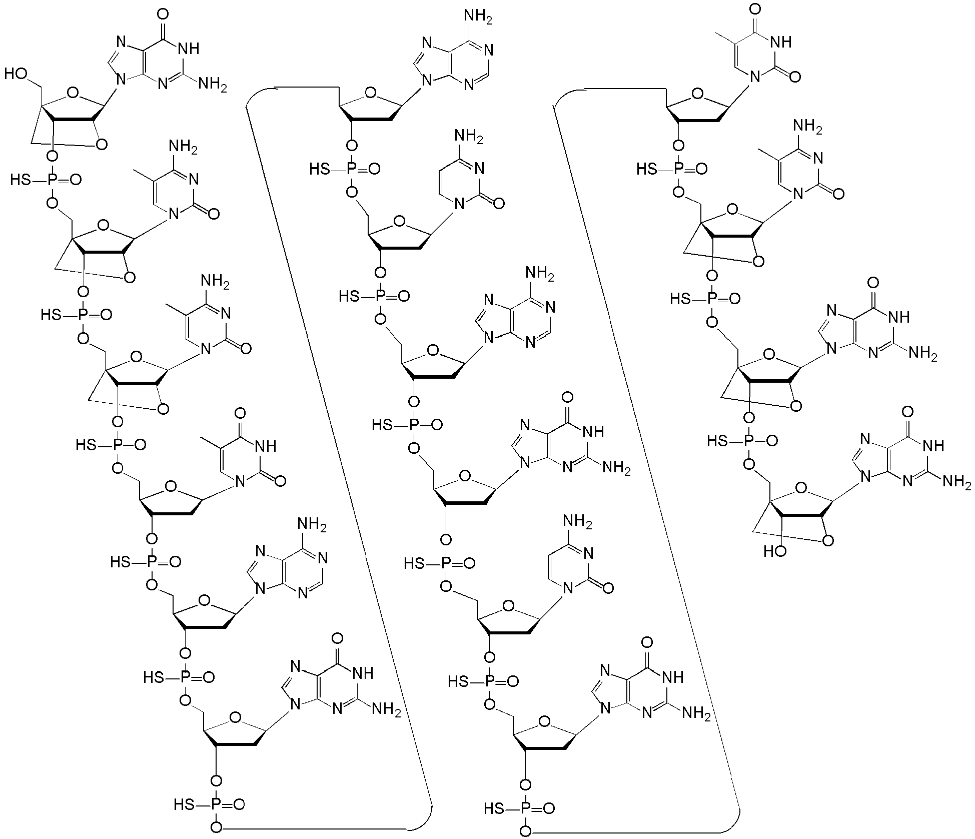

Aspect [19] The modified oligonucleotide according to Aspect [15] represented by the following formula or a salt thereof:

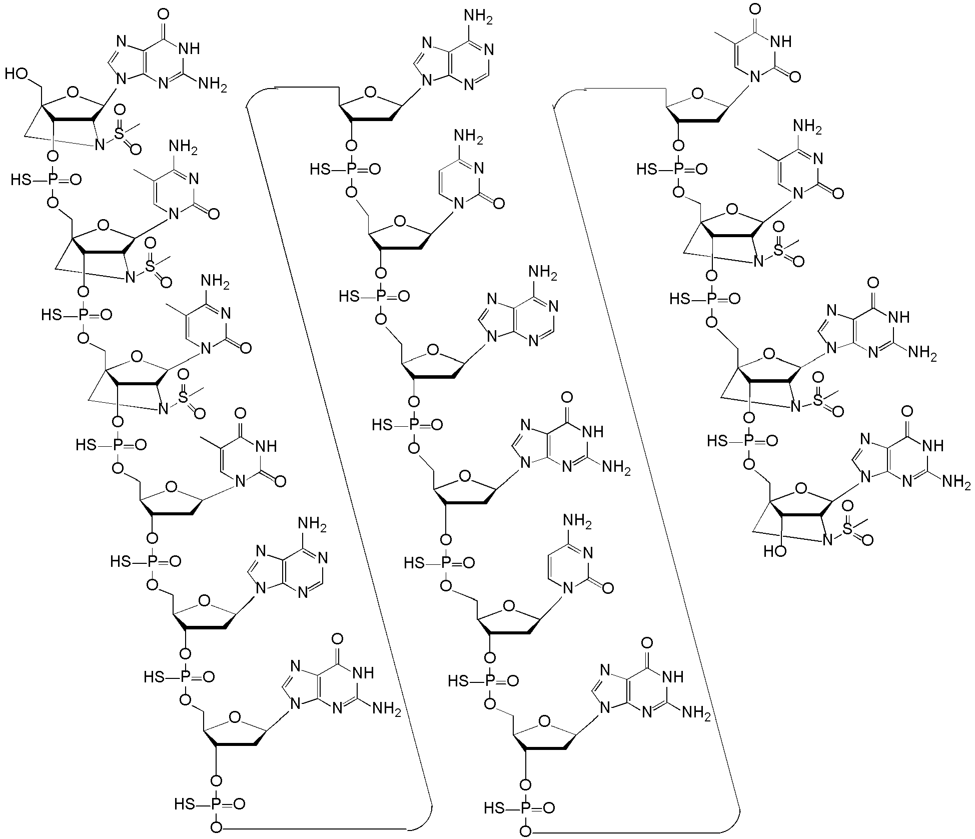

Aspect [20] The modified oligonucleotide according to Aspect [16] represented by the following formula or a salt thereof:

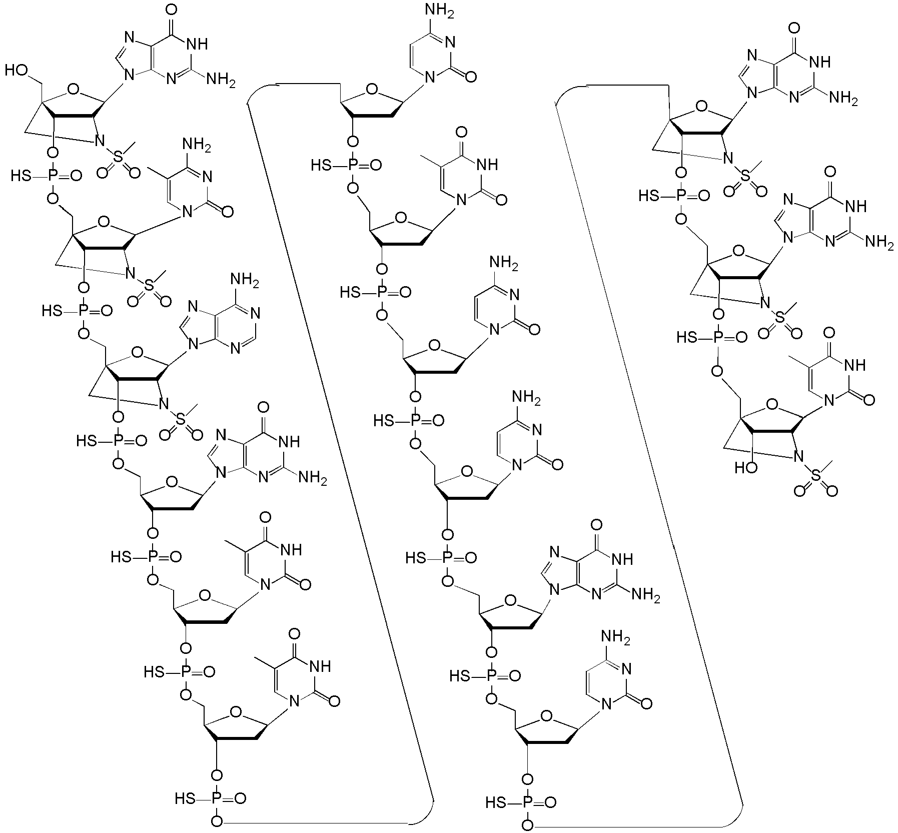

Aspect [21] The modified oligonucleotide according to Aspect [17] represented by the following formula or a salt thereof:

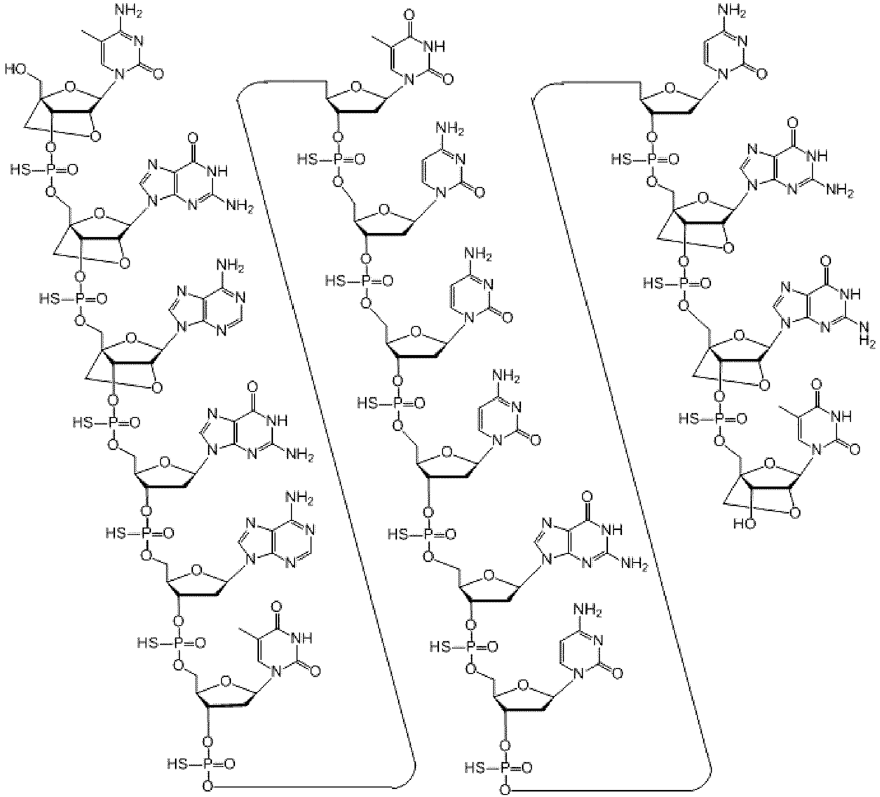

Aspect [22] A pharmaceutical composition comprising: the modified oligonucleotide according to any one of Aspects [1] - [21] or a pharmaceutically acceptable salt thereof; and a pharmaceutically acceptable carrier.

Aspect [23] The pharmaceutical composition according to Aspect [22], for therapeutically treating, preventing, or delaying progress of a DUX4-related disease.

Aspect [24] The pharmaceutical composition according to Aspect [23], wherein the DUX4-related disease is facioscapulohumeral muscular dystrophy.

Aspect [25] A method for therapeutically treating, preventing or delaying progress of a DUX4-related disease in a subject, comprising: administering an effective amount of the modified oligonucleotide according to any one of Aspects [1] - [21] to a subject in need thereof.

Aspect [26] Use of the modified oligonucleotide according to any one of Aspects [1] - [21], in manufacture of a medicament for therapeutically treating, preventing or delaying progress of a DUX4-related disease.

Aspect [27] Use of the modified oligonucleotide according to any one of Aspects [1] - [21], for therapeutically treating, preventing or delaying progress of a DUX4-related disease.

### [Effect of the Invention]

According to the present invention, a modified oligonucleotide effective for treating diseases such as facioscapulohumeral muscular dystrophy caused by abnormal expression of the DUX4 gene can be provided.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:
[Fig. 1] A figure showing effects of suppressing DUX4 gene expression by administration of various DUX4 modified oligonucleotides.
[Fig. 2] A figure showing effects of suppressing DUX4 gene expression by administration of various DUX4 modified oligonucleotides.
[Fig. 3] A figure showing dose-dependent effects of suppressing DUX4 gene expression by administration of DUX4 modified oligonucleotides.
[Fig. 4] A figure showing effects of suppressing DUX4 gene expression by administration of various DUX4 modified oligonucleotides.
[Fig. 5] Figures showing effects of suppressing creatine kinase in DUX4 Tg mice and suppressing DUX4 mRNA by administration of a DUX4 modified oligonucleotide (Compound No. 3).
[Fig. 6] Figures showing effects of suppressing creatine kinase in DUX4 Tg mice and suppressing DUX4 mRNA by administration of DUX4 modified oligonucleotides (Compound No. 123, Compound No. 247 and Compound No. 113).

### DETAILED DESCRIPTION OF THE EMBODIMENTS

Embodiments will now be described with reference to the accompanying drawings, wherein like reference numerals designate corresponding or identical elements throughout the various drawings.

It is to be understood that both the foregoing summary and the following detailed description are exemplary and explanatory only and are not restrictive of the present invention as claimed. In the present specification, unless specifically stated otherwise, the use of a singular form includes a plural form. In the present specification, unless specifically stated otherwise, the use of "or" means "and/or." Further, the use of the term "including" and its other forms such as "includes" and "included" are not limiting. Further, unless specifically stated otherwise, the term "element" or the like includes an element that include one unit and an element that includes more than one subunit.

Section headings used herein are for organizational purposes only and should not be construed as limiting the subject matter described. All documents or portions of documents cited in this application, including, but not limited to, patents, patent applications, reports, books and articles, are expressly incorporated herein by reference, with respect to the portions of the document discussed herein, and in their entirety.

### (Definitions)

Unless a specific definition is given, the nomenclature used in connection with analytical chemistry, synthetic organic chemistry, and medical chemistry and pharmaceutical chemistry, and their procedures and techniques described herein are those that are well known in the art and are commonly used. Standard techniques can be used for chemical synthesis and chemical analysis as used herein. Where permitted, all patents, patent applications, published patent applications and other publications referred to throughout the disclosure of this specification, and GenBank accession numbers and relevant sequence information as well as other data available through databases such as the National Center for Biotechnology Information (NCBI), are incorporated by reference with respect to portions of the documents discussed herein, and in its entirety.

Further, this specification is filed together with a sequence listing in electronic format. However, the information of the sequence listing described in the electronic format is incorporated herein by reference in its entirety.

Unless otherwise indicated, the following terms have the following meanings.

A "nucleic acid" refers to a molecule consisting of a monomeric nucleotide. Examples of nucleic acids include, but not limited to, ribonucleic acid (RNA), deoxyribonucleic acid (DNA), single-stranded nucleic acid, double-stranded nucleic acid, small interfering ribonucleic acid (siRNA), and microRNA (miRNA). A nucleic acid can also include combinations of these elements in a single molecule.

A "nucleobase" means a heterocyclic moiety that can pair with the base of another nucleic acid. Nucleobases include "modified nucleobases" and "unmodified nucleobases."

A "nucleobase sequence" means an order of contiguous nucleobases independent of any sugar linkage or nucleobase modification.

A "nucleoside" means a nucleobase linked to a sugar. In certain embodiments, a nucleoside is linked to a phosphate group.

A "nucleotide" means a nucleoside having a phosphate group or the like covalently linked to a sugar moiety of the nucleoside. Naturally occurring nucleotides have ribose or deoxyribose sugar moieties and are covalently linked by phosphodiester bonds through phosphate groups.

An "oligomer compound" or "oligomer" refers to a polymer of linked monomeric subunits that is capable of hybridizing to at least one region of a nucleic acid molecule.

An "oligonucleotide" means a polymer of linked nucleosides in which nucleosides and internucleoside linkages may be, independently of each other, modified or unmodified.

An "unmodified nucleotide" means a nucleotide consisting of a naturally occurring nucleobase, a sugar moiety and an internucleoside linkage. In certain embodiments, the unmodified nucleotide is, but is not limited to, an RNA nucleotide (that is, a β-D-ribonucleoside) or a DNA nucleotide (that is, a β-D-deoxyribonucleoside).

A "modified nucleotide" means a nucleotide having, independently, a modified sugar moiety, a modified internucleoside linkage or a modified nucleobase. A "modified nucleoside" means a nucleoside having, independently, a modified sugar moiety or a modified nucleobase.

An "internucleoside linkage" refers to a chemical bond between nucleosides.

"Linked nucleosides" means adjacent nucleosides that are bonded or linked by an internucleoside linkage.

A "naturally occurring internucleoside linkage" means a 3'-5 'phosphodiester linkage.

A "modified internucleoside linkage" refers to a substitution or any change from a naturally occurring internucleoside linkage (that is, a phosphodiester internucleoside linkage). For example, there is a phosphorothioate internucleoside linkage, but it is not limited thereto.

A "phosphorothioate internucleoside linkage" means an internucleoside linkage in which a phosphodiester linkage is modified by replacing one of non-bridging oxygen atoms with a sulfur atom. A phosphorothioate linkage is one example of the modified internucleoside linkage.

A "modified nucleobase" refers to any nucleobase other than adenine, cytosine, guanine, thymidine or uracil. For example, there is a 5-methylcytosine, but it is not limited thereto. "Unmodified nucleobases" means the purine bases adenine (A) and guanine (G), and the pyrimidine bases thymine (T), cytosine (C) and uracil (U).

A "modified oligonucleotide" means an oligonucleotide that contains at least one of the modified nucleoside and/or the modified internucleoside linkage.
"Salt" is a generic term for compounds in which one or more dissociable hydrogen ions contained in an acid are replaced by cations such as metal ions and ammonium ions, and examples of salts of a modified oligonucleotide include, but not limited to, salts (for example, a sodium salt, and a magnesium salt) formed with inorganic ions (for example, sodium ions, and magnesium ions) on thio (S) groups of phosphorothioate linkages or functional groups (for example, an amino group) in a modified nucleobase.

A "sugar" or a "sugar moiety" means a natural or modified sugar moiety.

A "natural sugar moiety" means a sugar found in DNA (2'-H) or RNA (2'-OH).

A "modified sugar" refers to a substitution or change from a natural sugar moiety. Examples of modified sugars include a substituted sugar moiety and a sugar moiety substitute.

A "substituted sugar moiety" means a furanosyl other than a natural sugar of RNA or DNA.

"2'-O-methoxyethyl" (as well as 2'-MOE and 2'-O(CH₂)₂-OCH₃) refers to O-methoxy-ethyl modification at 2'-position of a furanosyl ring. A 2'-O-methoxyethyl modified sugar is a modified sugar.

A "2'-O-methoxyethyl nucleotide" means a nucleotide containing a 2'-O-methoxyethyl modified sugar moiety.

"2'-O-methyl" (as well as 2'-OMe and 2'-OCH₃) refers to an O-methyl modification at 2'-position of a furanosyl ring. A 2'-O-methyl modified sugar is a modified sugar.

A "2'-O-methyl nucleotide" means a nucleotide that contains a 2'-O-methyl modified sugar moiety.

A "sugar surrogate" for a "sugar moiety substitute" is intended to indicate replacement of only a sugar unit (a furanose ring). A sugar surrogate can replace a naturally occurring sugar moiety of a nucleoside, and, as a result, the resulting nucleoside subunits can be linked to each other and/or to other nucleosides to form an oligomeric compound that can hybridize with a complementary oligomeric compound. Such structures include rings (for example, 4, 6 or 7 membered rings) containing a different number of atoms from furanosyl; replacement of oxygen in furanosyl with non-oxygen atoms (for example, carbon, sulfur or nitrogen); or both the change in the number of atoms and the replacement of oxygen. Such structures may also contain substitutions corresponding to those described regarding the substituted sugar moiety (for example, a 6-membered carbocyclic bicyclic sugar substitute optionally containing additional substituents). Sugar substitutes also include more complex sugar replacements (for example, acyclic peptide nucleic acids). Examples of sugar surrogates include, but are not limited to, morpholino, cyclohexenyl and cyclohexitol.

A "bicyclic sugar" means a furanosyl ring modified by bridging of two different carbon atoms present on the same ring. Preferably, a "bicyclic sugar" means a modified sugar in which the 2' and 4' positions of the furanosyl ring are modified by bridging. A "bicyclic nucleic acid" refers to a nucleoside or nucleotide in which a furanose moiety of the nucleoside or nucleotide contains a "bicyclic sugar."

"LNA" means a nucleoside or nucleotide commonly referred to as a 2',4'-locked nucleic acid, and examples thereof include a nucleoside or nucleotide represented by the general formula: 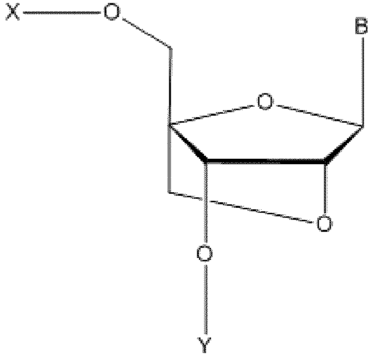 [wherein B is a nucleobase; and X and Y are each independently a hydrogen atom, a protecting group of a hydroxyl group, a phosphate group that may be substituted, a phosphorus moiety, a covalent attachment to a support, or the like] (see WO 98/39352). Typical specific examples thereof include a nucleoside or a nucleotide represented by the following formula:

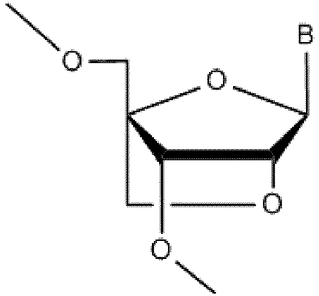

"GuNA" is a nucleoside or nucleotide represented by the following formula: 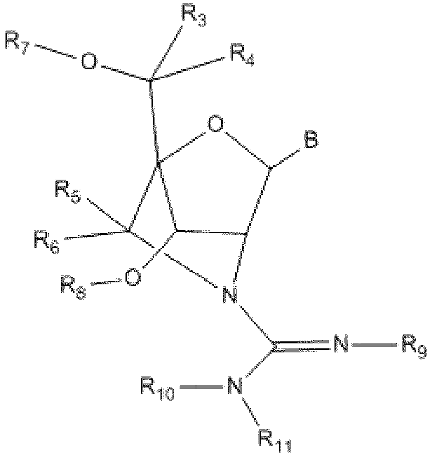 [wherein, B is a nucleobase; R₃, R₄, R₅ and R₆ are each independently a hydrogen atom or a C₁₋₆ alkyl group that may be substituted with one or more substituents; R₇ and R₈ are each independently a hydrogen atom, a protecting group of a hydroxyl group, a phosphate group that may be substituted, a phosphorus moiety, a covalent attachment to a support, or the like; and R₉, R₁₀, and R₁₁ are each independently a hydrogen atom, a C₁₋₆ alkyl group that may be substituted with one or more substituents, or a protecting group of an amino group]. (See, for example, WO 2014/046212, and WO 2017/047816).

"ALNA [mU]" is a nucleoside or nucleotide represented by the following general formula (I): 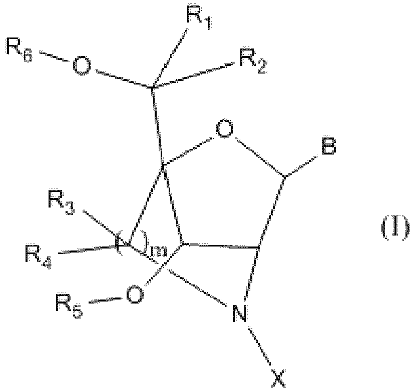 [wherein B is a nucleobase; R₁, R₂, R₃ and R₄ are each independently a hydrogen atom, or a C₁₋₆ alkyl group that may be substituted with one or more substituents; R₅ and R₆ are each independently a hydrogen atom, a protecting group of a hydroxyl group, or a phosphate group that may be substituted, a phosphorus moiety, a covalent attachment to a support, or the like; m is 1 or 2; X is a group represented by the following formula (II-1): 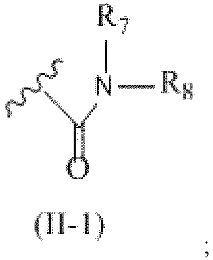 the symbol: 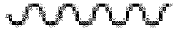 described in the formula (II-1) represents a point of attachment to a 2'-amino group; one of R₇ and R₈ is a hydrogen atom, and the other is a methyl group that may be substituted with one or more substituents] (see, for example, Japanese Patent Application No. 2018-212424). A typical specific example is a nucleoside or nucleotide in which one of R₇ and R₈ is a hydrogen atom and the other is an unsubstituted methyl group.

"ALNA [ipU]" is a nucleoside or nucleotide represented by the general formula (I) defined in the above "ALNA [mU]," and in the formula, X is a group represented by the following formula (II-1): 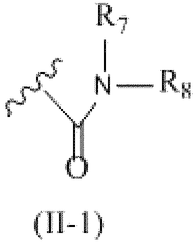 wherein one of R₇ and R₈ is a hydrogen atom, and the other is an isopropyl group that may be substituted with one or more substituents (see, for example, Japanese Patent Application No. 2018-212424). A typical specific example is a nucleoside or nucleotide in which one of R₇ and R₈ is a hydrogen atom and the other is an unsubstituted isopropyl group.

"ALNA [Trz]" is a nucleoside or nucleotide represented by the general formula (I) defined in the above "ALNA [mU]," and in the formula, X is a group represented by the following formula (II-2): 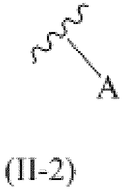 wherein A is a triazolyl group that may be substituted with one or more substituents (see, for example, Japanese Patent Application No. 2018-212424). A typical specific example is a nucleoside or nucleotide in which A is a triazolyl group that may have one or more methyl groups, more specifically, a 1,5-dimethyl-1,2,4-triazol-3-yl group.

"ALNA [Oxz]" is a nucleoside or nucleotide represented by the general formula (I) defined in the above "ALNA [mU]," and in the formula, X is a group represented by the following formula (II-2): 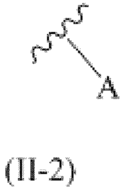 wherein A is an oxadiazolyl group that may be substituted with one or more substituents (see, for example, Japanese Patent Application No. 2018-212424). A typical specific example is a nucleoside or nucleotide in which A is an oxadiazolyl group that may have one or more methyl groups, more specifically, a 5-methyl-1,2,4-oxadiazol-3-yl group.

"ALNA [Ms]" is a nucleoside or nucleotide represented by the general formula (I) defined in the above "ALNA [mU]," and in the formula, X is a group represented by the following formula (II-3): 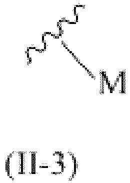 wherein M is a sulfonyl group substituted with a methyl group that may be substituted with one or more substituents (see, for example, Japanese Patent Application No. 2018-212424). A typical specific example is a nucleoside or nucleotide in which M is a sulfonyl group substituted with an unsubstituted methyl group.

A "5-methylcytosine" means a cytosine modified with a methyl group attached to a 5-position. A 5-Methylcytosine is a modified nucleobase.

A "single-stranded oligonucleotide" means an oligonucleotide that is not hybridized to a complementary strand.

"DUX4" means a nucleic acid or protein of a transcription factor that is also called a double homeobox 4. DUX4 has, for example, various splicing variants transcribed from the DUX4 gene, or single nucleotide substitutions thereof (SNPs), and may be the variants and/or the SNPs.

Many splicing variants have been reported for DUX4 mRNA. Human DUX4-s (SEQ ID NO: 6 in the sequence listing) consists of short exon 1 (exon 1s), exon 2, and exon 3 due to an atypical splicing donor site within exon 1, and encodes a nontoxic short DUX4 protein. Human DUX4-FL consists of exon 1, exon 2, and exon 3, and encodes a full-length DUX4 protein. DUX4-FL includes DUX4-FL1 (SEQ ID NO: 1 in the sequence listing) which is a mature mRNA that does not contain intron 1, and DUX4-FL2 (SEQ ID NO: 5 in the sequence listing) which contains intron 1, and both encode a full-length DUX4 protein and, when expressed in muscle, cause FSHD (see the above Non-Patent Document 2).

DUX4 mRNA is also expressed in normal testis, and, in addition to DUX4-FL, exon 1, exon 2, exon 6, exon 7 splicing variants and/or exon 1, exon 2, exon 4, exon 5, exon 6, exon 7 splicing variants are expressed (see the above Non-Patent Document 1).

The DUX4 protein functions as a transcription factor, and examples of genes whose transcription is modulated by DUX4 include MBD3L2, ZSCAN4, TRIM43, DEFB103, ZNF217 and the like (see the above Non-Patent Document 2).

The DUX4 mRNA targeted by a modified oligonucleotide of the present invention is, for example, preferably human DUX4, more preferably DUX4-FL, and even more preferably DUX4-FL1 described in SEQ ID NO: 1 in the sequence listing. Further, as a target site of the modified oligonucleotide of the present invention with respect to DUX4, exon 1, intron 1, exon 2, intron 2, and exon 3 are preferable.

It is known that the DUX4 gene is expressed by fusion with other genes due to chromosomal abnormalities such as translocation. As the other genes, for example, IGH (Yasuda et al., Nature Genetics 48 (5), 569 (2016)), CIC (Yoshimoto et al., Cancer research 77, 2927 (2017)), EWSR1 (Sirvent et al., Cancer Genetics and Cytogenetics 195, 12 (2009)) are reported, and are thought to be causative genes of B-cell acute lymphocytic leukemia, differentiated round cell sarcoma, fetal rhabdomyosarcoma, and the like. Modified oligonucleotides of the present invention also include compounds targeting these fusion genes.

"Expression of DUX4" means a level of mRNA transcribed from a gene encoding DUX4 or a level of a protein translated from the mRNA. Expression of DUX4 can be determined using methods known in the art, such as Northern or Western blot, PCR.

A "DUX4 nucleic acid" means any nucleic acid that encodes DUX4. For example, in certain embodiments, a DUX4 nucleic acid includes a DNA sequence encoding DUX4, an RNA sequence transcribed from DNA encoding DUX4 (including genomic DNA containing introns and exons), and a mRNA precursor or a spliced mature mRNA encoding DUX4. Further, in certain embodiments, DNA and RNA sequences of genes generated by fusion of the DUX4 gene and other genes are included.

"DUX4 mRNA" means mRNA encoding a DUX4 protein.

"Contiguous nucleobases" or "consecutive nucleobases" mean nucleobases that are immediately adjacent to each other.

"Complementary" means ability with respect to pairing between nucleobases of a first nucleic acid and a second nucleic acid.

"Fully complementary (also called complementarity)" or "100% complementary (also called complementarity)" means that all nucleobases in a nucleobase sequence of a first nucleic acid have complementary nucleobases in a second nucleobase sequence of a second nucleic acid. In certain embodiments, a first nucleic acid is a modified oligonucleotide and a target nucleic acid is a second nucleic acid.

"Hybridization" means annealing of a complementary nucleic acid molecule. In certain embodiments, examples of complementary nucleic acid molecules include a modified oligonucleotide and a target nucleic acid.

"Specifically hybridizable" refers to a modified oligonucleotide that has sufficient complementarity between a modified oligonucleotide and a target nucleic acid to induce a desired effect, while exhibiting minimal or no effect on a non-target nucleic acid under conditions where specific binding is desired, that is, under physiological conditions for in vivo assays and therapeutic treatments.

"Mismatch" or "non-complementary nucleobase" refers to a case where a nucleobase of a first nucleic acid cannot pair with a corresponding nucleobase of a second nucleic acid or a target nucleic acid.

"Targeting" or "to target" means a process of designing and selecting a modified oligonucleotide that specifically hybridizes to a target nucleic acid and induces a desired effect.

A "target nucleic acid," a "target RNA" and a "target RNA transcript" all refer to a nucleic acid that can be targeted by a modified oligonucleotide. In certain embodiments, a target nucleic acid includes a region of a DUX4 nucleic acid.

A "target segment" refers to a nucleotide sequence of a target nucleic acid that is targeted by a modified oligonucleotide. A "5' target site" refers to a 5'-most nucleotide of a target segment. A "3' target site" refers to a 3'-most nucleotide of a target segment.

An "active target region" or a "target region" means a region targeted by one or more active modified oligonucleotides. An "active modified oligonucleotide" means a modified oligonucleotide that reduces a target nucleic acid level or a protein level.

"Antisense inhibition" means that, as compared to a target nucleic acid level or a target protein level in the absence of a modified oligonucleotide, a target nucleic acid level or a protein level in the presence of a modified oligonucleotide complementary to the target nucleic acid is reduced.

An "siRNA" means a double-stranded RNA oligonucleotide having a nucleobase sequence that enables hybridization with respect to a corresponding region or segment of a target nucleic acid.

An "shRNA" means a hairpin-type single-stranded RNA oligonucleotide having a nucleobase sequence that enables hybridization with respect to a corresponding region or segment of a target nucleic acid.

An "snoRNA" means a single-stranded oligonucleotide that is a non-coding RNA present in nucleolus, has a nucleobase sequence that enables hybridization with respect to a corresponding region or segment of a target RNA nucleic acid, and guides chemical modification of methylation or pseudouridylation of a target RNA nucleic acid.

An "miRNA" means a single-stranded or double-stranded RNA oligonucleotide that is a non-coding RNA modulating the expression of other genes, and has a nucleobase sequence that enables hybridization with respect to a corresponding region or segment of a target nucleic acid.

A "cap structure" or a "terminal cap moiety" means a chemical modification incorporated at either end of a modified oligonucleotide.

A "chemically distinct region" refers to a region of a modified oligonucleotide that is in some way chemically different from another region of the same modified oligonucleotide. For example, a region having a 2'-O-methoxyethyl nucleotide is chemically different from a region having a nucleotide that is not 2'-O-methoxyethyl modified.

A "chimeric modified oligonucleotide" means a modified oligonucleotide having at least two chemically distinct regions.

"Motif' means a pattern of chemically distinct regions in a modified oligonucleotide.

A "gapmer" means a chimeric modified oligonucleotide in which an internal region having multiple nucleosides that support RNase H cleavage is positioned between external regions having one or more nucleosides, and the nucleosides forming the internal region are chemically distinct from the nucleoside or nucleosides forming the external regions. The internal region can be referred to as a "gap segment" and the external regions can be referred to as "wing segments." A wing segment positioned at 5' from a gap segment can be referred to as a "5' wing segment," and a wing segment positioned at 3' from a gap segment can be referred to as a "3' wing segment."

"Immediately adjacent" means that there are no intervening elements between immediately adjacent elements.

"Nuclear ribonuclease" means a ribonuclease found in nucleus. Examples of nuclear ribonucleases include, but are not limited to, RNase H including RNase HI and RNase H₂, and double-stranded RNase drosha, and other double-stranded RNases.

In certain embodiments, a gapmer means a modified oligonucleotide having a 5' wing segment, a 3' wing segment, and a gap segment, the 5' and 3' wing segments each having 1 - 8 nucleosides, and the gap segment having 6 or more nucleosides and being positioned between and immediately adjacent to the two wing segments, all the nucleosides of the gap segment being nucleosides that contain no modified sugar or one or two of the nucleosides of the gap segment being nucleosides that each contain a modified sugar and the other nucleosides of the gap segment being nucleosides that contain no modified sugar.

An "agent" refers to an active substance that can provide a therapeutic benefit when administered to an animal. A "first agent" means a therapeutic compound of the present invention. For example, the first agent may be a modified oligonucleotide targeting DUX4. A "second agent" means a second therapeutic compound of the present invention (for example, a second modified oligonucleotide targeting DUX4) and/or a therapeutic compound that does not target DUX4.

A "pharmaceutically acceptable salt" means a physiologically and pharmaceutically acceptable salt of a modified oligonucleotide, that is, a salt that retains a desired biological activity of a modified oligonucleotide and does not impart an undesired toxic effect to the modified oligonucleotide.

A "diluent" means an ingredient in a composition that lacks pharmacological activity, but is pharmaceutically necessary or desirable. For example, a diluent in a composition to be injected may be a liquid such as saline.

A "DUX4-related disease" refers to a disease caused by abnormal expression of DUX4 mRNA or DUX4 protein, or mRNA or protein of a fusion gene due to translocation of the DUX4 gene. Examples thereof include, but are not limited to, facioscapulohumeral muscular dystrophy, B-cell acute lymphocytic leukemia, differentiated round cell sarcoma, fetal rhabdomyosarcoma, and the like.

"Facioscapulohumeral muscular dystrophy" or "FSHD" means muscular dystrophy arising from muscle weakness of facial, scapular, and brachial muscles. In humans, it is thought that along with shortening of the genomic repeat (D4Z4) which is mainly near the telomere (end of the chromosome) of chromosome 4, a genome structure changes as a result, and the DUX4 gene, which is not originally expressed in muscle (progenitor) cells, is ectopically expressed and causes cell death (FSHD1). Further, in some FSHD patients, a gene mutation has been found in SMCHD1, one of genomic structural regulators that suppress gene expression (FSHD2).

FSHD is a type of muscular dystrophy associated with progressive muscle weakness and muscle fiber loss. Unlike Duchenne muscular dystrophy and Becker muscular dystrophy, which mainly affect the lower body, FSHD occurs in the upper body, mainly the facial, scapular, and brachial muscles. However, it may also occur in the pelvis, lower back, and lower limbs. Symptoms of FSHD often appear after the age of 10 to 26, but it is not uncommon for them to develop much later. In some cases, it may not occur at all. Usually, the symptoms are mild and the rate of deterioration is also very slow. Facial muscle weakness is common, and eyelid ptosis, inability to whistle, decreased facial expression changes, melancholy or angry facial expression, difficulty pronouncing words, scapular weakness (causing deformations such as conspicuous scapula (winged shoulder blades) and slopping shoulders), lower limb weakness, hearing loss, and possible heart disease and the like may occur.

An "active pharmaceutical agent" means a substance (or substances) in a pharmaceutical composition that provides a therapeutic benefit when administered to an animal. For example, in certain embodiments, a modified oligonucleotide targeting DUX4 is an active pharmaceutical agent.

"Simultaneously administered" refers to co-administration of two agents in any manner in which pharmacological effects resulting from both agents are simultaneously expressed in a patient. Simultaneous administration does not require that both agents be administered in a single pharmaceutical composition, in the same dosage form, or by the same route of administration. The pharmacological effects resulting from both agents do not need to be expressed simultaneously. The pharmacological effects need only overlap within a certain period of time and need not be coextensive.

"Administering" means providing an agent to an animal, and includes, but is not limited to, administering by a medical professional and self-administering.

"Amelioration" refers to a lessening of at least one indicator, sign, or symptom of an associated disease, disorder, or condition. Severity of an indicator can be determined by a subjective or objective measure that is known to a person skilled in the art.

"Animal" refers to a human or non-human animal, including, but not limited to, mice, rats, rabbits, dogs, cats, pigs, and non-human primates, including, but not limited to, monkeys and chimpanzees.

"Co-administration" means administration of two or more agents to an individual. The two or more agents may be present in a single pharmaceutical composition, or may be present in separate pharmaceutical compositions. Each of the two or more agents can be administered through the same or different routes of administration. Co-administration includes parallel or sequential administration.

A "dose" means a specified amount of a pharmaceutical agent provided in a single administration, or in a specified period of time. In certain embodiments, a dose can be administered in one, two, or more boluses, tablets, or injections. For example, in certain embodiments where subcutaneous administration is desired, a desired dose requires a volume that is not easily accommodated by a single injection, and thus, two or more injections can be used to achieve the desired dose. In certain embodiments, a pharmaceutical agent is administered by infusion over an extended period of time or continuously. A dose can be described as an amount of a pharmaceutical agent per hour, day, week, or month.

An "effective amount" or a "therapeutically effective amount" means an amount of an active pharmaceutical agent that is sufficient to achieve a desired physiological outcome in an individual in need of the agent. The effective amount can vary from individual to individual depending on the health and physical conditions of the individual to be treated, the taxonomic group of the individual to be treated, the formulation of the composition, assessment of the individual's medical condition, and other relevant factors.

"Identifying an animal having facioscapulohumeral muscular dystrophy (FSHD)" means identifying an animal diagnosed with a disorder or condition of FSHD, or identifying an animal susceptible to a disorder or condition of FSHD. For example, an individual with a family history may be predisposed to disorders or conditions of FSHD

Such identification can be accomplished using any method, including examining the individual's medical history and standard clinical tests or assessments. As FSHD, FSHD1 and FSHD2 are known depending on the pathogenesis, and both are included.

An "individual" means a human or non-human animal selected for treatment or therapy.

"Myotonia" means abnormally slow muscle relaxation after voluntary contraction or electrical stimulation.

"Parenteral administration" means administration via injection or infusion. Examples of parenteral administration include subcutaneous administration, intravenous administration, intramuscular administration, intraarterial administration, intraperitoneal administration, or intracranial administration, for example, intrathecal or intraventricular administration. Administration may be continuous or long-term, short-term or intermittent.

A "pharmaceutical composition" means a mixture of substances suitable for administration to an individual. For example, a pharmaceutical composition can contain one or more active agents and a sterile aqueous solution.

"Preventing" means delaying or preventing the onset or development of a disease, disorder, unfavorable health condition, or one or more symptoms associated with the disease, disorder or undesired health condition for a period of time from minutes to indefinitely. "Preventing" also means reducing a risk of developing a disease, a disorder, or an undesirable health condition.

"Therapeutically treating" means alleviating or eliminating a disease, disorder, or unfavorable health condition, or one or more symptoms associated with the disease, disorder, or unfavorable condition, or partially eliminating or eradicating one or more causes of the disease, disorder, or unfavorable health condition itself.

"Treating" is intended to include preventing or therapeutically treating described above. For example, "treating" includes administering a pharmaceutical composition of the present invention to cause a change or improvement in the disease, disorder, or undesired health condition.

A "prodrug" means a therapeutic agent that is prepared in an inactive form that is converted to an active form within a body or cells thereof by an action of an endogenous enzyme or other chemical substances or conditions.

"Side effects" means physiological responses that can be attributed to a treatment other than the desired effects. In certain embodiments, side effects include injection site reactions, liver function test abnormalities, renal function abnormalities, liver toxicity, renal toxicity, central nervous system abnormalities, myopathies, and malaise. For example, an elevated level of alanine aminotransferase (ALT), aspartate aminotransferase (AST), or γ-glutamyl transpeptidase (γ-GTP) in the blood can indicate liver toxicity or liver function abnormality. For example, elevated bilirubin can indicate liver toxicity or liver function abnormality. Further, elevated urinary protein and elevated levels of creatinine and urea nitrogen (UN) in the blood may indicate renal toxicity or renal function abnormality.

"Subcutaneous administration" means administration just below the skin.

A "therapeutically effective amount" means an amount of an agent that provides a therapeutic benefit to an individual.

### (Specific Embodiments)

### Embodiments

Certain specific embodiments described below provide, but are not limited to, compounds for inhibiting Expression of DUX4, methods of using the compounds, and pharmaceutical compositions containing the compounds.

Certain embodiments provide a method for reducing expression of DUX4 in an animal, including administering to the animal a compound containing a modified oligonucleotide that targets DUX4.

Certain embodiments provide a method for administering a modified oligonucleotide to hinder accumulation of pathogenic DUX4 transcription factors by inhibiting DUX4 gene transcription, or inhibiting DUX4 mRNA translation, or guiding DUX4 mRNA cleavage.

Certain embodiments provide a method for treating an animal having facioscapulohumeral muscular dystrophy, the method including the following steps: a) identifying the animal having facioscapulohumeral muscular dystrophy; and b) administering to the animal a therapeutically effective amount of a compound containing a modified oligonucleotide that targets DUX4.

Certain embodiments provide a method for alleviating myotonia in a subject in need thereof. The method includes administering to the subject a modified oligonucleotide complementary to DUX4 mRNA. The modified oligonucleotide activates a ribonuclease or a nuclear ribonuclease when bound to DUX4 mRNA, thereby reducing myotonia. In certain embodiments, a subject has or is suspected of having facioscapulohumeral muscular dystrophy, or, has or is suspected of having high expression of DUX4 mRNA, has or is suspected of having a reduced number of D4Z4 repeats on human chromosome 4, or has or is suspected of having an SMCHD1 (DNA methylase) mutation.

In certain embodiments, a modified oligonucleotide used in a method of the present invention is chimeric. In certain embodiments, a modified oligonucleotide of a method used in the present invention is a gapmer.

In certain embodiments of the method of the present invention described herein, administration is subcutaneous. In certain embodiments, administration is intravenous or intramuscular.

In certain embodiments, a modified oligonucleotide used in the method of the present invention targets a DUX4 protein coding region, an intron, a 5' UTR or a 3' UTR of DUX4 mRNA. In certain embodiments, a modified oligonucleotide used in the method of the present invention targets exon 1, exon 2, exon 3, intron 1, and intron 2 of DUX4 mRNA.

In certain embodiments of the method of the present invention described herein, DUX4 mRNA is cleaved by a nuclear ribonuclease RNase HI.

In certain embodiments of the method of the present invention, DUX4 mRNA is reduced in a muscle tissue. In certain embodiments, splicing variants DUX4-FL1 (SEQ ID NO: 1 in the sequence listing), DUX4-FL2 (SEQ ID NO: 5 in the sequence listing) are preferentially reduced.

In certain embodiments, DUX4 mRNA has a sequence set forth in GenBank accession number NM_001293798.2 (incorporated herein as SEQ ID NO: 1 in the sequence listing). A splicing variant of SEQ ID NO: 1 in the sequence listing is also referred to as DUX4-FL1 or mature mRNA of DUX4. In certain embodiments, DUX4 mRNA has a sequence set forth in GenBank accession number NM_001306068.2 (incorporated herein as SEQ ID NO: 5 in the sequence listing). A splicing variant of SEQ ID NO: 5 in the sequence listing is also referred to as DUX4-FL2. In certain embodiments, DUX4 has a sequence set forth in GenBank accession number NM_001363820.1 (incorporated herein as SEQ ID NO: 6 in the sequence listing). A splicing variant of SEQ ID NO: 6 in the sequence listing is also referred to as DUX4-s. In certain embodiments, DUX4 mRNA has an SNP of the above-described splicing variant.

In certain embodiments, the modified oligonucleotide is a modified oligonucleotide that consists of 12 - 30 residues, and has a nucleobase sequence containing a modified oligonucleotide that is a nucleobase sequence that includes at least 8 contiguous nucleobase sequences that are complementary to an equal length portion of positions 126 - 147, 232 - 248, 1306 - 1325, or 1472 - 1495 from a 5' end of a nucleobase of a mature mRNA of DUX4 of SEQ ID NO: 1 in the sequence listing. In certain embodiments, the modified oligonucleotide is a modified oligonucleotide that consists of 12 - 30 residues, and has a nucleobase sequence containing a modified oligonucleotide that is a nucleobase sequence that includes at least 9, 10, 11, or 12 contiguous nucleobase sequences that are complementary to an equal length portion of positions 126 - 147, 232 - 248, 1306 - 1325, or 1472 - 1495 from a 5' end of a nucleobase of a mature mRNA of DUX4 of SEQ ID NO: 1 in the sequence listing.

The modified oligonucleotide may consists of at least 8 contiguous nucleobase sequences that are complementary to an equal length portion of positions 126 - 147, 232 - 248, 1306 - 1325 or 1472 - 1495 from a 5' end of a nucleobase sequence of a mature mRNA of DUX4 of SEQ ID NO: 1 in the sequence listing, and may have, in addition to this nucleobase sequence, an additional sequence on the 5' end side and/or the 3' end side.

In certain embodiments, the modified oligonucleotide is a nucleobase sequence that includes at least 8, 9, 10, 11, 12, 13, 14, 15, 16, 17, 18, 19, 20, 21 or 22 contiguous nucleobase sequences that are complementary to an equal length portion of positions 126 - 147 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a nucleobase sequence that includes at least 8, 9, 10, 11, 12, 13, 14, 15, 16, or 17 contiguous nucleobase sequences that are complementary to an equal length portion of positions 232 - 248 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a nucleobase sequence that includes at least 8, 9, 10, 11, 12, 13, 14, 15, 16, 17, 18, 19, or 20 contiguous nucleobase sequences that are complementary to an equal length portion of positions 1306 - 1325 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a nucleobase sequence that includes at least 8, 9, 10, 11, 12, 13, 14, 15, 16, 17, 18, 19, 20, 21, 22, 23, or 24 contiguous nucleobase sequences that are complementary to an equal length portion of positions 1472 - 1495 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a modified oligonucleotide that consists of 12 - 30 residues, and includes a nucleobase sequence that is complementary to an equal length portion of positions 126 - 147 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a modified oligonucleotide that consists of 12 - 30 residues, and includes a nucleobase sequence that is complementary to an equal length portion of positions 232 - 248 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a modified oligonucleotide that consists of 12 - 30 residues, and includes a nucleobase sequence that is complementary to an equal length portion of positions 1306 - 1325 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a modified oligonucleotide that consists of 12 - 30 residues, and includes a nucleobase sequence that is complementary to an equal length portion of positions 1472 - 1495 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide consists of 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a modified oligonucleotide that consists of 12 - 30 residues, and has a nucleobase sequence containing a modified oligonucleotide that is a nucleobase sequence that includes at least 8 contiguous nucleobase sequences that are complementary to an equal length portion of positions 128 - 143, 233 - 248, 1309 - 1323 or 1480 - 1495 from a 5' end of a nucleobase of a mature mRNA of DUX4 of SEQ ID NO: 1 in the sequence listing. In certain embodiments, the modified oligonucleotide is a modified oligonucleotide that consists of 12 - 30 residues, and has a nucleobase sequence containing a modified oligonucleotide that is a nucleobase sequence that includes at least 9, 10, 11, or 12 contiguous nucleobase sequences that are complementary to an equal length portion of positions 128 - 143, 233 - 248, 1309 - 1323 or 1480 - 1495 from a 5' end of a nucleobase of a mature mRNA of DUX4 of SEQ ID NO: 1 in the sequence listing.

In certain embodiments, the modified oligonucleotide is a nucleobase sequence that includes at least 8, 9, 10, 11, 12, 13, 14, 15, or 16 contiguous nucleobase sequences that are complementary to an equal length portion of positions 128 - 143 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a nucleobase sequence that includes at least 8, 9, 10, 11, 12, 13, 14, 15, or 16 contiguous nucleobase sequences that are complementary to an equal length portion of positions 233 - 248 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a nucleobase sequence that includes at least 8, 9, 10, 11, 12, 13, 14, or 15 contiguous nucleobase sequences that are complementary to an equal length portion of positions 1309 - 1323 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a modified oligonucleotide of 30 or less residues that includes at least 8, 9, 10, 11, 12, 13, 14, 15, or 16 contiguous nucleobase sequences that are complementary to an equal length portion of positions 1480 - 1495 from a 5' end of a nucleobase of a mature mRNA of DUX4 of SEQ ID NO: 1 in the sequence listing, and consists of a nucleobase sequence having at a 3' end a complementary base of a base of the position 1480 from the 5' end of the nucleobase sequence of SEQ ID NO: 1.

In certain embodiments, the modified oligonucleotide is a modified oligonucleotide that consists of 12 - 30 residues, and includes a nucleobase sequence that is complementary to an equal length portion of positions 128 - 143 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a modified oligonucleotide that consists of 12 - 30 residues, and includes a nucleobase sequence that is complementary to an equal length portion of positions 233 - 248 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a modified oligonucleotide that consists of 12 - 30 residues, and includes a nucleobase sequence that is complementary to an equal length portion of positions 1309 - 1323 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a a modified oligonucleotide consisting of 12 - 30 residues, and is a nucleobase sequence that is complementary to an equal length portion of positions 1480 - 1495 from a 5' end of a nucleobase of a mature mRNA of DUX4 of SEQ ID NO: 1 in the sequence listing, and consists of a nucleobase sequence that has at a 3' end a complementary base of a base of a position 1480 from a 5' end of a nucleobase of SEQ ID NO: 1, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide consists of 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide provided herein targets any one of the following regions of SEQ ID NO: 1 in the sequence listing: positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or1480 - 1499 from a 5' end. In certain embodiments, the modified oligonucleotide provided herein targets any one of the following regions of SEQ ID NO: 1 in the sequence listing: positions 128 - 143, 232 - 247, 233 - 248, 1309 - 1323 and 1480 - 1495 from a 5' end.

In certain embodiments, the modified oligonucleotide provided herein has a nucleobase sequence that includes a complementary region that contains at least 8 contiguous nucleobases that are complementary with respect to a target region. In certain embodiments, the modified oligonucleotide provided herein has a nucleobase sequence that includes a complementary region that contains at least 8 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein has a nucleobase sequence that includes a complementary region that contains at least 8 contiguous nucleobases that are complementary with respect to a target region, and the target region targets positions 128 - 143, 232 - 247, 233 - 248, 1309 - 1323 or 1480 - 1495 from a 5' end of SEQ ID NO: 1 in the sequence listing.

In certain embodiments, the modified oligonucleotide provided herein has a nucleobase sequence that includes a complementary region that contains at least 10 contiguous nucleobases that are complementary with respect to a target region. In certain embodiments, the modified oligonucleotide provided herein has a nucleobase sequence that includes a complementary region that contains at least 10 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein has a nucleobase sequence that includes a complementary region that contains at least 10 contiguous nucleobases that are complementary with respect to a target region, and the target region targets positions 128 - 143, 232 - 247, 233 - 248, 1309 - 1323 or 1480 - 1495 from a 5' end of SEQ ID NO: 1 in the sequence listing.

In certain embodiments, the modified oligonucleotide provided herein has a nucleobase sequence that includes a complementary region that contains at least 12 contiguous nucleobases that are complementary with respect to a target region. In certain embodiments, the modified oligonucleotide provided herein has a nucleobase sequence that includes a complementary region that contains at least 12 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein has a nucleobase sequence that includes a complementary region that contains at least 12 contiguous nucleobases that are complementary with respect to a target region, and the target region targets positions 128 - 143, 232 - 247, 233 - 248, 1309 - 1323 or 1480 - 1495 from a 5' end of SEQ ID NO: 1 in the sequence listing.

In certain embodiments, the modified oligonucleotide provided herein has a nucleobase sequence that includes a complementary region that contains at least 14 contiguous nucleobases that are complementary with respect to a target region. In certain embodiments, the modified oligonucleotide provided herein has a nucleobase sequence that includes a complementary region that contains at least 14 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein has a nucleobase sequence that includes a complementary region that contains at least 14 contiguous nucleobases that are complementary with respect to a target region, and the target region targets positions 128 - 143, 232 - 247, 233 - 248, 1309 - 1323 or 1480 - 1495 from a 5' end of SEQ ID NO: 1 in the sequence listing.

In certain embodiments, the modified oligonucleotide is a modified oligonucleotide that includes a nucleobase sequence that is complementary to an equal length portion of positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of a nucleobase of a mature mRNA of DUX4 of SEQ ID NO: 1 in the sequence listing. Further, in certain embodiments, the modified oligonucleotide is a modified oligonucleotide that includes a nucleobase sequence that is complementary to an equal length portion of positions 128 - 143, 232 - 247, 233 - 248, 1309 - 1323, or 1480 - 1495 from a 5' end of a nucleobase of a mature mRNA of DUX4 of SEQ ID NO: 1 in the sequence listing.

In certain embodiments, the modified oligonucleotide is a modified oligonucleotide including a nucleobase sequence set forth in any one of SEQ ID NOs: 2 - 4, 7 - 64, 69 - 97, and 102 - 109 in the sequence listing. Further, in certain embodiments, the modified oligonucleotide is a modified oligonucleotide including a nucleobase sequence set forth in any one of SEQ ID NOs: 2 - 4, 75, and 78 in the sequence listing.

In certain embodiments, the modified oligonucleotide is a modified oligonucleotide consisting of a nucleobase sequence set forth in any one of SEQ ID NOs: 2 - 4, 7 - 64, 69 - 97, and 102 - 109 in the sequence listing. Further, in certain embodiments, the modified oligonucleotide is a modified oligonucleotide consisting of a nucleobase sequence set forth in any one of SEQ ID NOs: 2 - 4, 75, and 78 in the sequence listing.

In certain embodiments, the modified oligonucleotide has a nucleobase sequence including at least 8 contiguous nucleobases of a nucleobase sequence set forth in any one of SEQ ID NOs: 2, 3, 4, 75, or 78 in the sequence listing.

In certain embodiments, the modified oligonucleotide has a nucleobase sequence including at least 10 contiguous nucleobases of a nucleobase sequence set forth in any one of SEQ ID NOs: 2, 3, 4, 75, or 78 in the sequence listing.

In certain embodiments, the modified oligonucleotide has a nucleobase sequence including at least 12 contiguous nucleobases of a nucleobase sequence set forth in any one of SEQ ID NOs: 2, 3, 4, 75, or 78 in the sequence listing.

In certain embodiments, the modified oligonucleotide has a nucleobase sequence including at least 14 contiguous nucleobases of a nucleobase sequence set forth in any one of SEQ ID NOs: 2, 3, 4, 75, or 78 in the sequence listing.

In certain embodiments, the modified oligonucleotide has a nucleobase sequence including a nucleobase consisting of a nucleobase sequence set forth in any one of SEQ ID NOs: 2, 3, 4, 75, or 78 in the sequence listing.

In certain embodiments, the modified oligonucleotide has a nucleobase sequence consisting of a nucleobase sequence set forth in any one of SEQ ID NOs: 2, 3, 4, 75, or 78 in the sequence listing.

In certain embodiments, the animal is a human.

In certain embodiments, the administration includes parenteral administration.

In certain embodiments, the compound is a single-stranded modified oligonucleotide.

In certain embodiments, the nucleobase sequence of the modified oligonucleotide is at least 90%, 91%, 92%, 93%, 94%, 95%, 96%, 97%, 98%, 99% or 100% complementary with respect to an equal length portion of any one of the regions of SEQ ID NO: 1 in the sequence listing, as measured over the entirety of the modified oligonucleotide. In certain embodiments, the nucleobase sequence of the modified oligonucleotide is 100% complementary with respect to an equal length portion of any one of the regions of SEQ ID NO: 1 in the sequence listing, as measured over the entirety of the modified oligonucleotide.

In certain embodiments, at least one internucleoside linkage of the modified oligonucleotide is a modified internucleoside linkage. In certain embodiments, each internucleoside linkage is a phosphorothioate internucleoside linkage.

In certain embodiments, at least one nucleoside of the modified oligonucleotide includes a modified sugar. In certain embodiments, at least one modified sugar is a bicyclic sugar. In certain embodiments, at least one modified sugar includes a 2'-O-methoxyethyl, a 2'-O-methyl and/or a 4'-(CH₂)ₙ-O-2' bridge (wherein n is 1 or 2).

In certain embodiments, a sugar moiety of the modified oligonucleotide includes a modified sugar that is at least one bicyclic sugar. In certain embodiments, at least one modified sugar is LNA, GuNA, ALNA [Ms], ALNA [mU], ALNA [ipU], ALNA [Oxz], and/or ALNA [Trz].

In certain embodiments, at least one nucleoside of the modified oligonucleotide includes a modified nucleobase. In certain embodiments, a modified nucleobase is a 5-methylcytosine.

In certain embodiments, the modified oligonucleotide is a gapmer, including: a) a gap segment consisting of linked deoxynucleosides; b) a 5' wing segment consisting of linked nucleosides; and c) a 3' wing segment consisting of linked nucleosides. The gap segment is positioned between the 5' wing segment and the 3' wing segment, and the nucleosides of the wing segments each include a modified sugar such as a 2'-O-methyl modified sugar, a 2'-O-methoxyethyl modified sugar or a bicyclic sugar.

In certain embodiments, the modified oligonucleotide is a gapmer, including: a) a gap segment that includes one or two nucleosides that each includes a modified sugar such as a 2'-O-methyl modified sugar or a 2'-O-methoxyethyl modified sugar, and includes other nucleosides that include no modified sugar; b) a 5' wing segment that includes linked nucleosides; and c) a 3' wing segment that includes linked nucleosides. The gap segment is positioned between the 5' wing segment and the 3' wing segment, and the nucleosides of the wing segments each include a modified sugar such as a 2'-O-methyl modified sugar, a 2'-O-methoxyethyl sugar or a bicyclic sugar.

In certain embodiments, the modified oligonucleotide is a gapmer, including: a) a gap segment that includes 6, 7, 8, 9, 10, 11, 12, 13, 14, 15 or 16 nucleosides and in which all the nucleosides are nucleosides containing no modified sugar, or one or two of the nucleosides each contain a modified sugar such as a 2'-O-methyl modified sugar or 2'-O-methoxyethyl modified sugar and the others of the nucleosides contain no modified sugar; b) a 5' wing segments that includes 2, 3, 4, 5, 6 or 7 nucleosides; and c) a 3' wing segments that includes 2, 3, 4, 5, 6, 7 or 8 nucleosides.

Here, the gap segment is positioned between the 5' wing segment and the 3' wing segment; the nucleosides of the wing segments each include a 2'-O-methyl modified sugar, a 2'-O-methoxyethyl sugar or a bicyclic sugar; internucleoside linkages of the modified oligonucleotide include a phosphorothioate linkage; and part or all of cytosine in the modified oligonucleotide may be 5'-methylcytosine.

In certain embodiments, the modified oligonucleotide is a gapmer, and the number of nucleosides forming the gapmer is 12, 13, 14, 15, 16, 17, 18, 19, 20, 21, 22, 23, 24, 25, 26, 27, 28, 29 or 30.

In certain embodiments, the modified oligonucleotide is a gapmer, and includes a 2'-O-methyl, a 2'-O-methoxyethyl, and/or a 4'-(CH₂)ₙ-O-2' bridge (wherein n is 1 or 2) at a sugar moiety of a nucleoside.

In certain embodiments, the modified oligonucleotide is a gapmer, and includes LNA, GuNA, ALNA [Ms], ALNA [mU], ALNA [ipU], ALNA [Oxz], and/or ALNA [Trz] at a sugar moiety of a nucleoside.

In certain embodiments, the modified oligonucleotide is a gapmer, which is a modified oligonucleotide that consists of 12 - 30 residues and has a nucleobase sequence containing a modified oligonucleotide that is a nucleobase sequence that includes at least 8 contiguous nucleobase sequences that are complementary to an equal length portion of positions 126 - 147, 232 - 248, 1306 - 1325 or 1472 - 1495 from a 5' end of a nucleobase of a mature mRNA of DUX4 of SEQ ID NO: 1 in the sequence listing. In certain embodiments, the modified oligonucleotide is a modified oligonucleotide that consists of 12 - 30 residues, and has a nucleobase sequence containing a modified oligonucleotide that is a nucleobase sequence that includes at least 9, 10, 11, or 12 contiguous nucleobase sequences that are complementary to an equal length portion of positions 126 - 147, 232 - 248, 1306 - 1325, or 1472 - 1495 from a 5' end of a nucleobase of a mature mRNA of DUX4 of SEQ ID NO: 1 in the sequence listing.

In certain embodiments, the modified oligonucleotide is a gapmer, and is a nucleobase sequence that includes at least 8, 9, 10, 11, 12, 13, 14, 15, 16, 17, 18, 19, 20, 21 or 22 contiguous nucleobase sequences that are complementary to an equal length portion of positions 126 - 147 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a gapmer, which is a nucleobase sequence that includes at least 8, 9, 10, 11, 12, 13, 14, 15, 16, or 17 contiguous nucleobase sequences that are complementary to an equal length portion of positions 232 - 248 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a gapmer, which is a nucleobase sequence that includes at least 8, 9, 10, 11, 12, 13, 14, 15, 16, 17, 18, 19, or 20 contiguous nucleobase sequences that are complementary to an equal length portion of positions 1306 - 1325 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a gapmer, which is a nucleobase sequence that includes at least 8, 9, 10, 11, 12, 13, 14, 15, 16, 17, 18, 19, 20, 21, 22, 23, or 24 contiguous nucleobase sequences that are complementary to an equal length portion of positions 1472 - 1495 from a 5' end of a nucleobase of a mature mRNA of DUX4 of SEQ ID NO: 1 in the sequence listing, and is a modified oligonucleotide of 30 or less residues.

In certain embodiments, the modified oligonucleotide is a gapmer of a modified oligonucleotide consisting of 12 - 30 residues, and includes a nucleobase sequence that is complementary to an equal length portion of positions 126 - 147 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a gapmer of a modified oligonucleotide consisting of 12 - 30 residues, and includes a nucleobase sequence that is complementary to an equal length portion of positions 233 - 248 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a gapmer of a modified oligonucleotide consisting of 12 - 30 residues, and includes a nucleobase sequence that is complementary to an equal length portion of positions 1309 - 1323 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide is a gapmer of a modified oligonucleotide consisting of 12 - 30 residues, and includes a nucleobase sequence that is complementary to an equal length portion of positions 1472 - 1495 from a 5' end of a nucleobase of a mature mRNA of DUX4 of SEQ ID NO: 1 in the sequence listing, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide includes12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues. In this case, the oligonucleotide included in the 5' wing segment and/or the 3' wing segment includes at least one nucleoside that includes at least one modified sugar selected from GuNA, ALNA [Ms], ALNA [mU], ALNA [ipU], ALNA [Oxz] and ALNA [Trz], and may further include a 2'-O-methoxyethyl modified sugar and/or a 2'-O-methyl modified sugar.

In certain embodiments, the modified oligonucleotide is a gapmer of a modified oligonucleotide consisting of 12 - 30 residues, and is a nucleobase sequence that is complementary to an equal length portion of positions 1480 - 1495 from a 5' end of a nucleobase of a mature mRNA of DUX4 of SEQ ID NO: 1 in the sequence listing, and consists of a nucleobase sequence that has at a 3' end a complementary base of a base of a position 1480 from a 5' end of a nucleobase of SEQ ID NO: 1, and is at least 90%, 91%, 92%, 93%, 94%, 95%, 96%, 97%, 98%, 99% or 100% complementary to SEQ ID NO: 1 in the sequence listing in the equal length portion. Further, in certain embodiments, the modified oligonucleotide consists of 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues.

In certain embodiments, the modified oligonucleotide provided herein is a gapmer, and targets any one of the following regions of SEQ ID NO: 1 in the sequence listing: positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from the 5' end. In certain embodiments, the modified oligonucleotide provided herein is a gapmer, and targets any one of the following regions of SEQ ID NO: 1 in the sequence listing: positions 128 - 143, 232 - 247, 233 - 248, 1309 - 1323 and 1480 - 1495 from the 5' end.

In certain embodiments, the modified oligonucleotide provided herein is a gapmer, and has a nucleobase sequence that includes a complementary region that contains at least 8 contiguous nucleobases that are complementary with respect to a target region. In certain embodiments, the modified oligonucleotide provided herein has a nucleobase sequence that includes a complementary region that contains at least 8 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein is a gapmer, and has a nucleobase sequence that includes a complementary region that contains at least 8 contiguous nucleobases that are complementary with respect to a target region, and the target region targets positions 128 - 143, 232 - 247, 233 - 248, 1309 - 1323 or 1480 - 1495 from the 5' end of SEQ ID NO: 1 in the sequence listing.

In certain embodiments, the modified oligonucleotide provided herein is a gapmer, and has a nucleobase sequence that includes a complementary region that contains at least 10 contiguous nucleobases that are complementary with respect to a target region. In certain embodiments, the modified oligonucleotide provided herein has a nucleobase sequence that includes a complementary region that contains at least 10 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein is a gapmer, and has a nucleobase sequence that includes a complementary region that contains at least 10 contiguous nucleobases that are complementary with respect to a target region, and the target region targets positions 128 - 143, 232 - 247, 233 - 248, 1309 - 1323 or 1480 - 1495 from the 5' end of SEQ ID NO: 1 in the sequence listing.

In certain embodiments, the modified oligonucleotide provided herein is a gapmer, and has a nucleobase sequence that includes a complementary region that contains at least 12 contiguous nucleobases that are complementary with respect to a target region. In certain embodiments, the modified oligonucleotide provided herein has a nucleobase sequence that includes a complementary region that contains at least 12 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein is a gapmer, and has a nucleobase sequence that includes a complementary region that contains at least 12 contiguous nucleobases that are complementary with respect to a target region, and the target region targets positions 128 - 143, 232 - 247, 233 - 248, 1309 - 1323 or 1480 - 1495 from the 5' end of SEQ ID NO: 1 in the sequence listing.

In certain embodiments, the modified oligonucleotide provided herein is a gapmer, and has a nucleobase sequence that includes a complementary region that contains at least 14 contiguous nucleobases that are complementary with respect to a target region. In certain embodiments, the modified oligonucleotide provided herein has a nucleobase sequence that includes a complementary region that contains at least 14 contiguous nucleobases that are complementary with respect to a target region, and the target region targets positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of SEQ ID NO: 1 in the sequence listing. In certain embodiments, the modified oligonucleotide provided herein is a gapmer, and has a nucleobase sequence that includes a complementary region that contains at least 14 contiguous nucleobases that are complementary with respect to a target region, and the target region targets positions 128 - 143, 232 - 247, 233 - 248, 1309 - 1323 or 1480 - 1495 from the 5' end of SEQ ID NO: 1 in the sequence listing.

In certain embodiments, the modified oligonucleotide is a gapmer, and is a modified oligonucleotide that consists of a nucleobase sequence that is complementary to an equal length portion of positions 126 - 141, 126 - 143, 127 - 142, 127 - 143, 127 - 144, 127 - 146, 128 - 143, 128 - 144, 128 - 147, 232 - 245, 232 - 247, 233 - 246, 233 - 247, 233 - 248, 234 - 247, 234 - 248, 1304 - 1323, 1306 - 1321, 1306 - 1324, 1307 - 1323, 1307 - 1324, 1307 - 1325, 1307 - 1326, 1308 - 1323, 1308 - 1324, 1308 - 1322, 1308 - 1325, 1309 - 1323, 1309 - 1324, 1309 - 1325, 1309 - 1322, 1310 - 1323, 1310 - 1324, 1472 - 1485, 1472 - 1486, 1472 - 1487, 1472 - 1488, 1473 - 1487, 1473 - 1488, 1473 - 1489, 1474 - 1488, 1474 - 1489, 1475 - 1490, 1476 - 1490, 1476 - 1491, 1476 - 1495, 1477 - 1495, 1478 - 1496, 1479 - 1495, 1479 - 1496, 1479 - 1498, 1480 - 1494, 1480 - 1495, 1480 - 1496, 1480 - 1497 or 1480 - 1499 from a 5' end of a nucleobase of a mature mRNA of DUX4 of SEQ ID NO: 1 in the sequence listing. In certain embodiments, the modified oligonucleotide is a gapmer, and is a modified oligonucleotide that consists of a nucleobase sequence that is complementary to an equal length portion of positions 128 - 143, 232 - 247, 233 - 248, 1309 - 1323, 1480 - 1495 from a 5' end of a nucleobase of a mature mRNA of DUX4 of SEQ ID NO: 1 in the sequence listing.

In certain embodiments, the modified oligonucleotide is a gapmer, and is a modified oligonucleotide consisting of a nucleobase sequence set forth in any one of SEQ ID NOs: 2 - 4, 7 - 64, 69 - 97, and 102 - 109 in the sequence listing. Further, in certain embodiments, the modified oligonucleotide is a gapmer, and is a modified oligonucleotide consisting of a nucleobase sequence set forth in any one of SEQ ID NOs: 2 - 4, 75, and 78 in the sequence listing.

In certain embodiments, the modified oligonucleotide is a gapmer, and is a modified oligonucleotide set forth in any one of Compound Nos. 1 - 112, 114 - 132, and 137 - 246. In certain embodiments, the modified oligonucleotide is a gapmer, and is a modified oligonucleotide set forth in any one of Compound Nos. 1 - 3, 123, 157, 204, 221, and 231.

In the present specification, in symbols such as "Gls" representing nucleotides, an abbreviation shown at a left position means a nucleobase portion, an abbreviation shown at a center position means a sugar moiety, and an abbreviation shown at a right position means a mode of an internucleoside linkage.

In certain embodiments, the modified oligonucleotide is a gapmer, and is represented by the formula:

GlsMlsMlsTdsAdsGdsAdsCdsAdsGdsCdsGdsTdsMlsGlsGl,

wherein nucleobases are represented according to the following symbols:
   A = adenine, T = thymine, G = guanine, C = cytosine, and M = 5-methylcytosine;
sugar moieties are represented according to the following symbols:
   l = LNA, and d = 2'-deoxyribose;
and internucleoside linkages are represented according to the following symbol:
   s = phosphorothioate.

In certain embodiments, the modified oligonucleotide is a gapmer, and is represented by the formula:

GmsMmsMmsTdsAdsGdsAdsCdsAdsGdsCdsGdsTdsMmsGmsGm,

wherein nucleobases are represented according to the following symbols:
   A = adenine, T = thymine, G = guanine, C = cytosine, and M = 5-methylcytosine;
sugar moieties are represented according to the following symbols:
   m = ALNA [Ms], and d = 2'-deoxyribose;
and internucleoside linkages are represented according to the following symbol:
   s = phosphorothioate.

In certain embodiments, the modified oligonucleotide is a gapmer, and is represented by the formula:

GmsMmsAmsGdsTdsTdsCdsTdsCdsCdsGdsCdsGmsGmsTm,

wherein nucleobases are represented according to the following symbols:
   A = adenine, T = thymine, G = guanine, C = cytosine, and M = 5-methylcytosine;
sugar moieties are represented according to the following symbols:
   m = ALNA [Ms], and d = 2'-deoxyribose;
and internucleoside linkages are represented according to the following symbol:
   s = phosphorothioate.

In certain embodiments, the modified oligonucleotide is a gapmer, and is represented according to the formula:

MlsGlsAlsGdsAdsTdsTdsCdsCdsCdsGdsCdsCdsGlsGlsTl,

wherein nucleobases are represented according to the following symbols:
   A = adenine, T = thymine, G = guanine, C = cytosine, and M = 5-methylcytosine;
sugar moieties are represented according to the following symbols:
   l = LNA, and d = 2'-deoxyribose;
and internucleoside linkages are represented according to the following symbol:
   s = phosphorothioate.

Certain embodiments provide a method for reducing DUX4 mRNA and/or DUX4 protein levels (for example, intramuscular levels) in an animal with a DUX4-related disease, the method including: administering to the animal an effective amount of a modified oligonucleotide with respect to DUX4.

Certain embodiments provide a method for reducing DUX4 mRNA and/or DUX4 protein levels (for example, intramuscular levels) in an animal with FSHD, the method including: administering to the animal an effective amount of a modified oligonucleotide with respect to DUX4. Further, certain embodiments provide a method of reducing muscle damage and/or improving motor function in an animal with FSHD, the method including: administering to the animal an effective amount of a modified oligonucleotide with respect to DUX4.

Certain embodiments provide a method for treating, that is, therapeutically treating, preventing, ameliorating, or alleviating, FSHD, the method including: administering to an animal an effective amount of a modified oligonucleotide with respect to DUX4.

Certain embodiments provide a method for preventing, ameliorating, or alleviating various symptoms of FSHD.(for example, facial muscle weakness, eyelid ptosis, inability to whistle, decreased facial expression changes, melancholy or angry facial expression, difficulty in pronouncing words, scapular weakness (deformations such as winged shoulder blades and slopping shoulders), lower limb weakness, hearing loss, and heart diseases), the method including: administering to an animal an effective amount of a modified oligonucleotide with respect to DUX4.

Certain embodiments provide a method for reducing levels (for example, the levels in B cells in the blood) of mRNA and/or protein generated by fusion of the DUX4 gene with an IGH gene in an animal with B-cell acute lymphocytic leukemia or the like, the method including: administering to an animal an effective amount of a modified oligonucleotide with respect to DUX4. Further, certain embodiments provide a method for treating, that is, therapeutically treating, preventing, ameliorating, or alleviating, B-cell acute lymphocytic leukemia or the like, the method including: administering to an animal an effective amount of a modified oligonucleotide with respect to DUX4.

Certain embodiments provide a method for reducing levels (for example, the levels in sarcoma) of mRNA and/or protein generated by fusion of the DUX4 gene with a CIC gene in an animal with differentiated round cell sarcoma or the like, the method including: administering to an animal an effective amount of a modified oligonucleotide with respect to DUX4. Further, certain embodiments provide a method for treating, that is, therapeutically treating, preventing, ameliorating, or alleviating, differentiated round cell sarcoma or the like, the method including: administering to an animal an effective amount of a modified oligonucleotide with respect to DUX4.

Certain embodiments provide a method for reducing levels (for example, the levels in sarcoma) of mRNA and/or protein generated by fusion of the DUX4 gene with an EWSR1 gene in an animal with fetal rhabdomyosarcoma or the like, the method including: administering to an animal an effective amount of a modified oligonucleotide with respect to DUX4. Further, certain embodiments provide a method for treating, that is, therapeutically treating, preventing, ameliorating, or alleviating, fetal rhabdomyosarcoma or the like, the method including: administering to an animal an effective amount of a modified oligonucleotide with respect to DUX4.

Certain embodiments provide a method for therapeutically treating, preventing, ameliorating, or alleviating a DUX4-related disease with reduced side effects, the method including: administering to an animal a modified oligonucleotide with respect to DUX4. In certain embodiments, side effects include injection site reactions, liver function test abnormalities, renal function abnormalities, liver toxicity (histopathological abnormal findings: degenerative necrosis of hepatocytes, hepatocellular hypertrophy, and the like), renal toxicity (histopathological abnormal findings, and the like), central nervous system abnormalities, myopathies, and malaise. For example, an elevated level of ALT, AST, γ-GTP, GLDH, ALP (alkaline phosphatase) or TBA (total bile acids) in blood may indicate hepatotoxicity or a liver function abnormality. For example, elevated bilirubin can indicate liver toxicity or liver function abnormality. Further, an elevated urinary protein level, or an elevated level of creatinine or UN in the blood may indicate renal toxicity or a renal function abnormality.

Certain embodiments provide use of any compound described herein or a pharmaceutical composition containing the compound in manufacture of a medicament for use in any of the treatment methods described herein. For example, certain embodiments provide use of a compound described herein or a pharmaceutical composition containing the compound in manufacture of a medicament for treating, that is, therapeutically treating, preventing, delaying or ameliorating FSHD. Certain embodiments provide use of a compound described herein or a pharmaceutical composition containing the compound in manufacture of a medicament for inhibiting expression of DUX4 and for treating, that is, therapeutically treating, preventing, delaying or ameliorating a DUX4-related disease and/or its symptoms. Certain embodiments provide use of a compound described herein or a pharmaceutical composition containing the compound in manufacture of a medicament for reducing expression of DUX4 in animals. Certain embodiments provide use of a compound described herein or a pharmaceutical composition containing the compound in manufacture of a medicament for alleviating myotonia by preferentially reducing a level of DUX4-FL mRNA (for example, the level in muscles) in animals. Certain embodiments provide use of a compound described herein or a pharmaceutical composition containing the compound in manufacture of a medicament for treating an animal with FSHD. Certain embodiments provide use of a compound described herein or a pharmaceutical composition containing the compound in manufacture of a medicament for treating one or more symptoms and outcomes associated with the development of FSHD including muscle stiffness, myotonia, facial muscle weakness, eyelid ptosis, inability to whistle, decreased facial expression changes, melancholy or angry facial expression, difficulty in pronouncing words, scapular weakness (deformations such as winged shoulder blades and slopping shoulders), lower limb weakness, hearing loss, and heart diseases. Certain embodiments provide use of a compound described herein or a pharmaceutical composition containing the compound in manufacture of a medicament for preventing DUX4 protein expression by guiding DUX4 gene transcription, DUX4 mRNA translation, and DUX4 mRNA cleavage.

Certain embodiments provide a kit for treating, that is, therapeutically treating, preventing, or ameliorating FSHD as described herein, the kit including: a) a compound described herein, and, optionally, b) an additional agent or therapy described herein. The kit can further include an instruction or a label for using the kit for treating, that is, therapeutically treating, preventing, or ameliorating FSHD

Certain embodiments provide use of any compound described herein or a pharmaceutical composition containing the compound in manufacture of a medicament for use in any of the treatment methods described herein. For example, certain embodiments provide use of a compound described herein or a pharmaceutical composition containing the compound in manufacture of a medicament for treating, that is, therapeutically treating, ameliorating, or preventing FSHD. Certain embodiments provide use of a compound described herein or a pharmaceutical composition containing the compound in manufacture of a medicament for inhibiting expression of DUX4 and for treating, that is, therapeutically treating, preventing, delaying or ameliorating a DUX4-related disease and/or its symptoms. Certain embodiments provide use of a compound described herein or a pharmaceutical composition containing the compound in manufacture of a medicament for reducing expression of DUX4 in animals. Certain embodiments provide use of a compound described herein or a pharmaceutical composition containing the compound in manufacture of a medicament for alleviating myotonia by preferentially reducing a level of DUX4-FL mRNA (for example, the level in muscles) in animals. Certain embodiments provide use of a compound described herein or a pharmaceutical composition containing the compound in manufacture of a medicament for treating an animal with FSHD. Certain embodiments provide a compound described herein or a pharmaceutical composition containing the compound for treating one or more symptoms and outcomes associated with the development of FSHD including muscle stiffness, myotonia, facial muscle weakness, eyelid ptosis, inability to whistle, decreased facial expression changes, melancholy or angry facial expression, difficulty in pronouncing words, scapular weakness (deformations such as winged shoulder blades and slopping shoulders), lower limb weakness, hearing loss, and heart diseases. Certain embodiments provide a modified oligonucleotide having a nucleobase sequence consisting of a nucleobase sequence of SEQ ID NOs: 2 - 4, 7 - 64, 69 - 97 or 102 - 109 in the sequence listing, or a compound of Compound Nos. 1 - 112, 114 - 132 or 137 - 246.

In certain embodiments, a modified oligonucleotide has a nucleobase sequence that, when described in a 5' to 3' direction, includes a reverse complementary strand of a target segment of a target nucleic acid targeted by the modified oligonucleotide. In certain such embodiments, a modified oligonucleotide has a nucleobase sequence that, when described in a 5' to 3' direction, includes a reverse complementary strand of a target segment of a target nucleic acid targeted by the modified oligonucleotide.

In certain embodiments, a modified oligonucleotide described herein that targets DUX4 has a length of 12 - 30 nucleotides. In other words, in some embodiments, a modified oligonucleotide is a linked nucleobase of 12 - 30 residues. In other embodiments, a modified oligonucleotide includes a modified oligonucleotide consisting of a linked nucleobase of 12 - 29 residues, 12 - 28 residues, 12 - 27 residues, 12 - 26 residues, 12 - 25 residues, 12 - 24 residues, 12 - 23 residues, 12 - 22 residues, 12 - 21 residues, 12 - 20 residues, 12 - 19 residues, 12 - 18 residues, 12 - 17 residues, 12 - 16 residues, 12 - 15 residues, 12 - 14 residues, 13 - 29 residues, 13 - 28 residues, 13 - 27 residues, 13 - 26 residues, 13 - 25 residues, 13 - 24 residues, 13 - 23 residues, 13 - 22 residues, 13 - 21 residues, 13 - 20 residues, 13 - 19 residues, 13 - 18 residues, 13 - 17 residues, 13 - 16 residues, 13 - 15 residues, 13 - 14 residues, 14 - 29 residues, 14 - 28 residues, 14 - 27 residues, 14 - 26 residues, 14 - 25 residues, 14 - 24 residues, 14 - 23 residues, 14 - 22 residues, 14 - 21 residues, 14 - 20 residues, 14 - 19 residues, 14 - 18 residues, 14 - 17 residues, 14 - 16 residues, or 14 - 15 residues. In certain such embodiments, a modified oligonucleotide includes a modified oligonucleotide consisting of a linked nucleobase of 12, 13, 14, 15, 16, 17, 18, 19, 20, 21, 22, 23, 24, 25, 26, 27, 28, 29 or 30 residues in length or a range defined by any two of the above values. In certain embodiments, a modified oligonucleotide of any one of these lengths includes at least 8 contiguous nucleobases of a nucleobase sequence described in any one of contiguous nucleobases (for example, SEQ ID NOs: 2 - 4, 7 - 64, 69 - 97 or 102 - 109 in the sequence listing) of at least 8, at least 9, at least 10, at least 11, at least 12, at least 13, at least 14, at least 15, at least 16, at least 17, at least 18 or at least 19 residues of a nucleobase sequence of any one of the exemplary modified oligonucleotides described herein.

It is possible to increase or decrease the length of an antisense compound such as an antisense oligonucleotide and/or introduce a mismatch base without eliminating activity. For example, according to Woolf et al., (Proc. Natl. Acad. Sci. USA 89: 7305 - 7309, 1992), a series of antisense oligonucleotides of 13 - 25 nucleobases in length were tested for their ability to induce cleavage of a target RNA in an oocyte injection model. An antisense oligonucleotide of 25 nucleobases in length with 8 or 11 mismatch bases near the ends of the antisense oligonucleotide was able to guide specific cleavage of the target mRNA, although to a lesser extent than an antisense oligonucleotide that contained no mismatches. Similarly, it is reported that target-specific cleavage was achieved using antisense oligonucleotides of 13 nucleobases including those with 1 or 3 mismatches.

According to Gautschi et al., (J. Natl. Cancer Inst. 93: 463 - 471, March 2001), an oligonucleotide having 100% complementarity with respect to bc1-2 mRNA and having 3 mismatches with respect to bc1-xL mRNA demonstrated an ability to reduce expression of both bc1-2 and bc1-xL in vitro and in vivo. Further, it is reported that this oligonucleotide demonstrated strong anti-tumor activity in vivo.

### Target Nucleic Acid, Target Region and NSequence

Examples of nucleotide sequence encoding DUX4 include, but are not limited to, the following sequences.
- A sequence set forth in GenBank accession number NM_001293798.2 (incorporated herein as SEQ ID NO: 1 in the sequence listing). A splicing variant of SEQ ID NO: 1 in the sequence listing is also referred to as DUX4-FL1 or mature mRNA of DUX4.
- A sequence set forth in GenBank accession number NM_001306068.2 (incorporated herein as SEQ ID NO: 5 in the sequence listing). A splicing variant of SEQ ID NO: 5 in the sequence listing is also referred to as DUX4-FL2.
- A sequence set forth in GenBank accession number NM_001363820.1 (incorporated herein as SEQ ID NO: 6 in the sequence listing). A splicing variant of SEQ ID NO: 6 in the sequence listing is also referred to as DUX4-s.
- SNPs of the above splicing variants.

The sequences set forth in SEQ ID NOs in the sequence listing in the Examples contained herein is independent of any modification with respect to a sugar moiety, an internucleoside linkage, or a nucleobase. Therefore, a modified oligonucleotide defined by a SEQ ID NO in the sequence listing can include, independently, one or more modifications with respect to a sugar moiety, an internucleoside linkage, or a nucleobase. A modified oligonucleotide described by a compound number indicates a combination of a nucleobase sequence and a motif.

In certain embodiments, a target region is a structurally defined region of a target nucleic acid. For example, a target region can include one or more of 3' UTR, 5' UTR, an exon, an intron, an exon/intron junction, a coding region, a translation initiation region, a translation termination region, or other defined nucleic acid regions. A structurally defined region for DUX4 can be obtained by an accession number from a sequence database such as NCBI, and such information is incorporated herein by reference. In certain embodiments, a target region can include a sequence from a 5' target site of one target segment within the target region to a 3' target site of another target segment within the target region.

Targeting includes determination of at least one target segment to which a modified oligonucleotide hybridizes such that a desired effect occurs. In certain embodiments, a desired effect is a reduction in an mRNA target nucleic acid level. In certain embodiments, a desired effect is reduction in a level of protein encoded by a target nucleic acid or a phenotypic change associated with a target nucleic acid.

A target region can contain one or more target segments. Multiple target segments within a target region may overlap. Alternatively, they may be non-overlapping. In certain embodiments, target segments within a target region are separated by no more than about 300 nucleotides. In certain embodiments, target segments within a target region are separated by a number of nucleotides of the target nucleic acid, the number being, or being about, or being no more than, or being no more than about 250, 200, 150, 100, 90, 80, 70, 60, 50, 40, 30, 20, or 10, or being in a range defined by any two of the preceding numbers. In certain embodiments, target segments within a target region are separated by no more than, or no more than about, 5 nucleotides on the target nucleic acid. In certain embodiments, target segments are contiguous. A target region defined by a range having a starting nucleic acid that is either a 5' target site or a 3' target site listed herein is contemplated.

A suitable target segment can be found in a 5' UTR, a coding region, a 3' UTR, an intron, an exon, or an exon/intron junction. A target segment containing a start codon or a stop codon is also a suitable target segment. A suitable target segment can specifically exclude a certain structurally defined region such as a start codon or a stop codon.

Determination of a suitable target segment can include a comparison of a sequence of a target nucleic acid to other sequences throughout a genome. For example, the BLAST algorithm can be used to identify regions of similarity between different nucleic acids. This comparison can prevent selection of a modified oligonucleotide sequence that can hybridize in a nonspecific manner to a sequence other than a selected target nucleic acid (that is, a non-target sequence or an off-target sequence).

There can be variation in activity (for example, as determined by percent reduction of a target nucleic acid level) of a modified oligonucleotide in an active target region. In certain embodiments, a decrease in DUX4 mRNA level is an indicator of inhibition of DUX4 protein expression. A decrease in DUX4 protein level is also an indicator of inhibition of target mRNA expression. Further, phenotypic changes, such as reducing myotonia and reducing myopathy, can be indicators of inhibition of DUX4 mRNA and/or protein expression.

### Hybridization

In some embodiments, hybridization occurs between a modified oligonucleotide disclosed herein and a DUX4 nucleic acid. The most common mechanism of hybridization involves hydrogen bonding (for example, Watson-Crick, Hoogsteen or reversed Hoogsteen hydrogen bonding) between complementary nucleobases of nucleic acid molecules. Hybridization strength can be represented by a melting temperature Tm. Tm is the temperature at which 50% of a hybridized double-stranded nucleic acid dissociates into a single-stranded nucleic acid. Tm varies depending on a salt condition of a solution, a length of a nucleic acid, a base sequence, and the like, but a higher Tm value indicates stronger hybridization.

Hybridization can occur under various conditions. A stringent condition is sequencedependent and is determined by the nature and composition of the nucleic acid molecules to be hybridized.

A method for determining whether or not a sequence is specifically hybridizable to a target nucleic acid is well known in the art (Sambrooke and Russell, Molecular Cloning: A Laboratory Manual, 3rd Ed., 2001). In certain embodiments, a modified oligonucleotide provided herein is capable of specifically hybridizing to a DUX4 nucleic acid.

### Complementarity

A modified oligonucleotide and a target nucleic acid are complementary to each other when a sufficient number of nucleobases of the modified oligonucleotide can hydrogen bond with corresponding nucleobases of the target nucleic acid such that a desired effect occurs (for example, antisense inhibition of the target nucleic acid, such as a DUX4 nucleic acid).

A modified oligonucleotide can hybridize over one or more segments of a DUX4 nucleic acid such that intervening or adjacent segments are not involved in the hybridization event (for example, a loop structure, a mismatch or a hairpin structure).

In certain embodiments, a modified oligonucleotide provided herein, or a specific portion thereof, is 90%, 91%, 92%, 93%, 94%, 95%, 96%, 97%, 98%, 99% or 100% complementary with respect to a DUX4 nucleic acid, a target region, a target segment, or a specific portion thereof. In certain embodiments, a modified oligonucleotide is at least 90%, at least 91%, at least 92%, at least 93%, at least 94%, at least 95%, at least 96%, at least 97%, at least 98%, at least 99% or 100% complementary with respect to a DUX4 nucleic acid, a target region, a target segment, or a specific portion thereof, and contains at least 8, at least 9, at least 10, at least 11, at least 12, at least 13, at least 14, at least 15, at least 16, at least 17, at least 18 or at least 19 contiguous nucleobases of a nucleobase sequence of any of the exemplary modified oligonucleotides described herein (for example, at least 8 contiguous nucleobases of a nucleobase sequence described in any one of SEQ ID NOs: 2, 3, 4, 7 - 64, 69 - 97 or 102 - 109 in the sequence listing). Percent complementarity of a modified oligonucleotide with respect to a target nucleic acid can be determined using a conventional method, and is measured over the entirety of the modified oligonucleotide.

For example, 18 of 20 nucleobases of a modified oligonucleotide are complementary with respect to a target region, and thus, the modified oligonucleotide that appears to hybridize specifically corresponds to 90 percent complementarity. In this example, the remaining noncomplementary nucleobases may be clustered or interspersed with complementary nucleobases, and need not be contiguous to each other or to the complementary nucleobases. Percent complementarity of a modified oligonucleotide with respect to a region of a target nucleic acid can be determined conventionally using the BLAST program (basic local alignment search tools) and the PowerBLAST program known in the art (Altschul et al., J. Mol. Biol., 1990, 215, 403 410; Zhang and Madden, Genome Res., 1997, 7, 649 656). Percent homology, sequence identity or complementarity, can be determined, for example, by the Gap program (Wisconsin Sequence Analysis Package, Version 8 for Unix, Genetics Computer Group, University Research Park, Madison Wis.), using default settings using the algorithm of Smith and Waterman (Adv. Appl. Math., 1981, 2, 482 489).

A site of a non-complementary nucleobase may be at a 5' end or a 3' end of a modified oligonucleotide. Alternatively, a non-complementary nucleobase (or non-complementary nucleobases) may be at an internal position of a modified oligonucleotide. When two or more non-complementary nucleobases are present, they can be either contiguous (that is, linked) or non-contiguous. In one embodiment, a non-complementary nucleobase is positioned in a wing segment of a gapmer modified oligonucleotide.

In certain embodiments, a modified oligonucleotide that has a length of, or a length of up to 12, 13, 14, 15, 16, 17, 18, 19, 20, 21, 22, 23, 24, 25, 26, 27, 28, 29 or 30 nucleobases includes no more than 3, no more than 2, or no more than 1 non-complementary nucleobase(s) with respect to a target nucleic acid, such as a DUX4 nucleic acid, or a specific portion thereof.

Modified oligonucleotides provided herein also include those that are complementary to a portion of a target nucleic acid. As used herein, a "portion" refers to a defined number of contiguous (that is, linked) nucleobases within a region or segment of a target nucleic acid. A "portion" can also refer to a defined number of contiguous nucleobases of a modified oligonucleotide. In certain embodiments, a modified oligonucleotide is complementary to at least an 8 nucleobase portion of a target segment. In certain embodiments, a modified oligonucleotide is complementary to at least a 10 nucleobase portion of a target segment. In certain embodiments, a modified oligonucleotide is complementary to at least a 15 nucleobase portion of a target segment. Also contemplated is a modified oligonucleotide that is complementary to a portion of at least 8, at least 9, at least 10, at least 11, at least 12, at least 13, at least 14, at least 15, at least 16, at least 17, at least 18, at least 19, at least 20, or more nucleobases of a target segment, or to a portion of nucleobases in a number in a range defined by any two of these values.

### Identity

A modified oligonucleotide provided herein can also have a defined percent identity with respect to a specific nucleotide sequence, a SEQ ID NO in the sequence listing, or compound represented by a specific Compound Number, or a portion thereof. As used herein, a modified oligonucleotide is identical to a sequence disclosed herein when it has the same nucleobase pairing ability. For example, a RNA that contains uracil in place of thymidine in a disclosed DNA sequence is considered identical to the DNA sequence since both uracil and thymidine pair with adenine. Shortened and lengthened versions of a modified oligonucleotide described herein and a compound having a non-identical base with respect to a modified oligonucleotide provided herein are also contemplated. The non-identical bases may be adjacent to each other or dispersed throughout the modified oligonucleotide. Percent identity of a modified oligonucleotide is calculated according to the number of bases that have identical base pairing with respect to a sequence being compared.

In certain embodiments, a modified oligonucleotide, or a portion thereof, is at least 90%, at least 91%, at least 92%, at least 93%, at least 94%, at least 95%, at least 96%, at least 97%, at least 98%, at least 99%, or 100% identical to one or more of the exemplary modified oligonucleotides disclosed herein or SEQ ID NOs in the sequence listing, or portions thereof.

### Modifications

A nucleoside is a base-sugar combination. A nucleobase (also known as base) portion of a nucleoside is normally a heterocyclic base moiety. A nucleotide is a nucleoside that further includes a phosphate group covalently linked to a sugar moiety of the nucleoside. For a nucleoside that includes a pentofuranosyl sugar, the phosphate group can be linked to the 2', 3' or 5' hydroxyl moiety of the sugar. An oligonucleotide is formed through covalent linkage of adjacent nucleosides to one another to form a linear polymer oligonucleotide. Within the oligonucleotide structure, the phosphate groups are commonly referred to as forming the internucleoside linkages of the oligonucleotide.

Modifications in a modified oligonucleotide include substitution or change with respect to internucleoside linkages, sugar moieties, or nucleobases. A modified modified oligonucleotide is often preferred over a native form because of desirable properties such as, for example, enhanced cellular uptake, enhanced affinity for nucleic acid targets, increased stability in the presence of nucleases, or increased inhibitory activity.

### Modified Internucleoside Linkages

A naturally occurring internucleoside linkage of RNA and DNA is a 3'-5' phosphodiester linkage. A modified oligonucleotide having one or more modified, that is, non-naturally occurring, internucleoside linkages is often selected over a modified oligonucleotide having naturally occurring internucleoside linkages because of desirable properties such as, for example, enhanced cellular uptake, enhanced affinity for target nucleic acids, and increased stability in the presence of nucleases.

An oligonucleotide having modified internucleoside linkages include internucleoside linkages that retain a phosphorus atom and internucleoside linkages that do not have a phosphorus atom. Representative phosphorus containing internucleoside linkages include, but are not limited to, one of more of phosphodiesters, phosphotriesters, methylphosphonates, phosphoramidate, and phosphorothioates. Methods for preparing phosphorous-containing and non-phosphorous-containing linkages are well known.

In certain embodiments, a modified oligonucleotide targeting a DUX4 nucleic acid includes one or more modified internucleoside linkages. In certain embodiments, the modified internucleoside linkages are phosphorothioate linkages. In certain embodiments, internucleoside linkages of a modified oligonucleotide are each a phosphorothioate internucleoside linkage.

### Modified Sugar Moieties

As the modified oligonucleotide of the present invention, a modified oligonucleotide in which at least one nucleoside contains a modified sugar is preferably used. In the present invention, the term "modified sugar" refers to a sugar in which a sugar moiety is modified, and a modified oligonucleotide containing one or more such modified sugars has advantageous characteristics such as enhanced nuclease stability and increased binding affinity. Preferably, at least one of the modified sugars has a bicyclic sugar or a substituted sugar moiety.

Examples of nucleosides having a modified sugar include, but are not limited to, nucleosides containing 5'-vinyl, 5'-methyl (R or S), 4'-S, 2'-F, 2'-OCH₃, 2'-OCH₂CH₃, 2'-OCH₂CH₂F and 2'-O(CH₂)₂OCH₃ substituents. The substituent at the 2' position can also be selected from allyl, amino, azido, thio, O-allyl, O-C₁ - C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ) and O-CH₂-C(=O)-N(R₁)-(CH₂)₂-N(Rₘ)(Rₙ) (wherein Rₗ, Rₘ and Rₙ are each independently H or substituted or unsubstituted C₁ - C₁₀ alkyl).

Examples of nucleosides having a bicyclic sugar include, but are not limited to, nucleosides that contain a bridge between the 4' and the 2' ribosyl ring atoms. In certain embodiments, an oligonucleotide provided herein includes one or more nucleosides having a bicyclic sugar, in which a bridge includes one of the following formulas: 4'-(CH₂)-O-2'(LNA); 4'-(CH₂)-S-2'; 4'-(CH₂)₂-O-2'(ENA); 4'-CH(CH₃)-O-2' and 4'-CH(CH₂OCH₃)-O-2' (and analogs thereof, see U.S. Patent No. 7,399,845); 4'-C(CH₃)(CH₃)-O-2' (and analogs thereof, see WO 2009/006478); 4'-CH₂-N(OCH₃)-2' (and analogs thereof; see WO 2008/150729); 4'-CH₂-ON(CH₃)-2' (and analogs thereof; see US 2004-0171570); 4'-CH₂-N(R)-O-2' (wherein R is H, C₁ - C₁₂ alkyl or a protecting group) (see U.S. Patent No. 7,427,672); 4'-CH₂-C(H)(CH₃)-2' (and analogs thereof; see Chattopadhyaya et al., J. Org. Chem., 2009, 74, 118 - 134); and 4'-CH₂-C(=CH₂)-2' (and analogues thereof; see WO 2008/154401).

Additional nucleosides having a bicyclic sugar are reported in published literatures (see, for example: Srivastava et al., J. Am. Chem. Soc., 2007, 129 (26) 8362 - 8379; Frieden et al., Nucleic Acids Research, 2003, 21, 6365 - 6372; Elayadi et al., Curr. Opinion Invens. Drugs, 2001, 2, 558 - 561; Braasch et al., Chem. Biol., 2001, 8, 1 - 7; Orum et al., Curr. Opinion Mol. Ther., 2001, 3, 239 - 243; Wahlestedt et al., Proc. Natl. Acad. Sci. U.S.A., 2000, 97, 5633 - 5638; Singh et al., Chem. Commun., 1998, 4, 455 - 456; Koshkin et al., Tetrahedron, 1998, 54, 3607 - 3630; Kumar et al., Bioorg. Med. Chem. Lett., 1998, 8, 2219 - 2222; Singh et al.; U.S. Patent NOs 7,399,845; 6,770,748; 6,525,191; and 6,268,490; US 2008-0039618; US 2007-0287831; US 2004-0171570; US 2009-0012281; WO 2010/036698; WO 2009/067647; WO 2009/067647; WO 2007/134181; WO 2005/021570; WO 2004/106356; WO 94/14226; WO 2009/006478; WO 2008/154401; and WO 2008/150729). The above nucleosides having a bicyclic sugar can be each prepared having one or more stereochemical sugar configurations including, for example, an α-L-ribofuranose and a β-D-ribofuranose.

GuNA, a nucleoside having a bicyclic sugar, has been reported as an artificial nucleoside having a guanidine bridge (see WO 2014/046212, and WO 2017/047816). Bicyclic nucleosides ALNA [Ms], ALNA [mU], ALNA [ipU], ALNA [Trz] and ALNA [Oxz] have been reported as cross-linked artificial nucleic acid amino LNA (ALNA) (see Japanese Patent Application No. 2018-212424).

In certain embodiments, a nucleoside having a bicyclic sugar includes a bridge between the 4' and the 2' carbon atoms of a pentofuranosyl sugar moiety, and the bridge contains 1 or 1 to 4 linked groups that are each independently selected from, but are not limited to, - [C(Rₐ)(R_{b})]ₙ-, -C(Rₐ)=C(R_{b})-, -C(Rₐ)=N-, -C(=NRₐ)-, -C(= O)-, -C(=S)-, -N(Rₐ)-, -O-, - Si(Rₐ)₂- and -S(=O)ₓ-, wherein: X is 0, 1, or 2; n is 1, 2, 3, or 4; Ra and R_{b} are each, independently, H, a protecting group, hydroxyl, C₁ - C₁₂ alkyl, substituted C₁ - C₁₂ alkyl, C₂ - C₁₂ alkenyl, substituted C₂ - C₁₂ alkenyl, C₂ - C₁₂ alkynyl, substituted C₂ - C₁₂ alkynyl, an aromatic ring group, a substituted aromatic ring group, a heterocyclic group, a substituted heterocyclic group, a C₅ - C₇ alicyclic group, a substituted C₅ - C₇ alicyclic group, halogen, OJ₁, NJ₁J₂, SJ₁, N₃, COOJ₁, acyl (C(=O)-H), substituted acyl, CN, sulfonyl (S(=O)₂-J₁) or sulfoxyl (S(=O)-J₁), J₁ and J₂ are each independently H, C₁ - C₁₂ alkyl, substituted C₁ - C₁₂ alkyl, C₂ - C₁₂ alkenyl, substituted C₂ - C₁₂ alkenyl, C₂ - C₁₂ alkynyl, substituted C₂ - C₁₂ alkynyl, an aromatic ring group, a substituted aromatic ring group, acyl (C(=O)-H), substituted acyl, a heterocyclic group, a substituted heterocyclic group, C₁ - C₁₂ aminoalkyl, substituted C₁ - C₁₂ aminoalkyl or a protecting group.

In certain embodiments, the bridge of the bicyclic sugar moiety is [C(Rₐ)(R_{b})]ₙ-, -[CRₐ) (R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2' (the nucleoside having a bicyclic sugar in this case is also referred to as LNA), 4'-(CH₂)₂-O-2', 4'-CH₂-O-N(R)-2' and 4'-CH₂-N(R)-O-2'-, wherein each R is, independently, H, a protecting group or C₁ - C₁₂ alkyl.

In certain embodiments, the bridge of the bicyclic sugar moiety is 4'-CH₂-O-2'-(LNA) or - CH₂-N(R)-, wherein each R is independently -SO₂-CH₃ (ALNA [Ms]), -CO-NH-CH₃ (ALNA [mU]), 1,5-dimethyl-1,2,4-triazol-3-yl (ALNA [Trz]), -CO-NH-CH(CH₃)₂ (ALNA [ipU]), or 5-methyl-2,4-oxadiazol-3-yl (ALNA [Oxz]) (Japanese Patent Application No. 2018-212424).

In certain embodiments, a nucleoside having a bicyclic sugar is further defined by an isomeric steric configuration. For example, a nucleoside containing a 4'-(CH₂)-O-2' bridge may exist in an α-L-steric configuration or a β-D-steric configuration.

In certain embodiments, nucleosides having a bicyclic sugar include those having a 4'-2' bridge, and examples of such bridges include, but are not limited to, α-L-4'-(CH₂)-O-2', β-D-4'-CH₂-O-2',4'-(CH₂)₂-O-2', 4'-CH₂-O-N(R)-2', 4'-CH₂-N(R)-O-2', 4'-CH(CH₃)-O-2', 4'-CH₂-S-2', 4'-CH₂-CH(CH₃)-2', and 4'-(CH₂)₃-2' (wherein, R is H, a protecting group, C₁ - C₁₂ alkyl, or urea or guanidine that may be substituted with C₁ - C₁₂ alkyl).

In certain embodiments, a nucleoside having a bicyclic sugar has the following formula: 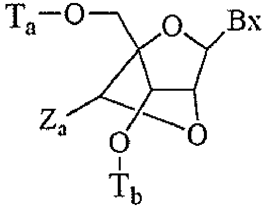 wherein
Bx is a heterocyclic base moiety;
Tₐ and T_{b} are each independently a hydrogen atom, a protecting group of a hydroxyl group, a phosphate group that may be substituted, a phosphorus moiety, a covalent attachment to a support, or the like; and
Zₐ is C₁ - C₆ alkyl, C₂ - C₆ alkenyl, C₂ - C₆ alkynyl, substituted C₁ - C₆ alkyl, substituted C₂ - C₆ alkenyl, substituted C₂ - C₆ alkynyl, acyl, substituted acyl, substituted amide, thiol or substituted thiol.

In certain embodiments, the substituents are each independently monosubstituted or polysubstituted with a substituent independently selected from halogen, oxo, hydroxyl, OJ_{c}, NJ_{c}J_{d}, SJ_{c}, N₃, OC(=X)J_{c} and NJₑC(=X)NJ_{c}J_{d} (wherein J_{c}, J_{d} and Jₑ are each independently H, C₁ - C₆ alkyl or substituted C₁ - C₆ alkyl; and X is O or NJ_{c}).

In certain embodiments, a nucleoside having a bicyclic sugar has the following formula: 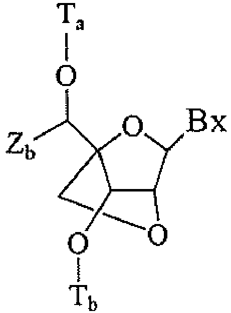 wherein
Bx is a heterocyclic base moiety;
Tₐ and T_{b} are each independently a hydrogen atom, a protecting group of a hydroxyl group, a phosphate group that may be substituted, a phosphorus moiety, a covalent attachment to a support, or the like; and
Z_{b} is C₁ - C₆ alkyl, C₂ - C₆ alkenyl, C₂ - C₆ alkynyl, substituted C₁ - C₆ alkyl, substituted C₂ - C₆ alkenyl, substituted C₂ - C₆ alkynyl or substituted acyl (C(=O)-).

In certain embodiments, a nucleoside having a bicyclic sugar has the following formula: 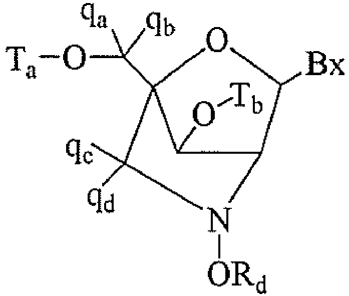 wherein
Bx is a heterocyclic base moiety;
Tₐ and T_{b} are each independently a hydrogen atom, a protecting group of a hydroxyl group, a phosphate group that may be substituted, a phosphorus moiety, a covalent attachment to a support, or the like;
R_{d} is C₁ - C₆ alkyl, substituted C₁ - C₆ alkyl, C₂ - C₆ alkenyl, substituted C₂ - C₆ alkenyl, C₂ - C₆ alkynyl or substituted C₂ - C₆ alkynyl; and
qₐ, q_{b}, q_{c} and q_{d} are each independently H, halogen, C₁ - C₆ alkyl, substituted C₁ - C₆ alkyl, C₂ - C₆ alkenyl, substituted C₂ - C₆ alkenyl, C₂ - C₆ alkynyl or substituted C₂ - C₆ alkynyl, C₁ - C₆ alkoxyl, substituted C₁ - C₆ alkoxyl, acyl, substituted acyl, C₁ - C₆ aminoalkyl or substituted C₁ - C₆ aminoalkyl.

In certain embodiments, a nucleoside having a bicyclic sugar has the following formula: 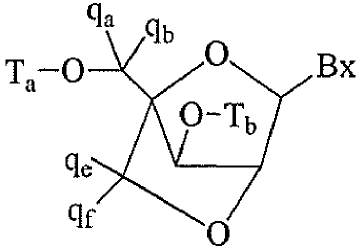 wherein
Bx is a heterocyclic base moiety;
Tₐ and T_{b} are each independently a hydrogen atom, a protecting group of a hydroxyl group, a phosphate group that may be substituted, a phosphorus moiety, a covalent attachment to a support, or the like;
qₐ, q_{b}, qₑ and q_{f} are each independently hydrogen, halogen, C₁ - C₁₂ alkyl, substituted C₁ - C₁₂ alkyl, C₂ - C₁₂ alkenyl, substituted C₂ - C₁₂ alkenyl, C₂ - C₁₂ alkynyl, substituted C₂ - C₁₂ alkynyl, C₁ - C₁₂ alkoxy, substituted C₁ - C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)-NJⱼJₖ or N(H)C(=S)NJⱼJₖ;
or qₑ and q_{f} are both =C(q_{g})(qₕ); and
q_{g} and qₕ are each independently H, halogen, C₁ - C₁₂ alkyl or substituted C₁ - C₁₂ alkyl.

Synthesis and preparation of adenine, cytosine, guanine, 5-methyl-cytosine, thymine and uracil bicyclic nucleoside (also referred to as LNA), which have 4'-CH₂-O-2' bridges, are described along with their oligomerization and nucleic acid recognition properties (Koshkin et al., Tetrahedron, 1998, 54, 3607 - 3630). Synthesis of a nucleoside having a bicyclic sugar is also described in WO 98/39352 and WO 99/14226.

Various analogs of bicyclic nucleosides having 4'-2' bridging groups such as 4'-CH₂-O-2' (the bicyclic nucleoside in this case is also referred to as LNA) and 4'-CH₂-S-2' have also been prepared (Kumar et al., Bioorg. Med. Chem. Lett., 1998, 8, 2219 - 2222). Preparation of oligodeoxyribonucleotide duplexes containing bicyclic nucleosides for use as substrates for nucleic acid polymerases has also been described (Wengel et al., WO 99/14226). Further, synthesis of 2'-amino-BNA (the bicyclic nucleoside in this case is also referred to as ALNA), that is, a conformationally restricted high-affinity oligonucleotide analog, has been described in the art (Singh et al., J. Org. Chem., 1998, 63, 10035 - 10039). Further, 2'-amino- and 2'-methylamino-BNAs have been prepared and thermal stability of duplexes with complementary RNA and DNA strands has been previously reported.

In certain embodiments, a nucleoside having a bicyclic sugar has the following formula: 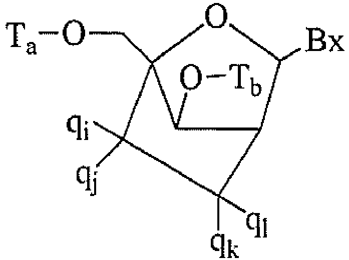 wherein
Bx is a heterocyclic base moiety;
Tₐ and T_{b} are each independently a hydrogen atom, a protecting group of a hydroxyl group, a phosphate group that may be substituted, a phosphorus moiety, a covalent attachment to a support, or the like;
qᵢ, qⱼ, qₖ and qₗ are each independently H, halogen, C₁ - C₁₂ alkyl, substituted C₁ - C₁₂ alkyl, C₂ - C₁₂ alkenyl, substituted C₂ - C₁₂ alkenyl, C₂ - C₁₂ alkynyl, substituted C₂ - C₁₂ alkynyl, C₁ - C₁₂ alkoxyl, substituted C₁ - C₁₂ alkoxyl, OJj, SJj, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qᵢ and qⱼ or qₗ and qₖ are both =C(q_{g})(qₕ), wherein q_{g} and qₕ are each independently H, halogen, C₁ - C₁₂ alkyl or substituted C₁ - C₁₂ alkyl.

One carbocyclic bicyclic nucleoside having a 4'-(CH₂)₃-2' bridge and the alkenyl analog bridge 4'-CH=CH-CH₂-2' have been described (Frier et al., Nucleic Acids Research, 1997, 25 (22), 4429 - 4443, and Albaek et al., J. Org. Chem., 2006, 71, 7731 - 7740). Synthesis and preparation of carbocyclic bicyclic nucleosides are also described, along with their oligomerization and biochemical studies (Srivastava et al., J. Am. Chem. Soc. 2007, 129 (26), 8362 - 8379).

In certain embodiments, examples of nucleosides having a bicyclic sugar include, but are not limited to,
(A) α-L-methyleneoxy (4'-CH₂-O-2') BNA,
(B) β-D-methyleneoxy (4'-CH₂-O-2') BNA,
(C) ethyleneoxy (4'-(CH₂)₂-O-2') BNA,
(D) aminooxy (4'-CH₂-O-N(R)-2') BNA,
(E) oxyamino (4'-CH₂-N(R)-O-2') BNA,
(F) methyl (methyleneoxy) (4'-CH(CH₃)-O-2') BNA (also called constrained ethyl or cEt),
(G) methylene-thio (4'-CH₂-S-2') BNA,
(H) methylene-amino (4'-CH₂-N(R)-2') BNA,
(I) methyl carbocyclic (4'-CH₂-CH(CH₃)-2') BNA,
(J) propylene carbocyclic (4'-(CH₂)₃-2') BNA, and
(K) Vvnyl BNA, as shown below: 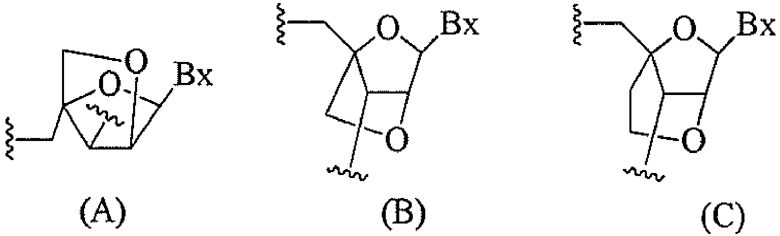 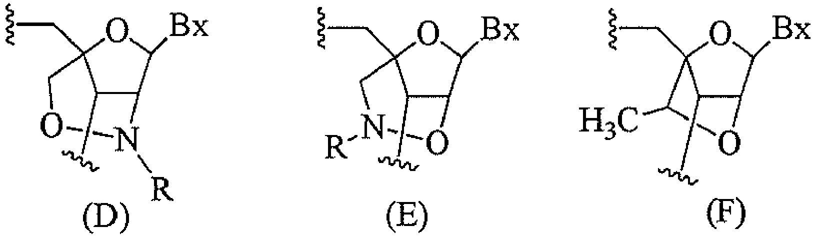 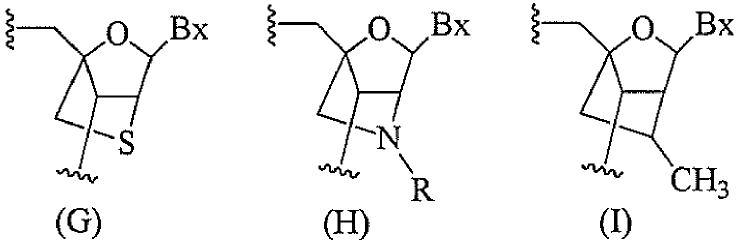 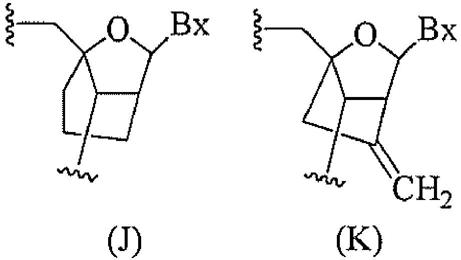
wherein Bx is a base moiety; and R is independently a protecting group, C₁ - C₆ alkyl or C₁ - C₆ alkoxy.

In certain embodiments (LNA), a nucleoside having a bicyclic sugar is a nucleoside represented by the following general formula: 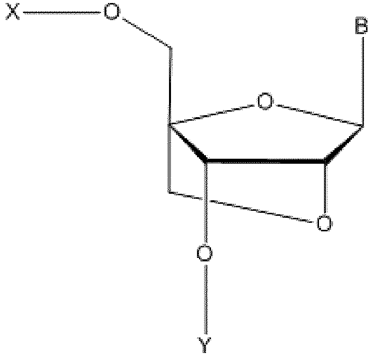 [wherein
B is a nucleobase; and
X and Y are each independently a hydrogen atom, a protecting group of a hydroxyl group, a phosphate group that may be substituted, a phosphorus moiety, or a covalent attachment to a support, or the like] (see WO 98/39352). Typical specific examples thereof include nucleotides represented by the following formula:

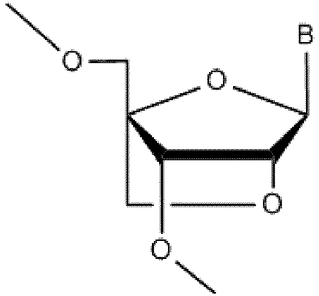

In certain embodiments (GuNA), a nucleoside having a bicyclic is a nucleoside represented by the following general formula: 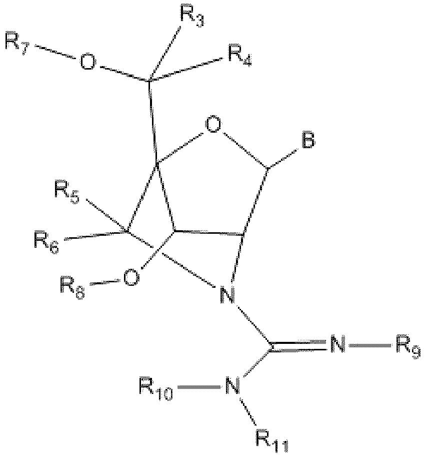 [wherein, B is a nucleobase; R₃, R₄, R₅ and R₆ are each independently a hydrogen atom or a C₁₋₆ alkyl group that may be substituted with one or more substituents; R₇ and R₈ are each independently a hydrogen atom, a protecting group of a hydroxyl group, a phosphate group that may be substituted, a phosphorus moiety, a covalent attachment to a support, or the like; and R₉, R₁₀, and R₁₁ are each independently a hydrogen atom, a C₁₋₆ alkyl group that may be substituted with one or more substituents, or a protecting group of an amino group] (see, for example, WO 2014/046212, and WO 2017/047816).

In certain embodiments (ALNA [mU]), a nucleoside containing a bicyclic sugar is a nucleoside represented by the following general formula (I): 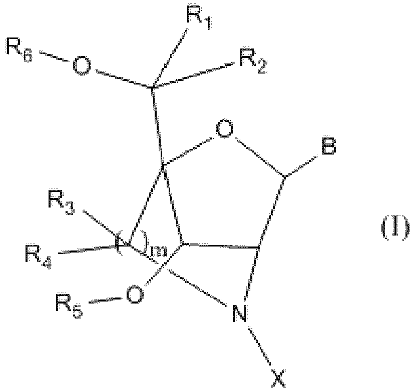 [wherein
B is a nucleobase;
R₁, R₂, R₃ and R₄ are each independently a hydrogen atom, or a C₁₋₆ alkyl group that may be substituted with one or more substituents;
R₅ and R₆ are each independently a hydrogen atom, a protecting group of a hydroxyl group, or a phosphate group that may be substituted, a phosphorus moiety, a covalent attachment to a support, or the like;
m is 1 or 2; and
X is a group represented by the following formula (II-1): 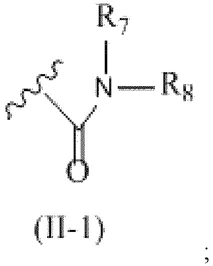 the symbol: 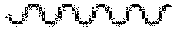
described in the formula (II-1) represents a point of attachment to a 2'-amino group;
one of R₇ and R₈ is a hydrogen atom, and the other is a methyl group that may be substituted with one or more substituents] (see, for example, Japanese Patent Application No. 2018-212424). A typical specific example is a nucleoside in which one of R₇ and R₈ is a hydrogen atom and the other is an unsubstituted methyl group.

In certain embodiments (ALNA [ipU]), a nucleoside having a bicyclic sugar is a nucleoside having the general formula (I) defined in the above ALNA [mU], and in the formula, X is a group represented by the following formula (II-1): 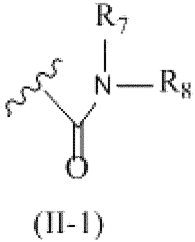 wherein one of R₇ and R₈ is a hydrogen atom, and the other is an isopropyl group that may be substituted with one or more substituents (see, for example, Japanese Patent Application No. 2018-212424). A typical specific example is a nucleoside in which one of R₇ and R₈ is a hydrogen atom and the other is an unsubstituted isopropyl group.

In certain embodiments (ALNA [Trz]), a nucleoside having a bicyclic is a nucleoside having the above general formula (I), and in the formula, X is a group represented by the following formula (II-2): 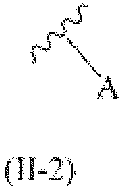 wherein A is a triazolyl group that may be substituted with one or more substituents (see, for example, Japanese Patent Application No. 2018-212424). A typical example of ALNA [Trz] is a nucleoside in which A is a triazolyl group that may have one or more methyl groups, more specifically, a 1,5-dimethyl-1,2,4-triazol-3-yl group.

In certain embodiments (ALNA [Oxz]), it is a nucleoside having the general formula (I) defined in the above ALNA [mU], and in the formula, X is a group represented by the following formula (II-2): 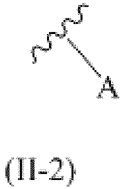 wherein A is an oxadiazolyl group that may be substituted with one or more substituents (see, for example, Japanese Patent Application No. 2018-212424). A typical specific example is a nucleoside or nucleotide in which A is an oxadiazolyl group that may have one or more methyl groups, more specifically, a 5-methyl-1,2,4-oxadiazol-3-yl group.

In certain embodiments (ALNA [Ms]), a nucleoside having a bicyclic is a nucleoside having the above general formula (I), and in the formula, X is a group represented by the following formula (II-3): 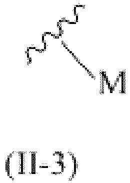 wherein M is a sulfonyl group substituted with a methyl group that may be substituted with one or more substituents (see, for example, Japanese Patent Application No. 2018-212424). A typical specific example of ALNA [Ms] is a nucleoside in which M is a sulfonyl group substituted with an unsubstituted methyl group.

In certain embodiments, a nucleoside is modified by replacement of a ribosyl ring with a sugar surrogate. Examples of such modifications include, but are not limited to, replacements of a ribosyl ring with a substitute ring system (sometimes referred to as a DNA analog), for example, a morpholino ring, a cyclohexenyl ring, a cyclohexyl ring or a tetrahydropyranyl ring, and, for example, that having one of the following formulas:

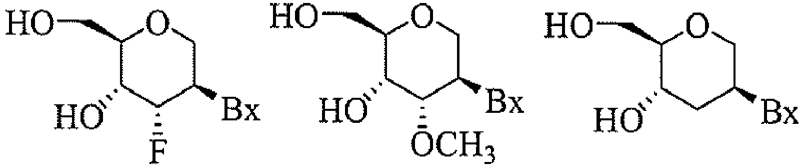

In certain embodiments, a sugar surrogate having the following formula is selected: 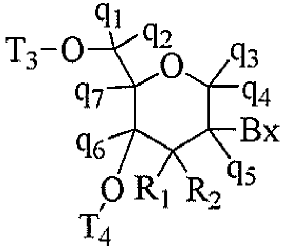 wherein
Bx is a heterocyclic base moiety;
T₃ and T₄ are each independently an internucleoside linking group linking a tetrahydropyran nucleoside analog to an oligomeric compound; or one of T₃ and T₄ is an internucleoside linking group linking a tetrahydropyran nucleoside analog to an oligomeric compound or an oligonucleotide, and the other of T₃ and T₄ is H, a hydroxyl protecting group, a linking conjugate group or a 5' or 3'-terminal group;
q₁, q₂, q₃, q₄, q₅, q₆ and q₇ are each independently H, C₁ - C₆ alkyl, substituted C₁ - C₆ alkyl, C₂ - C₆ alkenyl, substituted C₂ - C₆ alkenyl, C₂ - C₆ alkynyl or substituted C₂ - C₆ alkynyl; and
one of R₁ and R₂ is hydrogen, and the other is selected from halogen, substituted or unsubstituted alkoxy, NJ₁J₂, SJ₁, N₃, OC(=X)J₁, OC(=X)NJ₁J₂, NJ₃C(= X)NJ₁J₂ and CN (wherein X is O, S or NJ₁; and J₁, J₂ and J₃ are each independently H or C₁ - C₆ alkyl).

In certain embodiments, q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, a THP nucleoside is provided in which one of R₁ and R₂ is F. In certain embodiments, R₁ is fluoro and R₂ is H; R₁ is methoxy and R₂ is H; and R₁ is methoxyethoxy and R₂ is H.

Examples of such sugar substitutes include, but are not limited to, those known in the art as hexitol nucleic acid (HNA), altitol nucleic acid (ANA) and mannitol nucleic acid (MNA) (see Leumann, C. J., Bioorg. & Med. Chem., 2002, 10, 841 - 854).

In certain embodiments, a sugar surrogate includes a ring having more than 5 atoms and more than one heteroatom. For example, a nucleoside containing a morpholino sugar moiety and use thereof in an oligomeric compound have been reported (see, for example, Braasch et al., Biochemistry, 2002, 41, 4503 - 4510; and U.S. Patent NOs 5,698,685; 5,166,315; 5,185,444; and 5,034,506).

As used herein, the term "morpholino" means a sugar surrogate having the following structure:

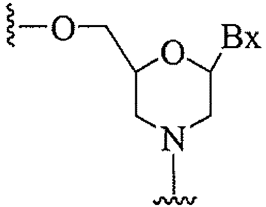

In certain embodiments, a morpholino can be modified, for example, by adding or changing various substituents from the above morpholino structure. Such a sugar surrogate is referred to herein as a "modified morpholino."

In certain embodiments, an oligonucleotide includes one or more modified cyclohexenyl nucleosides which are nucleosides having a 6-membered cyclohexenyl in place of a pentofuranosyl residue of a naturally occurring nucleoside. Examples of modified cyclohexenyl nucleosides include, but are not limited to, those described in the art (see, for example, according to sharing, WO 2010/036696 published on April 10, 2010, Robeyns et al., J. Am. Chem. Soc., 2008, 130 (6), 1979 - 1984; Horvath et al., Tetrahedron Letters, 2007, 48, 3621 - 3623; Nauwelaerts et al., J. Am. Chem. Soc., 2007, 129 (30), 9340 - 9348; Gu et al., Nucleosides, Nucleotides & Nucleic Acids, 2005, 24 (5 - 7), 993 - 998; Nauwelaerts et al., Nucleic Acids Research, 2005, 33 (8), 2452 - 2463; Robeyns et al., Acta Crystallographica, Section F: Structural Biology and Crystallization Communications, 2005, F61 (6), 585 - 586; Gu et al., Tetrahedron, 2004, 60 (9), 2111 - 2123; Gu et al., Oligonucleotides, 2003, 13 (6), 479 - 489; Wang et al., J. Org. Chem., 2003, 68, 4499 - 4505; Verbeure et al., Nucleic Acids Research, 2001, 29 (24), 4941 - 4947; Wang et al., J. Org. Chem., 2001, 66, 8478 - 82; Wang et al., Nucleosides, Nucleotides & Nucleic Acids, 2001, 20 (4 - 7), 785 - 788; Wang et al., J. Am. Chem., 2000, 122, 8595 - 8602; WO 06/047842; and WO 01/049687; text of each of these is incorporated herein by reference in its entirety).

Certain modified cyclohexenyl nucleosides have the following formula: 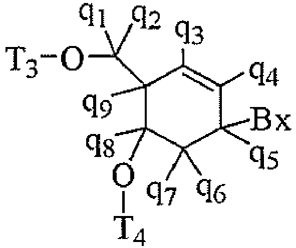 wherein
Bx is a heterocyclic base moiety;
T₃ and T₄ are each independently an internucleoside linking group linking a cyclohexenyl nucleoside analog to an oligonucleotide compound; or one of T₃ and T₄ is an internucleoside linking group linking a tetrahydropyran nucleoside analog to an oligonucleotide compound, and the other of T₃ and T₄ is H, a hydroxyl protecting group, a linking conjugate group or a 5'- or 3'-terminal group; and
q₁, q₂, q₃, q₄, q₅, q₆, q₇, q₈ and q₉ are each independently H, C₁ - C₆ alkyl, substituted C₁ - C₆ alkyl, C₂ - C₆ alkenyl, substituted C₂ - C₆ alkenyl, C₂ - C₆ alkynyl, substituted C₂ - C₆ alkynyl or other sugar substituent.

Many other bicyclic and tricyclic sugar substitute ring systems are known in the art that can be used to modify a nucleoside for incorporation into an oligonucleotide (see, for example, a review: Leumann, Christian J., Bioorg. & Med. Chem., 2002, 10, 841 - 854). Such ring systems can undergo various additional substitutions to enhance activity.

A method for preparing a modified sugar is well known to a person skilled in the art. Some representative U.S. patents that teach preparation of such modified sugars include, but are not limited to: U.S. Patent NOs. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,670,633; 5,700,920; 5,792,847 and 6,600,032, as well as WO 2005/121371, and each of these is incorporated herein by reference in its entirety.

In a nucleotide having a modified sugar moiety, a nucleobase moiety (natural, modified or a combination thereof) is maintained during hybridization with an appropriate nucleic acid target.

### Modified Nucleobases

Modification or substitution of a nucleobase (or base) is structurally distinguishable from a naturally occurring or synthetic unmodified nucleobase and is further functionally interchangeable with such an unmodified nucleobase. Both natural and modified nucleobases are capable of participating in hydrogen bonding. Such nucleobase modification can impart nuclease stability, binding affinity or some other beneficial biological properties to a modified oligonucleotide. Modified nucleobases include synthetic and natural nucleobases such as, for example, 5-methylcytosine (5-me-C). Certain nucleobase substitutions, including 5-methylcytosine substitution, are particularly useful for increasing the binding affinity of a modified oligonucleotide with respect to a target nucleic acid. For example, 5-methylcytosine substitution has been shown to increase nucleic acid duplex stability by 0.6 - 1.2 °C (Sanghvi, Y. S., Crooke, S. T. and Lebleu, B., eds., Antisense Research and Applications, CRC Press, Boca Raton, 1993, pp. 276 - 278).

Additional modified nucleobases include 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azouracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo, especially 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine, and 3-deazaguanine and 3-deazaadenine.

Heterocyclic base moieties can also include those in which a purine or pyrimidine base is replaced with other heterocycles, for example 7-deaza-adenine, 7-deazaguanosine, 2-aminopyridine and 2-pyridone. Nucleobases that are particularly useful for increasing the binding affinity of a modified oligonucleotide include 5-substituted pyrimidines, 6-azapyrimidines and N-2, N-6 and O-6 substituted purines (including 2 aminopropyladenine, 5-propynyluracil and 5-propynylcytosine).

In certain embodiments, a modified oligonucleotide targeting a DUX4 nucleic acid includes one or more modified nucleobases. In certain embodiments, a modified oligonucleotide targeting a DUX4 nucleic acid includes one or more modified nucleobases. In certain embodiments, a modified nucleobase is a 5-methylcytosine. In certain embodiments, each cytosine is 5-methylcytosine.

### Certain Modified Oligonucleotide Motifs

In certain embodiments, a modified oligonucleotide targeting to a DUX4 nucleic acid has a chemically modified subunit arranged in a pattern or a motif in order to provide the modified oligonucleotide with properties such as enhanced inhibitory activity, increased binding affinity for the target nucleic acid, or resistance to degradation by in vivo nuclease.

A chimeric modified oligonucleotide typically contains at least one modified region in order to provide increased resistance to degradation by nuclease, increased cellular uptake, increased binding affinity for the target nucleic acid, and/or increased inhibitory activity. A second region of a chimeric modified oligonucleotide can optionally serve as a substrate for intracellular endonuclease RNase H which cleaves the RNA strand of an RNA:DNA duplex.

A modified oligonucleotide having a gapmer motif is a chimeric modified oligonucleotide. In a gapmer, an internal region having multiple nucleotides that supports RNaseH cleavage is positioned between external regions having multiple nucleotides that are chemically distinct from nucleosides of the internal region. In a case of a modified oligonucleotide having a gapmer motif, a gap segment generally serves as a substrate for endonuclease cleavage, while a wing segment includes a modified nucleoside. In certain embodiments, gapmer regions are differentiated by types of sugar moieties that respectively contain different regions. In some embodiments, the types of sugar moieties that are used to differentiate gapmer regions can include:, β-D-ribonucleosides, β-D-deoxyribonucleosides, 2'-modified nucleosides (such 2'-modified nucleosides can include 2'-MOE and 2'-O-CH₃, among others) and bicyclic sugar-modified nucleosides (such bicyclic sugar-modified nucleosides can include those having LNA, GuNA, ALNA [Ms], ALNA [mU], ALNA [ipU], ALNA [Trz] and/or ALNA [Oxz]). A wing-gap-wing motif is often described as "X-Y-Z," where "X" represents a length of a 5' wing region, "Y" represents a length of a gap region, and "Z" represents a length of a 3' wing region. As used herein, a gapmer described as "X-Y-Z" has a steric configuration such that the gap segment is positioned immediately adjacent to each of the 5' wing segment and the 3' wing segment. Thus, no intervening nucleotides exist between the 5' wing segment and the gap segment or between the gap segment and the 3' wing segment. Any of the modified oligonucleotides described herein can have a gapmer motif. In some embodiments, X and Z are the same, and in other embodiments they are different. In a preferred embodiment, Y is 8 to 16 nucleotides. X, Y or Z can be any one of 1, 2, 3, 4, 5, 6, 7, 8, 9, 10, 11, 12, 13, 14, 15, 16, 17, 18, 19, 20, 25, 30 or more nucleotides. Therefore, gapmers include, but are not limited to, for example, 2-10-3, 2-14-2, 2-15-2, 2-16-2, 3-6-7, 3-7-5, 3-8-3, 3-8-4, 3-8-5, 3-9-2, 3-9-3, 3-9-4, 3-9-5, 3-9-8, 3-10-2, 3-10-3, 3-10-4, 3-11-3, 3-12-3, 3-14-3, 4-7-4, 4-7-5, 4-8-3, 4-8-4, 4-9-3, 4-10-3, 5-6-4, 5-6-5, 5-7-3, 5-7-4, 5-8-3, 5-8-4, or 7-6-3.

In certain embodiments, a modified oligonucleotide targeting a DUX4 nucleic acid has a 3-10-3 gapmer motif. In certain embodiments, a modified oligonucleotide targeting a DUX4 nucleic acid has a 3-9-3 gapmer motif. In certain embodiments, a modified oligonucleotide targeting a DUX4 nucleic acid has a 3-9-4 gapmer motif. In certain embodiments, a modified oligonucleotide targeting a DUX4 nucleic acid has a 3-8-5 gapmer motif.

In certain embodiments, these gapmer modified oligonucleotides include at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous nucleobases of a nucleobase sequence of any of the exemplary modified oligonucleotides described herein (for example, at least 8 contiguous nucleobases of a nucleobase sequence described in any one of SEQ ID NOs: 2,3,4, 7 - 64, 69 - 97 or 102 - 109 in the sequence listing).

In certain embodiments, the present invention provides an oligomeric compound containing an oligonucleotide. In certain embodiments, such an oligonucleotide includes one or more chemical modifications. In certain embodiments, a chemically modified oligonucleotide includes one or more modified sugars. In certain embodiments, a chemically modified oligonucleotide includes one or more modified nucleobases. In certain embodiments, a chemically modified oligonucleotide includes one or more modified internucleoside linkages. In certain embodiments, chemical modifications (sugar modification, nucleobase modification and/or binding modification) define patterns or motifs. In certain embodiments, patterns of chemical modifications of sugar moieties, internucleoside linkages and nucleobases are each independent of one another. Thus, an oligonucleotide can be described by its sugar modification motif, internucleoside linkage motif and/or nucleobase modification motif (as used herein, a nucleobase modification motif describes chemical modification with respect to nucleobases independent of a sequence of the nucleobases).

### Certain Sugar Motifs

In certain embodiments, an oligonucleotide includes one or more types of modified sugar moieties and/or naturally occurring sugar moieties arranged along the oligonucleotide or a region thereof in a defined pattern or sugar modification motif. Such motifs can include any one of the sugar modifications discussed herein and/or other known sugar modifications.

In certain embodiments, an oligonucleotide includes or consists of a region having a gapmer sugar modification motif, which includes two external regions, that is, "wing segments," and one internal region, that is, a "gap segment." The three regions of a gapmer motif (the 5' wing segment, the gap segment, and the 3' wing segment) form a contiguous sequence of nucleosides wherein at least some of the sugar moieties of the nucleosides of each of the wing segments differ from at least some of the sugar moieties of the nucleosides of the gap segment. In particular, at least the sugar moieties of the nucleosides of the wing segments that are closest to the gap segment (the 3'-most nucleoside of the 5' wing segment and the 5'-most nucleoside of the 3' wing segment) differ from the sugar moieties of the neighboring nucleosides of the gap segment, and thus, boundary sides between the wing segments and the gap segment are defined. In certain embodiments, the sugar moieties in the gap segment are the same as one another. In certain embodiments, the gap segment includes one or more nucleosides having a sugar moiety that is different from a sugar moiety of one or more other nucleosides of the gap segment. In certain embodiments, the sugar modification motifs of the two wing segments are the same as one another (symmetric gapmer). In certain embodiments, the sugar modification motif of the 5' wing segment is different from the sugar modification motif of the 3' wing segment (asymmetric gapmer).

### Certain 5' Wing Segments

In certain embodiments, the 5' wing segment of a gapmer consists of 1 to 5 linked nucleosides. In certain embodiments, the 5' wing segment of a gapmer consists of 2 to 5 linked nucleosides. In certain embodiments, the 5' wing segment of a gapmer consists of 3 to 5 linked nucleosides. In certain embodiments, the 5' wing segment of a gapmer consists of 4 or 5 linked nucleosides. In certain embodiments, the 5' wing segment of a gapmer consists of 1 to 4 linked nucleosides. In certain embodiments, the 5' wing segment of a gapmer consists of 1 to 3 linked nucleosides. In certain embodiments, the 5' wing segment of a gapmer consists of 1 or 2 linked nucleosides. In certain embodiments, the 5' wing segment of a gapmer consists of 2 to 4 linked nucleosides. In certain embodiments, the 5' wing segment of a gapmer consists of 2 or 3 linked nucleosides. In certain embodiments, the 5' wing segment of a gapmer consists of 3 or 4 linked nucleosides. In certain embodiments, the 5' wing segment of a gapmer consists of 1 nucleoside. In certain embodiments, the 5' wing segment of a gapmer consists of 2 linked nucleosides. In certain embodiments, the 5' wing segment of a gapmer consists of 3 linked nucleosides. In certain embodiments, the 5' wing segment of a gapmer consists of 4 linked nucleosides. In certain embodiments, the 5' wing segment of a gapmer consists of 5 linked nucleosides.

In certain embodiments, the 5' wing segment of a gapmer includes at least 1 bicyclic nucleoside. In certain embodiments, the 5' wing segment of a gapmer includes at least 2 bicyclic nucleosides. In certain embodiments, the 5' wing segment of a gapmer includes at least 3 bicyclic nucleosides. In certain embodiments, the 5' wing segment of a gapmer includes at least 4 bicyclic nucleosides. In certain embodiments, the 5' wing segment of a gapmer includes at least 1 constrained ethyl nucleoside. In certain embodiments, the 5' wing segment of a gapmer includes at least one LNA-containing nucleoside, GuNA-containing nucleoside, ALNA [Ms]-containing nucleoside, ALNA [mU]-containing nucleoside, ALNA [ipU]-containing nucleoside, ALNA [Trz]-containing nucleoside and/or ALNA [Oxz]-containing nucleoside. In certain embodiments, each nucleoside of the 5' wing segment of a gapmer is a bicyclic nucleoside. In certain embodiments, each nucleoside of the 5' wing segment of a gapmer is a constrained ethyl nucleoside. In certain embodiments, each nucleoside of the 5' wing segment of a gapmer is an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside.

In certain embodiments, the 5' wing segment of a gapmer includes at least one non-bicyclic modified nucleoside. In certain embodiments, the 5' wing segment of a gapmer includes at least one 2'-substituted nucleoside. In certain embodiments, the 5' wing segment of a gapmer includes at least one, for example, three, four, or five 2'-MOE nucleosides. In certain embodiments, the 5' wing segment of a gapmer includes at least one 2'-OMe nucleoside. In certain embodiments, each nucleoside of the 5' wing segment of a gapmer is a non-bicyclic modified nucleoside. In certain embodiments, each nucleoside of the 5' wing segment of a gapmer is a 2'-substituted nucleoside. In certain embodiments, each nucleoside of the 5' wing segment of a gapmer is a 2'-MOE nucleoside. In certain embodiments, each nucleoside of the 5' wing segment of a gapmer is a 2'-OMe nucleoside.

In certain embodiments, the 5' wing segment of a gapmer includes at least one bicyclic nucleoside and at least one non-bicyclic modified nucleoside. In certain embodiments, the 5' wing segment of a gapmer includes at least one bicyclic nucleoside and at least one 2'-substituted nucleoside. In certain embodiments, the 5' wing segment of a gapmer includes at least one bicyclic nucleoside and at least one 2'-MOE nucleoside. In certain embodiments, the 5' wing segment of a gapmer includes at least one bicyclic nucleoside and at least one 2'-OMe nucleoside. In certain embodiments, the 5' wing segment of a gapmer includes at least one bicyclic nucleoside and at least one 2'-deoxynucleoside.

In certain embodiments, the 5' wing segment of a gapmer includes at least one constrained ethyl nucleoside and at least one non-bicyclic modified nucleoside. In certain embodiments, the 5' wing segment of a gapmer includes at least one constrained ethyl nucleoside and at least one 2'-substituted nucleoside. In certain embodiments, the 5' wing segment of a gapmer includes at least one constrained ethyl nucleoside and at least one 2'-MOE nucleoside. In certain embodiments, the 5' wing segment of a gapmer includes at least one constrained ethyl nucleoside and at least one 2'-OMe nucleoside. In certain embodiments, the 5' wing segment of a gapmer includes at least one constrained ethyl nucleoside and at least one 2'-deoxynucleoside.

In certain embodiments, the 5' wing segment of a gapmer includes at least one modified nucleoside selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 5' wing segment of a gapmer includes at least 2 modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 5' wing segment of a gapmer includes at least 3 modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 5' wing segment of a gapmer includes at least 4 modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 5' wing segment of a gapmer includes at least 5 modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside.

In certain embodiments, the 5' wing segment of a gapmer includes two LNA-containing nucleosides. In certain embodiments, the 5' wing segment of a gapmer includes three LNA-containing nucleosides. In certain embodiments, the 5' wing segment of a gapmer includes four LNA-containing nucleosides. In certain embodiments, the 5' wing segment of a gapmer includes three ALNA [Ms]-containing nucleosides. In certain embodiments, the 5' wing segment of a gapmer includes two GuNA-containing nucleosides. In certain embodiments, the 5' wing segment of a gapmer includes three GuNA-containing nucleosides. In certain embodiments, the 5' wing segment of a gapmer includes 3 ALNA [mU]-containing nucleosides. In certain embodiments, the 5' wing segment of a gapmer includes three ALNA [ipU]-containing nucleosides. In certain embodiments, the 5' wing segment of a gapmer includes two LNA-containing nucleosides and one GuNA-containing nucleoside. In certain embodiments, the 5' wing segment of a gapmer includes three ALNA [Trz]-containing nucleosides. In certain embodiments, the 5' wing segment of a gapmer includes three ALNA [Ms]-containing nucleosides and one 2'-OMe nucleoside. In certain embodiments, the 5' wing segment of a gapmer includes three ALNA [Ms]-containing nucleosides and two 2'-OMe nucleosides.

In certain embodiments, the 5' wing segment of a gapmer includes three constrained ethyl nucleosides. In certain embodiments, the 5' wing segment of a gapmer includes two bicyclic nucleosides and two non-bicyclic modified nucleosides. In certain embodiments, the 5' wing segment of a gapmer includes two constrained ethyl nucleosides and two 2'-OMe nucleosides. In certain embodiments, the 5' wing segment of a gapmer includes two bicyclic nucleosides and two non-bicyclic modified nucleosides. In certain embodiments, the 5' wing segment of a gapmer includes two constrained ethyl nucleosides and two 2'-OMe nucleosides. In certain embodiments, the 5' wing segment of a gapmer includes two constrained ethyl nucleosides and three 2'-OMe nucleosides.

In certain embodiments, the 5' wing segment of a gapmer consists of one ALNA [Ms]-containing nucleoside. In certain embodiments, the 5' wing segment of a gapmer consists of two linked ALNA [Ms]-containing nucleosides. In certain embodiments, the 5' wing segment of a gapmer consists of three linked ALNA [Ms]-containing nucleosides. In certain embodiments, the 5' wing segment of a gapmer consists of four linked ALNA [Ms]-containing nucleosides. In certain embodiments, the 5' wing segment of a gapmer consists of five linked ALNA [Ms]-containing nucleosides. In certain embodiments, the 5' wing segment of a gapmer consists of linked one, two or three ALNA [Ms]-containing nucleosides and one 2'-OMe nucleoside. In certain embodiments, the 5' wing segment of a gapmer consists of linked one, two or three ALNA [Ms]-containing nucleosides and two 2'-OMe nucleosides. In certain embodiments, the 5' wing segment of a gapmer consists of linked one, two or three ALNA [Ms]-containing nucleosides and three 2'-OMe nucleosides. In certain embodiments, the 5' wing segment of a gapmer consists of linked three ALNA [Ms]-containing nucleosides and one 2'-MOE nucleoside. In certain embodiments, the 5' wing segment of a gapmer consists of linked two ALNA [Ms]-containing nucleosides and two 2'-MOE nucleosides. In certain embodiments, the 5' wing segment of a gapmer consists of linked three LNA-containing nucleosides and two 2'-MOE nucleosides. In certain embodiments, the 5' wing segment of a gapmer consists of linked two LNA-containing nucleosides and two 2'-MOE nucleosides.

In certain embodiments, the 5' wing segment of a gapmer consists of linked one, two or three ALNA [Ms]-containing nucleosides and one 5-methylcytosine. In certain embodiments, the 5' wing segment of a gapmer consists of linked one, two or three ALNA [Ms]-containing nucleosides and two 5-methylcytosines. In certain embodiments, the 5' wing segment of a gapmer consists of linked one, two or three ALNA [Ms]-containing nucleosides and three 5-methylcytosines.

### Certain 3' Wing Segments

In certain embodiments, the 3' wing segment of a gapmer consists of 1 to 8 linked nucleosides. In certain embodiments, the 3' wing segment of a gapmer consists of 2 to 5 linked nucleosides. In certain embodiments, the 3' wing segment of a gapmer consists of 3 to 5 linked nucleosides. In certain embodiments, the 3' wing segment of a gapmer consists of 4 or 5 linked nucleosides. In certain embodiments, the 3' wing segment of a gapmer consists of 1 to 4 linked nucleosides. In certain embodiments, the 35' wing segment of a gapmer consists of 1 to 3 linked nucleosides. In certain embodiments, the 3' wing segment of a gapmer consists of 1 or 2 linked nucleosides. In certain embodiments, the 3' wing segment of a gapmer consists of 2 to 4 linked nucleosides. In certain embodiments, the 3' wing segment of a gapmer consists of 2 or 3 linked nucleosides. In certain embodiments, the 3' wing segment of a gapmer consists of 3 or 4 linked nucleosides. In certain embodiments, the 3' wing segment of a gapmer consists of 1 nucleoside. In certain embodiments, the 3' wing segment of a gapmer consists of 2 linked nucleosides. In certain embodiments, the 3' wing segment of a gapmer consists of 3 linked nucleosides. In certain embodiments, the 3' wing segment of a gapmer consists of 4 linked nucleosides. In certain embodiments, the 3' wing segment of a gapmer consists of 5 linked nucleosides. In certain embodiments, the 3' wing segment of a gapmer consists of 6 linked nucleosides. In certain embodiments, the 3' wing segment of a gapmer consists of 7 linked nucleosides. In certain embodiments, the 3' wing segment of a gapmer consists of 8 linked nucleosides.

In certain embodiments, the 3' wing segment of a gapmer includes at least one bicyclic nucleoside. In certain embodiments, the 3' wing segment of a gapmer includes at least two bicyclic nucleosides. In certain embodiments, the 3' wing segment of a gapmer includes at least three bicyclic nucleosides. In certain embodiments, the 3' wing segment of a gapmer includes at least four bicyclic nucleosides. In certain embodiments, the 3' wing segment of a gapmer includes at least one constrained ethyl nucleoside. In certain embodiments, the 3' wing segment of a gapmer includes at least one LNA-containing nucleoside, GuNA-containing nucleoside, ALNA [Ms]-containing nucleoside, ALNA [mU]-containing nucleoside, ALNA [ipU]-containing nucleoside, ALNA [Trz]-containing nucleoside and/or ALNA [Oxz]-containing nucleoside. In certain embodiments, each nucleoside of the 3' wing segment of a gapmer is a bicyclic nucleoside. In certain embodiments, each nucleoside of the 3' wing segment of a gapmer is a constrained ethyl nucleoside. In certain embodiments, each nucleoside of the 3' wing segment of a gapmer is an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside.

In certain embodiments, the 3' wing segment of a gapmer includes at least one non-bicyclic modified nucleoside. In certain embodiments, the 3' wing segment of a gapmer includes at least one 2'-substituted nucleoside. In certain embodiments, the 3' wing segment of a gapmer includes at least one 2'-MOE nucleoside. In certain embodiments, the 3' wing segment of a gapmer includes at least one 2'-OMe nucleoside. In certain embodiments, each nucleoside of the 3' wing segment of a gapmer is a non-bicyclic modified nucleoside. In certain embodiments, each nucleoside of the 3' wing segment of a gapmer is a 2'-substituted nucleoside. In certain embodiments, each nucleoside of the 3' wing segment of a gapmer is a 2'-MOE nucleoside. In certain embodiments, each nucleoside of the 3' wing segment of a gapmer is a 2'-OMe nucleoside.

In certain embodiments, the 3' wing segment of a gapmer includes at least one bicyclic nucleoside and at least one non-bicyclic modified nucleoside. In certain embodiments, the 3' wing segment of a gapmer includes at least one bicyclic nucleoside and at least one 2'-substituted nucleoside. In certain embodiments, the 3' wing segment of a gapmer includes at least one bicyclic nucleoside and at least one 2'-MOE nucleoside. In certain embodiments, the 3' wing segment of a gapmer includes at least one bicyclic nucleoside and at least one 2'-OMe nucleoside. In certain embodiments, the 3' wing segment of a gapmer includes at least one bicyclic nucleoside and at least one 2'-deoxynucleoside.

In certain embodiments, the 3' wing segment of a gapmer includes at least one constrained ethyl nucleoside and at least one non-bicyclic modified nucleoside. In certain embodiments, the 3' wing segment of a gapmer includes at least one constrained ethyl nucleoside and at least one 2'-substituted nucleoside. In certain embodiments, the 3' wing segment of a gapmer includes at least one constrained ethyl nucleoside and at least one 2'-MOE nucleoside. In certain embodiments, the 3' wing segment of a gapmer includes at least one constrained ethyl nucleoside and at least one 2'-OMe nucleoside. In certain embodiments, the 3' wing segment of a gapmer includes at least one constrained ethyl nucleoside and at least one 2'-deoxynucleoside.

In certain embodiments, the 3' wing segment of a gapmer includes at least one modified nucleoside selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 3' wing segment of a gapmer includes at least two modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 3' wing segment of a gapmer includes at least three modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 3' wing segment of a gapmer includes at least four modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 3' wing segment of a gapmer includes at least five modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 3' wing segment of a gapmer includes at least six modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 3' wing segment of a gapmer includes at least seven modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside. In certain embodiments, the 3' wing segment of a gapmer includes at least eight modified nucleosides selected from a 2'-MOE nucleoside, a 2'-OMe nucleoside, an LNA-containing nucleoside, a GuNA-containing nucleoside, an ALNA [Ms]-containing nucleoside, an ALNA [mU]-containing nucleoside, an ALNA [ipU]-containing nucleoside, an ALNA [Trz]-containing nucleoside and/or an ALNA [Oxz]-containing nucleoside.

In certain embodiments, the 3' wing segment of a gapmer includes two LNA-containing nucleosides. In certain embodiments, the 3' wing segment of a gapmer includes three LNA-containing nucleosides. In certain embodiments, the 3' wing segment of a gapmer includes four LNA-containing nucleosides. In certain embodiments, the 3' wing segment of a gapmer includes three ALNA [Ms]-containing nucleosides. In certain embodiments, the 3' wing segment of a gapmer includes two GuNA-containing nucleosides. In certain embodiments, the 3' wing segment of a gapmer includes three GuNA-containing nucleosides. In certain embodiments, the 3' wing segment of a gapmer includes three ALNA [mU]-containing nucleosides. In certain embodiments, the 3' wing segment of a gapmer includes three ALNA [ipU]-containing nucleosides. In certain embodiments, the 3' wing of a gapmer includes two LNA-containing nucleosides and one GuNA-containing nucleoside. In certain embodiments, the 3' wing segment of a gapmer includes three ALNA [Trz]-containing nucleosides. In certain embodiments, the 3' wing segment of a gapmer includes three ALNA [Ms]-containing nucleosides and one 2'-OMe nucleoside. In certain embodiments, the 3' wing segment of a gapmer includes three ALNA [Ms]-containing nucleosides and two 2'-OMe-containing nucleosides. In certain embodiments, the 3' wing segment of a gapmer includes three 2'-MOE nucleosides. In certain embodiments, the 3' wing segment of a gapmer includes four 2'-MOE nucleosides. In certain embodiments, the 3' wing segment of a gapmer includes five 2'-MOE. In certain embodiments, the 3' wing segment of a gapmer includes three ALNA [Ms]-containing nucleosides and one 2'-MOE-containing nucleoside. In certain embodiments, the 3' wing segment of a gapmer includes three ALNA [Ms]-containing nucleosides and two 2'-MOE nucleosides. In certain embodiments, the 3' wing segment of a gapmer includes two ALNA [Ms]-containing nucleosides and two 2'-MOE nucleosides. In certain embodiments, the 3' wing segment of a gapmer includes two ALNA [Ms]-containing nucleosides and three 2'-MOE nucleosides. In certain embodiments, the 3' wing segment of a gapmer includes three ALNA [Ms]-containing nucleosides and five 2'-MOE nucleosides. In certain embodiments, the 3' wing segment of a gapmer includes one ALNA [Ms]-containing nucleoside and three 2'-MOE nucleosides. In certain embodiments, the 3' wing segment of a gapmer includes two LNA-containing nucleosides and two 2'-MOE nucleosides.

In certain embodiments, the 3' wing segment of a gapmer includes three constrained ethyl nucleosides. In certain embodiments, the 3' wing segment of a gapmer includes two bicyclic nucleosides and two non-bicyclic modified nucleosides. In certain embodiments, the 3' wing segment of a gapmer includes two constrained ethyl nucleosides and two 2'-OMe nucleosides. In certain embodiments, the 3' wing segment of a gapmer includes two bicyclic nucleosides and two non-bicyclic modified nucleosides. In certain embodiments, the 3' wing segment of a gapmer includes two constrained ethyl nucleosides and two 2'-OMe nucleosides. In certain embodiments, the 3' wing segment of a gapmer includes two constrained ethyl nucleosides and three 2'-OMe nucleosides.

In certain embodiments, the 3' wing segment of a gapmer consists of one ALNA [Ms]-containing nucleoside. In certain embodiments, the 3' wing segment of a gapmer consists of two linked ALNA [Ms]-containing nucleosides. In certain embodiments, the 3' wing segment of a gapmer consists of three linked ALNA [Ms]-containing nucleosides. In certain embodiments, the 3' wing segment of a gapmer consists of four linked ALNA [Ms]-containing nucleosides. In certain embodiments, the 3' wing segment of a gapmer consists of five linked ALNA [Ms]-containing nucleosides. In certain embodiments, the 3' wing segment of a gapmer consists of three, four or five linked 2'-MOE nucleosides. In certain embodiments, the 3' wing segment of a gapmer consists of linked one, two or three ALNA [Ms]-containing nucleosides and one 2'-OMe nucleoside. In certain embodiments, the 3' wing segment of a gapmer consists of linked one, two or three ALNA [Ms]-containing nucleosides and two 2'-OMe nucleosides. In certain embodiments, the 3' wing segment of a gapmer consists of linked one, two or three ALNA [Ms]-containing nucleosides and three 2'-OMe nucleosides. In certain embodiments, the 3' wing segment of a gapmer consists of linked one, two or three ALNA [Ms]-containing nucleosides and one 2'-MOE nucleoside. In certain embodiments, the 3' wing segment of a gapmer consists of linked one, two or three ALNA [Ms]-containing nucleosides and two 2'-MOE nucleosides. In certain embodiments, the 3' wing segment of a gapmer consists of linked one, two or three ALNA [Ms]-containing nucleosides and three 2'-MOE nucleosides. In certain embodiments, the 3' wing segment of a gapmer consists of linked one, two or three ALNA [Ms]-containing nucleosides and five 2'-MOE nucleosides. In certain embodiments, the 3' wing segment of a gapmer consists of linked two LNA-containing nucleosides and two 2'-MOE nucleosides.

In certain embodiments, the 3' wing segment of a gapmer consists of linked one, two or three ALNA [Ms]-containing nucleosides and one 5-methylcytosine. In certain embodiments, the 3' wing segment of a gapmer consists of linked one, two or three ALNA [Ms]-containing nucleosides and two 5-methylcytosines. In certain embodiments, the 3' wing segment of a gapmer consists of linked one, two or three ALNA [Ms]-containing nucleosides and three 5-methylcytosines. In certain embodiments, the 3' wing segment of a gapmer consists of linked one, two, three, four or five 2'-MOE nucleosides and one 5-methylcytosine.

In certain embodiments, the 5' wing segment of a gapmer consists of one ALNA [Ms]-containing nucleoside and the 3' wing segment consists of one ALNA [Ms]-containing nucleoside. In certain embodiments, the 5' wing segment of a gapmer consists of two linked ALNA [Ms]-containing nucleosides and the 3' wing segment consists of two linked ALNA [Ms]-containing nucleosides. In certain embodiments, the 5' wing segment of a gapmer consists of three linked ALNA [Ms]-containing nucleosides and the 3' wing segment consists of three linked ALNA [Ms]-containing nucleosides. In certain embodiments, the 5' wing segment of a gapmer consists of four linked ALNA [Ms]-containing nucleosides and the 3' wing segment consists of four linked ALNA [Ms]-containing nucleosides. In certain embodiments, the 5' wing segment of a gapmer consists of five linked ALNA [Ms]-containing nucleosides and the 3' wing segment consists of five linked ALNA [Ms]-containing nucleosides.

In certain embodiments, the 5' wing segment of a gapmer consists of linked one, two or three ALNA [Ms]-containing nucleosides and one 2'-OMe nucleoside; and the 3' wing segment consists of linked one, two or three ALNA [Ms]-containing nucleosides and one 2'-OMe nucleoside. In certain embodiments, the 5' wing segment of a gapmer consists of linked one, two or three ALNA [Ms]-containing nucleosides and two 2'-OMe nucleosides; and the 3' wing segment consists of linked one, two or three ALNA [Ms]-containing nucleosides and two 2'-OMe nucleosides. In certain embodiments, the 5' wing segment of a gapmer consists of linked one, two or three ALNA [Ms]-containing nucleosides and three 2'-OMe nucleosides; and the 3' wing segment consists of linked one, two or three ALNA [Ms]-containing nucleosides and three 2'-OMe nucleosides.

In certain embodiments, the 5' wing segment of a gapmer consists of linked one, two or three ALNA [Ms]-containing nucleosides and one or two 2'-MOE nucleosides; and the 3' wing segment consists of linked one, two or three ALNA [Ms]-containing nucleosides and one or two 2'MOE nucleosides. In certain embodiments, the 5' wing segment of a gapmer consists of one, two or three linked ALNA [Ms]-containing nucleosides; and the 3' wing segment consists of linked one, two or three ALNA [Ms]-containing nucleosides and one, two or three 2'-MOE nucleosides. In certain embodiments, the 5' wing segment of a gapmer consists of one, two or three linked ALNA [Ms]-containing nucleosides; and the 3' wing segment consists of one, two, three, four or five linked 2'-MOE nucleosides. In certain embodiments, the 5' wing segment of a gapmer consists of one, two, three, four or five linked 2'-MOE nucleosides; and the 3' wing segment consists of one, two or three linked ALNA [Ms]-containing nucleosides.

In certain embodiments, the 5' wing segment of a gapmer consists of one, two or three linked ALNA [Ms]-containing nucleosides and includes one 5-methylcytosine; and the 3' wing segment of the gapmer consists of one, two or three linked ALNA [Ms]-containing nucleosides and includes one 5-methylcytosine. In certain embodiments, the 5' wing segment of a gapmer consists of one, two or three linked ALNA [Ms]-containing nucleosides and includes two 5-methylcytosines; and the 3' wing segment of the gapmer consists of one, two or three linked ALNA [Ms]-containing nucleosides and includes one 5-methylcytosine. In certain embodiments, the 5' wing segment of a gapmer consists of one, two or three linked ALNA [Ms]-containing nucleosides and includes two 5-methylcytosines; and the 3' wing segment of the gapmer consists of one, two or three linked ALNA [Ms]-containing nucleosides and includes two 5-methylcytosines. In certain embodiments, the 5' wing segment of a gapmer consists of one, two or three linked ALNA [Ms]-containing nucleosides and includes three 5-methylcytosines; and the 3' wing segment of the gapmer consists of one, two or three linked ALNA [Ms]-containing nucleosides and includes three 5-methylcytosines. In certain embodiments, the 5' wing segment of a gapmer consists of one, two or three linked ALNA [Ms]-containing nucleosides and includes one 5-methylcytosine; and the 3' wing segment of the gapmer consists of one, two, three, four or five linked 2'-MOE nucleosides and includes one 5-methylcytosine. In certain embodiments, the 5' wing segment of a gapmer consists of one, two or three linked ALNA [Ms]-containing nucleosides; and the 3' wing segment of the gapmer consists of linked one, two or three ALNA [Ms]-containing nucleosides and one, two or three 2'-MOE nucleosides, and includes one 5-methylcytosine.

In certain embodiments, the gap segment of a gapmer includes 10 contiguous nucleosides, and includes a 2'-OMe nucleoside as either the 1st, 2nd, 3rd, 4th, 5th, 6th, 7th, 8th, 9th or 10th nucleoside, with the remaining nucleosides being deoxynucleosides.

### Compositions and Methods for Formulating Pharmaceutical Compositions

A modified oligonucleotide can be mixed with one or more pharmaceutically acceptable active or inactive substances for preparation of a pharmaceutical composition or formulation. A composition and a method for formulating a pharmaceutical composition depend on a number of criteria that include, but are not limited to, a route of administration, an extent of a disease, or a dose to be administered.

A modified oligonucleotide targeting a DUX4 nucleic acid can be utilized in a pharmaceutical composition by combining the modified oligonucleotide with a suitable pharmaceutically acceptable diluent or carrier. Examples of a pharmaceutically acceptable diluent include phosphate buffered saline (PBS). PBS is a diluent suitable for use in a composition to be delivered parenterally. Therefore, in one embodiment, a pharmaceutical composition including a modified oligonucleotide targeting a DUX4 nucleic acid and a pharmaceutically acceptable diluent is used in a method described herein. In certain embodiments, the pharmaceutically acceptable diluent is PBS.

Pharmaceutical compositions including modified oligonucleotides include any pharmaceutically acceptable salt, ester, or salt of such an ester, or any other oligonucleotides that can provide (directly or indirectly) biologically active metabolites or residues thereof when administered to animals including humans. Therefore, for example, the present disclosure also relates to a pharmaceutically acceptable salt of a modified oligonucleotide, a prodrug, a pharmaceutically acceptable salt of such a prodrug, and other bioequivalents. Suitable pharmaceutically acceptable salts include, but are not limited to, sodium salts and potassium salts.

A prodrug can include incorporating additional nucleosides at one or both ends of a modified oligonucleotide cleaved by endogenous nucleases within the body in order to form an active modified oligonucleotide.

### Conjugated Modified Oligonucleotide

A modified oligonucleotide can be covalently linked to one or more moieties or conjugates that enhance activity, cellular distribution or cellular uptake of a resulting modified oligonucleotide. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

For example, a modified oligonucleotide can also be modified so as to have one or more stabilizing groups that are generally attached to one or both ends of the modified oligonucleotide in order to enhance properties such as nuclease stability. A cap structure is included in a stabilizing group. These terminal modifications can protect a modified oligonucleotide having a terminal nucleic acid from degradation by exonucleases, and can help in intracellular delivery and/or localization. A cap can be present at the 5' end (5' cap), or at the 3' end (3' cap), or can be present on both ends. Cap structures are well known in the art and include, for example, an inverted deoxy abasic cap. Additional 3' and 5' stabilizing groups that can be used to cap one or both ends of a modified oligonucleotide in order to impart nuclease stability include those disclosed in WO 03/004602.

### Cell Culture and Modified Oligonucleotide Treatment

An effect of a modified oligonucleotide with respect to a level, activity or expression of a DUX4 nucleic acid can be tested in vitro in various cell types. Cell types used for such analyses are available from commercial suppliers (for example, American Type Culture Collection, Manassus, VA; Zen-Bio, Inc., Research Triangle Park, NC; Clonetics Corporation, Walkersville, MD), and cells are cultured according to the supplier's instructions using commercially available reagents (for example, Invitrogen Life Technologies, Carlsbad, CA). Exemplary cell types include, but are not limited to, C2C12 cells, HepG2 cells, Hep3B cells, primary hepatocytes, A549 cells, GM04281 fibroblasts and LLC-MK2 cells. These cells can be used by transfecting a vector expressing human DUX4 mRNA. The vector is preferably expressed as a fusion protein with a reporter gene such as luciferase or GFP, and an example thereof is psiCHECK-2 vector (Promega).

### In Vitro Testing of Modified Oligonucleotide

A method for treating cells with a modified oligonucleotide is described herein, and can be suitably modified according to the type of the modified oligonucleotide.

In general, cells are treated with a modified oligonucleotide when the cells reach about 60 - 80% confluence in culture.

A modified oligonucleotide can be introduced into cells, for example, by using a lipofection method.

One reagent commonly used to introduce a modified oligonucleotide into cultured cells is a cationic lipid transfection reagent, LIPOFECTIN (registered trademark) (Invitrogen, Carlsbad, CA). A modified oligonucleotide is mixed with LIPOFECTIN (registered trademark) in OPTI-MEM (registered trademark) (Invitrogen, Carlsbad, CA) to achieve a desired final modified oligonucleotide concentration and a LIPOFECTIN (registered trademark) concentration that typically ranges 2 - 12 ug/mL per 100 nM modified oligonucleotide.

Another reagent used to introduce a modified oligonucleotide into cultured cells is LIPOFECTAMINE 2000 (registered trademark) (Invitrogen, Carlsbad, CA). A modified oligonucleotide is mixed with LIPOFECTAMINE 2000 (registered trademark) in OPTI-MEM (registered trademark) 1 serum reduction medium (Invitrogen, Carlsbad, CA) to achieve a desired modified oligonucleotide concentration and a LIPOFECTAMINE (registered trademark) concentration that typically ranges 2 - 12 ug/mL per 100 nM modified oligonucleotide.

Another reagent used to introduce a modified oligonucleotide into cultured cells is Cytofectin (registered trademark) (Invitrogen, Carlsbad, CA). A modified oligonucleotide is mixed with Cytofectin (registered trademark) in OPTI-MEM (registered trademark) 1 serum reduction medium (Invitrogen, Carlsbad, CA) to achieve a desired modified oligonucleotide concentration and a Cytofectin (registered trademark) concentration that typically ranges 2 - 12 ug/mL per 100 nM modified oligonucleotide.

Another technique used to introduce a modified oligonucleotide into cultured cells is electroporation.

A modified oligonucleotide can be introduced into cells without using LIPOFECTION or the like. A method for suppressing expression of a target gene of the modified oligonucleotide in this case is called a Gymnosis method.

Cells are treated with a modified oligonucleotide using a conventional method. Typically, cells are harvested 16 - 48 hours after a modified oligonucleotide treatment, at which time an RNA or protein level of a target nucleic acid is measured using a method known in the art and described herein. In general, when a treatment is performed in multiple repetitions, data is presented as an average of the repeated treatment.

The concentration of a modified oligonucleotide used varies from cell line to cell line. A method for determining an optimal modified oligonucleotide concentration with respect to a particular cell line is well known in the art. A modified oligonucleotide is typically used at a concentration ranging from 1 nM to 300 nM when transfected using LIPOFECTAMINE2000 (registered trademark), LIPOFECTIN or Cytofectin. A modified oligonucleotide is used at a higher concentration ranging from 625 to 20,000 nM when transfected using electroporation. From an inhibition rate of gene expression at each concentration, a concentration IC₅₀ of a modified oligonucleotide that suppresses 50% gene expression can be calculated.

### RNA Isolation

RNA analysis can be performed on total cellular RNA or poly(A)+ mRNA. A method for RNA isolation is well known in the art. RNA is prepared using a method well known in the art, for example, using a TRIZOL (registered trademark) reagent (Invitrogen, Carlsbad, CA) according to a manufacturer recommended protocol.

### Analysis of Inhibition of Target Level or Expression

Inhibition of a DUX4 nucleic acid level or expression can be assayed using various methods known in the art. For example, a target nucleic acid level can be quantified, for example, using Northern blot analysis, competitive polymerase chain reaction (PCR) or quantitative real-time PCR. RNA analysis can be performed on total cellular RNA or poly(A)+ mRNA. A method for RNA isolation is well known in the art. Northern blot analysis is also routinely performed in the art. Quantitative real-time PCR can be conveniently accomplished using the commercially available ABI PRISM (registered trademark) 7600, 7700, or 7900 Sequence Detection System that is available from PE-Applied Biosystems, Foster City, CA and is used according to manufacturer's instructions.

### Quantitative Real-Time PCR Analysis of Target RNA Level

Quantification of a target RNA level can be accomplished by quantitative real-time PCR using the ABI PRISM (registered trademark) 7600, 7700, or 7900 Sequence Detection System (PE-Applied Biosystems, Foster City, CA) according to manufacturer's instructions. A method for quantitative real-time PCR is well known in the art.

Prior to real-time PCR, isolated RNA is subjected to a reverse transcriptase (RT) reaction, and, as a result, complementary DNA (cDNA) is generated which is then used as a substrate for real-time PCR amplification. The RT and real-time PCR reactions are performed sequentially in the same sample well. RT and real-time PCR reagents are obtained from Invitrogen (Carlsbad, CA). The RT and real-time-PCR reactions are performed using methods well known to a person skilled in the art.

A gene (or RNA) target quantity obtained by real time PCR is normalized using either an expression level of a gene of which expression is constant, such as cyclophilin A, or by quantifying total RNA using RIBOGREEN (registered trademark) (Invitrogen, Inc. Carlsbad, CA). Expression of Cyclophilin A is quantified by performing multiplex or separate real time PCR simultaneously with the target. Total RNA is quantified using a RIBOGREEN (registered trademark) RNA quantification reagent (Invitrogen, Inc. Eugene, OR). A method for RNA quantification using RIBOGREEN (registered trademark) is taught in Jones, L. J., et al, (Analytical Biochemistry, 1998, 265, 368 - 374). RIBOGREEN (registered trademark) fluorescence is measured using a CYTOFLUOR (registered trademark) 4000 instrument (PE Applied Biosystems).

Probes and primers are designed to hybridize to a DUX4 nucleic acid. Methods for designing real-time PCR probes and primers are well known in the art, and can include the use of software such as PRIMER EXPRESS (registered trademark) software (Applied Biosystems, Foster City, CA).

### Analysis of Protein Level

Antisense inhibition of a DUX4 nucleic acid can be assessed by measuring a DUX4 protein level. A DUX4 protein level can be examined or quantified using various methods well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). An antibody directed to a target can be identified and obtained from a variety of sources, such as the MSRS catalog of antibodies (Aerie Corporation, Birmingham, MI), or can be prepared using conventional monoclonal or polyclonal antibody generation methods well known in the art.

### Analysis of Gene Expression

A level of DUX4 gene expression can also be measured using a reporter gene such as luciferase. For example, using the psiCHECK-2 vector (Promega), DUX4 gene expression can be measured by an amount of luminescence of Renilla luciferase which is a fusion protein with DUX4, and, by correcting with an amount of luminescence of Firefly luciferase present on the same vector, non-specific effects such as cell death can be excluded.

### In Vivo Testing of Modified Oligonucleotide

A modified oligonucleotide is tested in an animal in order to evaluate its ability to inhibit DUX4 expression and to produce a phenotype change. The test can be performed in a normal animal or in an experimental disease model, such as a DUX4 transgenic mouse model (Jones, T. et al., PLoS One 2018; 13 (2), Article number e0192657) or a DUX4 gene-expressing mouse using a gene recombinant AAV virus (Wallace, L M et al., Mol Ther 2012; 20 (7): 1417, Wallace, L M et al., Ann Neurol 2011; 69 (3): 540).

For administration to an animal, a modified oligonucleotide is formulated in a pharmaceutically acceptable diluent, such as phosphate-buffered saline. Administration includes a parenteral route of administration. Following a period of treatment with a modified oligonucleotide, RNA is isolated from a tissue, and a change in DUX4 nucleic acid expression is measured. A change in DUX4 protein level is also measured.

### Certain Antisense Mechanisms

FSHD is caused by abnormal expression of the DUX4 gene (particularly, the DUX4-FL splicing variants) in muscle. On the other hand, DUX4 is also expressed in testis and the like in healthy individuals. In some DUX4 splicing variants expressed in testis and the like, in addition to DUX4-FL, exon 1, exon 2, exon 6, exon 7 splicing variants and/or exon 1, exon 2, exon 4, exon 5, exon 6, exon 7 splicing variants are expressed (the above Non-Patent Document 1).

### Certain Biomarkers

At least in part, for example, gene expression of MBD3L2, ZSCAN4, TRIM43, DEFB103, ZNF217 or the like is modulated by an accumulation level of the DUX4 protein (the above Non-Patent Document 2). Further, creatinine kinase in blood can be measured as a marker for myopathy.

### Certain Indications

In certain embodiments, provided herein is a method for treating an individual, the method including administering one or more pharmaceutical compositions described herein. In certain embodiments, the individual has FSHD

Therefore, provided herein is a method for ameliorating a symptom associated with FSHD in a subject in need thereof. In certain embodiments, a method is provided for reducing incidence of one or more symptoms associated with FSHD. In certain embodiments, a method is provided for reducing severity of a symptom associated with FSHD. In certain embodiments, symptoms associated with FSHD include muscle stiffness, myotonia, facial muscle weakness, eyelid ptosis, inability to whistle, decreased facial expression changes, melancholy or angry facial expression, difficulty in pronouncing words, scapular weakness (deformations such as winged shoulder blades and slopping shoulders), lower limb weakness, hearing loss, and heart diseases.

In certain embodiments, the method of the invention includes administering a therapeutically effective amount of a compound targeting a DUX4 nucleic acid to an individual in need thereof.

In certain embodiments, administration of a modified oligonucleotide targeting a DUX4 nucleic acid results in a reduction in DUX4 expression by at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95% or at least about 99% or a range defined by any two of these values.

In certain embodiments, a pharmaceutical composition containing a modified oligonucleotide targeting DUX4 is used in preparation of a medicament for treating a patient having or susceptible to a DUX4-related disease such as FSHD

In certain embodiments, a method described herein includes administering a compound containing a modified oligonucleotide having contiguous nucleobase portions as described herein of a sequence set forth in SEQ ID NOs: 2, 3, 4, 7 - 64, 69 - 97 or 102 - 109 in the sequence listing.

### Administration

In certain embodiments, a compound and a pharmaceutical composition described herein are administered parenterally.

In certain embodiments, parenteral administration is by infusion. The infusion may be long-term or continuous, short-term or intermittent. In certain embodiments, an infused pharmaceutical agent is delivered using a pump. In certain embodiments, parenteral administration is by injection (for example, by bolus injection). An injectable drug can be delivered with a syringe.

Examples of parenteral administration include subcutaneous administration, intravenous administration, intramuscular administration, intraarterial administration, Intracavitary administration or intracranial administration, for example, intrathecal or intraventricular administration. Administration may be continuous or long-term, short-term or intermittent.

In certain embodiments, delivery of a compound of a pharmaceutical composition described herein results in down-regulation of at least 70% in target mRNA and/or protein levels. In certain embodiments, delivery of a compound or composition described herein results in 10%, 20%, 30%, 40%, 50%, 55%, 60%, 65%, 70%, 75%, 80%, 85%, 90%, 95% or 100% down-regulation in target mRNA and/or target protein levels over at least 1 day, at least 3 days, at least 5 days, at least 7 days, at least 10 days, at least 14 days, at least 20 days, at least 21 days, at least 28 days, at least 30 days, at least 35 days, at least 40 days, at least 45 days, at least 50 days, at least 55 days, at least 60 days, at least 65 days, at least 70 days, at least 75 days, at least 76 days, at least 77 days, at least 78 days, at least 79 days, at least 80 days, at least 85 days, at least 90 days, at least 95 days, at least 100 days, at least 105 days, at least 110 days, at least 115 days, at least 120 days, at least 1 year.

In certain embodiments, a modified oligonucleotide is delivered by injection or infusion once daily, once every three days, once a week, once every two weeks, once every three weeks, once every month, once every two months, once every three months, once every six months,twice a year or once a year.

### Certain Combination Therapies

In certain embodiments, a first agent including a modified oligonucleotide of the present invention is co-administered with one or more second agents. In certain embodiments, such second agents are designed to treat the same FSHD as the first agent described herein. In certain embodiments, such second agents are designed to treat a disease, a disorder, or a condition different from the first agent described herein. In certain embodiments, such second agents are designed to treat an undesired side effect of one or more pharmaceutical compositions described herein. In certain embodiments, a second agent is co-administered with a first agent to treat an undesired effect of the first agent. In certain embodiments, a second agent is co-administered with a first agent to produce a combinational effect. In certain embodiments, a second agent is co-administered with a first agent to produce a synergistic effect.

In certain embodiments, a first agent and one or more second agents are administered at the same time. In certain embodiments, a first agent and one or more second agents are administered at different times. In certain embodiments, a first agent and one or more second agents are prepared together in a single pharmaceutical formulation. In certain embodiments, a first agent and one or more second agents are prepared separately.

### Certain Compounds

In certain embodiments, a compound disclosed herein can synthesize an oligomer using a phosphoramidite method using commercially available amidites (including LNA) for DNA and RNA synthesis. An artificial nucleic acid GuNA can synthesize an oligomer by using a method described in WO 2014/046212 and WO 2017/047816. Artificial nucleic acids ALNA [Ms], ALNA [mU], ALNA [ipU], ALNA [Trz] and ALNA [Oxz] can synthesize an oligomer by using a method described in Japanese Patent Application No. 2018-212424.

In certain embodiments, a compound disclosed herein enjoys a benefit of one or more in vitro and/or in vivo properties that are improved as compared to a suitable comparative compound.

In certain embodiments, a compound of Compound No. 1 having a sequence (from 5' to 3') ngagattcccgccggt (n is 5-methylcytosine, incorporated herein as SEQ ID NO: 2), that is, a gapmer in which the 5' wing and the 3' wing each consist of three LNA-containing nucleosides, is a compound in which each internucleoside linkage is a phosphorothioate linkage.

In certain embodiments, a compound of Compound No. 2 having a sequence (from 5' to 3') gnagttctccgcggt (n is 5-methylcytosine, incorporated herein as SEQ ID NO: 3 in the sequence listing), that is, a gapmer in which the 5' wing and the 3' wing each consist of three ALNA [Ms]-containing nucleosides, is a compound in which each internucleoside linkage is a phosphorothioate linkage.

In certain embodiments, a compound of Compound No. 3 having a sequence (from 5' to 3') gnntagacagcgtngg (n is 5-methylcytosine, incorporated herein as SEQ ID NO: 4 in the sequence listing), that is, a gapmer in which the 5' wing and the 3' wing each consist of three LNA-containing nucleosides, is a compound in which each internucleoside linkage is a phosphorothioate linkage.

In certain embodiments, a compound of Compound No. 123 having a sequence (from 5' to 3') gnntagacagcgtngg (n is 5-methylcytosine, incorporated herein as SEQ ID NO: 4 in the sequence listing), that is, a gapmer in which the 5' wing and the 3' wing each consist of three ALNA [Ms]-containing nucleosides, is a compound in which each internucleoside linkage is a phosphorothioate linkage.

### Non-limiting disclosure and incorporation by reference

While certain compounds, compositions and methods described herein have been described with specificity in accordance with certain embodiments, the following examples serve only to illustrate the compounds described herein and are not intended to limit the same. Each of the references, GENBANK accession numbers, and the like described in this application is incorporated herein by reference in its entirety.

The sequence listing attached to this application specifies each sequence as either "RNA" or "DNA" as appropriate. However, in practice, these sequences can be modified with any combination of chemical modifications. A person skilled in the art will readily understand that a designation such as "RNA" or "DNA" for describing a modified oligonucleotide is in some cases arbitrary. For example, an oligonucleotide containing a nucleoside having a 2'-OH sugar moiety and a thymine base can be described as a DNA having a modified sugar (2'-OH with respect to a natural 2'-H of a DNA), or as a RNA having a modified base (thymine (methylated uracil) with respect to a natural uracil of a RNA).

Therefore, the nucleic acid sequences provided herein, including, but not limited to, those in the sequence listing, are intended to include, but not limited to, nucleic acids containing any combination of natural or modified RNA and/or DNA, including those nucleic acids having modified nucleobases. By way of a non-limiting additional example, an oligomeric compound having a nucleobase sequence "ATCGATCG" includes any oligomeric compound, whether modified or unmodified, having such a nucleobase sequence, this includes, but is not limited to, those compounds containing RNA bases, such as those having a sequence "AUCGAUCG," and those having some DNA bases and some RNA bases such as "AUCGATCG," and oligomeric compounds having other modified or naturally occurring bases such as "ATmeCGAUCG" (here, meC indicates a cytosine base containing a methyl group at position 5).

### [Examples]

### Non-limiting disclosure and incorporation by reference

While certain compounds, compositions and methods described herein have been described with specificity in accordance with certain embodiments, the following examples serve only to illustrate the compounds described herein and are not intended to limit the same. Each of the references described in this application is incorporated herein by reference in its entirety.

Structures of artificial nucleic acids used in the present specification are shown in the following structural formulas together with their respective abbreviations.

### Structures and Abbreviations of Artificial Nucleic Acids

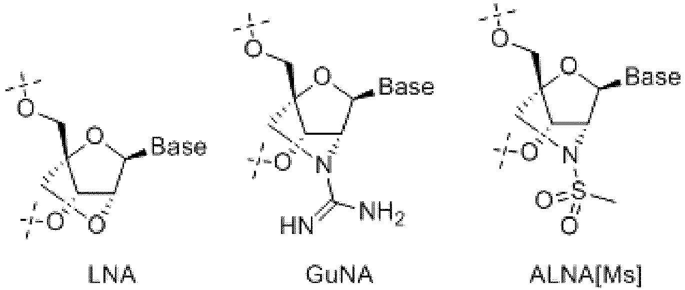

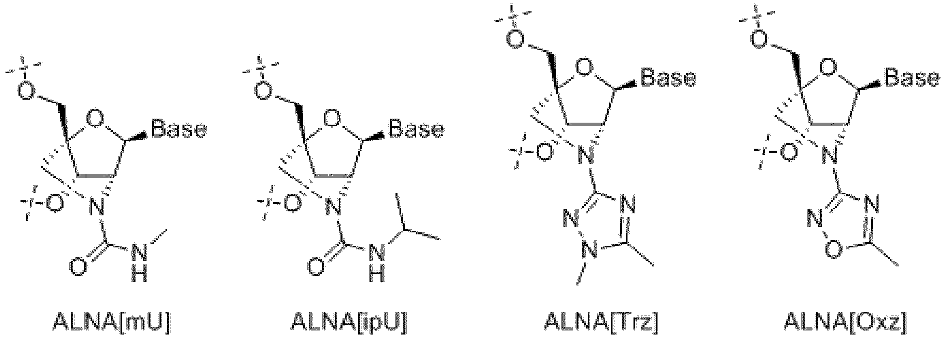

### Example 1

### Synthesis and purification of modified oligonucleotide compound for in vitro evaluation

Using various amidites (an LNA amidite was purchased from Chem Genes and Hongene Biotechnology Limited; a 2'-OMe amidite was purchased from Sigma-Aldrich; GuNA was synthesized using methods described in WO 2014/046212 and WO 2017/047816; and ALNA [Ms], ALNA [mU], ALNA [ipU], ALNA [Trz] and ALNA [Oxz] were synthesized using methods described in Japanese Patent Application No. 2018-212424), a modified oligonucleotide compound was synthesized on a 0.2 or 1.0 µmol scale using a CPG or polystyrene carrier using a DNA/RNA oligonucleotide automatic synthesizer nS-8II (manufactured by Gene Design Inc.). All the amidites were adjusted to a 0.1 M acetonitrile solution; a coupling time for unnatural nucleosides was 10 minutes; and other steps were performed under standard conditions of nS-8II. Activator 42 (Sigma-Aldrich) was used as an activator, and Sulfurizing Reagent II (Gren Research Corporation) was used for thiolation. A synthesized oligonucleotide was added with a 28% aqueous ammonia solution and reacted at 60 - 65 °C for 8 hours to cut out from a carrier and deprotect a base part. After ammonia was concentrated and distilled off, reverse phase HPLC purification was performed.

### Example 2

### Synthesis and purification of modified oligonucleotide compound for in vivo evaluation

Using various amidites, a modified oligonucleotide compound was synthesized using a polystyrene carrier on a 20 - 50 µmol scale using an automatic DNA/RNA oligonucleotide synthesizer AKTA oligopilot plus 10 (manufactured by GE Healthcare Japan). A DNA amidite was adjusted to a 0.1M, and an unnatural amidite was adjusted to a 0.05 - 0.1M acetonitrile solution; a coupling recycle time for unnatural nucleosides was 20 minutes; and, when a first base was introduced into a universal carrier, coupling, thiolation, and capping steps were each performed twice consecutively. Other steps were performed under standard conditions of AKTA oligopilot plus 10. Activator 42 (Sigma-Aldrich) was used as an activator, and Sulfurizing Reagent II (Gren Research Corporation) was used for thiolation. A synthesized oligonucleotide was subjected to a decyanoethyl treatment on a solid phase using 20% diethylamine acetonitrile or 50% triethylamine/acetonitrile, and was added with a 28% aqueous ammonia solution and reacted at 60 - 65 °C for 8-24 hours to cut out from a carrier and deprotect a base part. After ammonia was concentrated and distilled off, purification was performed using an anion exchange column. An excess salt contained after the anion exchange was removed by a desalting column.

### Example 3

### Confirmation of purity of modified oligonucleotide compound

Purification and purity confirmation of synthesized modified oligonucleotide compounds were performed by reverse phase HPLC under the following conditions. All the compounds had a purity of 85% or more.

### Reversed phase HPLC (purification)

Mobile phase:
   Solution A: 400 mM hexafluoroisopropanol, 15 mM triethylamine
   Solution B: methanol
   Gradient: A:B = 85:15 → 70:30 (10 min)
Columns used:
   Preparative Waters XBridge @ Oligonucleotide BEH C18 OBDTM Prep Column, 130 Å, 2.5 µm, 10 mm ^{∗} 50 mm
Flow rate:
   Preparative 5 mL/min
Column temperature: 60 °C
Detection: UV (260nm)

### Reversed phase HPLC (purity confirmation)

Mobile phase:
   Solution A: 400 mM hexafluoroisopropanol, 15 mM triethylamine aqueous solution
   Solution B: methanol
Gradient: A:B = 80:20 → 70:30 (6.5 min)
Columns used:
   Analysis Waters ACQUITY UPLC @ Oligonucleotide BEH C18 Column, 130 Åm 1.7 µm, 2.1 mm ^{∗} 50 mm
Flow rate: 0.2 mL/min
Column temperature: 60 °C
Detection: UV (260nm)

### Anion exchange purification

Mobile phase:
Solution A: 1 mM NaOH 20% acetonitrile aqueous solution
Solution B: 1 mM NaOH, 1.5 M NaCl / 20% acetonitrile aqueous solution
Column used: TSKgel SuperQ-5PW (13) cp21.1 ^{∗} 15 mm
Flow rate: 7 mL/min
Column temperature: room temperature
Detection: UV (260nm)

### Desalting column

Mobile phase:
   Solution A: 20% acetonitrile aqueous solution
   Solution B: 20% acetonitrile aqueous solution
Columns used:
GE HiPrep 26/10 Desalting ^{∗} 4 in series
Flow rate: 12 mL/min
Column temperature: room temperature

### Example 4

### Measurement of molecular weight of modified oligonucleotide compound

A molecular weight of a synthesized modified oligonucleotide compound was determined using Waters ZQ under the following conditions.
Mobile phase:
   Solution A: 400 mM hexafluoroisopropanol, 15 mM triethylamine aqueous solution
   Solution B: methanol
Gradient: A:B = 80:20 → 70:30 (6.5 min)
Columns used:
   Waters ACQUITY UPLC @ Oligonucleotide BEH C18 Column, 130 Åm 1.7 µm, 2.1 mm ^{∗} 50 mm
Flow rate: 0.2 mL/min
Column temperature: 60 °C
Detection: UV (260nm)

### Example 5

### Molecular weight of synthesized modified oligonucleotide compound

Synthesized modified oligonucleotide compounds are shown in Table 1 below. In the notation of the compounds, each nucleotide is represented by three letters. However, a 3'-terminal nucleotide is represented by two letters since there is no internucleoside linkage.
1) The first letter is capitalized and indicates the following nucleobases:
   A = adenine, T = thymine, G = guanine, C = cytosine, U = uracil, M = 5-methylcytosine;
2) the second letter indicates the following sugar moieties:
   1 = LNA, g = GuNA, m = ALNA[Ms], u = ALNA[mU], p = ALNA[ipU], t = ALNA[Trz], e = 2'-MOE, o = 2'-OMe, d = 2'-deoxyribose,
3) the third letter indicates the following internucleoside linkages:
   s = phosphorothioate, p = phosphodiester.

A target position indicates a 5' target site of DUX4 mature mRNA of a modified oligonucleotide (a position of SEQ ID NO: 1 in the sequence listing corresponding to a 3' end of a modified oligonucleotide).

### [Table 1-1]

**Table 1**

| **Com-pound No.** | **Sequ-ence No.** | **Target Position** | **Sequence** | **m/z** | **Ion Species** |
|---|---|---|---|---|---|
| 1 | 2 | 233 | MlsGlsAlsGdsAdsTdsTdsCdsCdsCds GdsCdsCdsGlsGlsTl | 1318.8 | [M-4H]4- |
| 2 | 3 | 1309 | GmsMmsAmsGdsTdsTdsCdsTdsCdsCds GdsCdsGmsGmsTm | 1807. 9 | [M-3H]3- |
| 3 | 4 | 1480 | GlsMlsMlsTdsAdsGdsAdsCdsAdsGds CdsGdsTdsMlsGlsGl | 1784. 3 | [M-3H]3- |
| 4 | 7 | 232 | GlsAlsTlsTdsCdsCdsCdsGdsCdsCds GdsGlsTlsGl | 4630.44 | [M-H]- |
| 5 | 8 | 233 | AgsGgsAgsTdsTdsCdsCdsCdsGdsCds CdsGgsGgsTg | 1214.8 | [M-4H]4- |
| 6 | 9 | 233 | GgsAgsGgsAdsTdsTdsCdsCdsCdsGds CdsCdsGgsGgsTg | 1300.8 | [M-4H]4- |
| 7 | 9 | 233 | GlsAlsGlsAdsTdsTdsCdsCdsCdsGds CdsCdsGlsGlsTl | 1239. 3 | [M-4H]4- |
| 8 | 10 | 233 | MmsGmsAdsGmsAdsTdsTdsMdsMdsMdsGdsM dsMmsGdsGmsTm | 1161.8 | [M-5H]5- |
| 9 | 10 | 233 | MmsGdsAmsGmsAdsTdsTdsMdsMdsMdsGdsM dsMmsGmsGdsTm | 1161.9 | [M-5H]5- |
| 10 | 2 | 233 | MgsGgsAgsGdsAdsTdsTdsCdsCdsCds GdsCdsCdsGgsGgsTg | 1381.0 | [M-4H]4- |
| 11 | 10 | 233 | MusGusAusGdsAdsTdsTdsMdsMdsMds GdsMdsMdsGusGusTu | 1136.5 | [M-5H]5- |
| 12 | 10 | 233 | MpsGpsApsGdsAdsTdsTdsMdsMdsMds GdsMdsMdsGpsGpsTp | 1170.2 | [M-5H]5- |
| 13 | 11 | 234 | GlsAlsGlsAdsTdsTdsCdsCdsCdsGds CdsMlsGlsGl | 4653. 17 | [M-H]- |
| 14 | 11 | 234 | GgsAgsGgsAdsTdsTdsCdsCdsCdsGds CdsMgsGgsGg | 1224. 5 | [M-4H]4- |
| 15 | 12 | 234 | GtsAtsGtsAdsTdsTdsMdsMdsMdsGds MdsMtsGtsGt | 1317.7 | [M-4H]4- |
| 16 | 12 | 234 | GusAusGusAdsTdsTdsMdsMdsMdsGds MdsMusGusGu | 1261.0 | [M-4H]4- |
| 17 | 12 | 234 | GmsAmsGmsAdsTdsTdsMdsMdsMdsGds MdsMmsGmsGm | 1292. 5 | [M-4H]4- |
| 18 | 13 | 234 | MmsGdsAmsGmsAdsTdsTdsMdsMdsMdsGdsM msMmsGdsGm | 1097. 5 | [M-5H]5- |
| 19 | 14 | 234 | MgsGgsAgsGdsAdsTdsTdsCdsCdsCds GdsCdsMgsGgsGg | 1304. 5 | [M-4H]4- |
| 20 | 14 | 234 | MlsGlsAlsGdsAdsTdsTdsCdsCdsCds GdsCdsMlsGlsGl | 1242. 6 | [M-4H]4- |
| 21 | 15 | 1306 | AlsGlsTlsTdsCdsTdsCdsCdsGdsCds GdsGdsTdsGlsTlsGl | 1769.7 | [M-3H]3- |
| 22 | 16 | 1307 | GlsMlsAlsGdsTdsTdsCdsTdsCdsCds GdsCdsGdsGdsTlsGlsTl | 1876.0 | [M-3H]3- |

**[Table 1-2]**

| **(Table 1 continued)** | | | | | |
|---|---|---|---|---|---|
| 23 | 17 | 1307 | GlsGlsMlsAdsGdsTdsTdsCdsTdsCds CdsGdsCdsGdsGdsTlsGlsTl | 1990.8 | [M-3H]3- |
| 24 | 18 | 1308 | MgsAgsGgsTdsTdsCdsTdsCdsCdsGds CdsGdsGgsTgsGg | 1736.7 | [M-3H]3- |
| 25 | 18 | 1308 | MgsAgsGgsTdsTdsCdsTdsCdsCdsGds CdsGdsGdsTgsGg | 1284. 9 | [M-4H]4- |
| 26 | 19 | 1308 | MmsAmsGdsTmsTdsMdsTdsMdsMdsGds MdsGmsGdsTmsGm | 1096.2 | [M-5H]5- |
| 27 | 19 | 1308 | MmsAdsGmsTmsTdsMdsTdsMdsMdsGds MdsGmsGmsTdsGm | 1370.6 | [M-4H]4- |
| 28 | 20 | 1308 | GlsCdsAlsGlsTdsTdsCdsTdsCdsCds GdsCdsGlsGlsTdsGl | 1765.4 | [M-3H]3- |
| 29 | 20 | 1308 | GlsCdsAlsGdsTlsTdsCdsTdsCdsCds GdsCdsGlsGdsTdsGl | 1755.3 | [M-3H]3- |
| 30 | 20 | 1308 | GgsCdsAgsGgsTdsTdsCdsTdsCdsCds GdsCdsGgsGgsTdsGg | 1385. 1 | [M-4H]4- |
| 31 | 20 | 1308 | GlsCdsAlsGlsTdsTdsCdsTdsCdsCds GdsCdsGlsGdsTlsGl | 1323.4 | [M-4H]4- |
| 32 | 97 | 1308 | GgsCdsAgsGdsTdsTdsCdsTdsCdsCds GdsMgsGdsGgsTdsGg | 1828. 3 | [M-3H]3- |
| 33 | 21 | 1308 | GlsMlsAlsGdsTdsTdsCdsTdsCdsCds GdsCdsGdsGlsTlsGl | 1769.4 | FM-3H]3- |
| 34 | 21 | 1308 | GlsMlsAlsGlsTdsTdsCdsTdsCdsCds GdsCdsGlsGlsTlsGl | 1788.3 | [M-3H]3- |
| 35 | 21 | 1308 | GlsMlsAdsGlsTdsTdsCdsTdsCdsCds GdsCdsGlsGdsTlsGl | 1769.2 | [M-3H]3- |
| 36 | 21 | 1308 | GmsMmsAmsGdsTdsTdsCdsTdsCdsCds GdsCdsGdsGmsTmsGm | 1923. 5 | [M-3H]3- |
| 37 | 21 | 1308 | GgsMgsAgsGdsTdsTdsCdsTdsCdsCds GdsCdsGdsGgsTgsGg | 1851.7 | [M-3H]3- |
| 38 | 21 | 1308 | GgsMgsAgsGdsTdsTdsCdsTdsCdsCds GdsCdsGgsGdsTgsGg | 1388. 3 | [M-4H]4- |
| 39 | 21 | 1308 | GgsMgsAdsGgsTdsTdsCdsTdsCdsCds GdsCdsGgsGdsTgsGg | 1389. 1 | [M-4H]4- |
| 40 | 21 | 1308 | GlsMlsAdsGlsTdsTdsCdsTdsCdsCds GdsCdsGlsGlsTdsGl | 1769.5 | [M-3H]3- |
| 41 | 22 | 1308 | GmsMmsAdsGmsTdsTdsMdsTdsMdsMds GdsMdsGmsGdsTmsGm | 1165. 5 | [M-5H]5- |
| 42 | 22 | 1308 | GmsMdsAmsGmsTdsTdsMdsTdsMdsMds GdsMdsGmsGmsTdsGm | 1165.4 | [M-5H]5- |
| 43 | 22 | 1308 | GmsMmsAdsGmsTdsTdsMdsTdsMdsMds GdsMdsGmsGmsTdsGm | 1165.2 | [M-5H]5- |
| 44 | 22 | 1308 | GmsMmsAdsGmsTdsTdsMdsTdsMdsMds GdsMdsGmsGdsTdsGm | 1144.2 | [M-5H]5- |
| 45 | 22 | 1308 | GmsMmsAdsGmsTdsTdsMdsTdsMdsMds GdsMmsGdsGmsTdsGm | 1165.2 | [M-5H]5- |

**[Table 1-3]**

| **(Table 1 continued)** | | | | | |
|---|---|---|---|---|---|
| 46 | 22 | 1308 | GlsMlsAdsGlsTdsTdsMdsTdsMdsMds GdsMdsGlsGlsTdsGl | 1341.2 | [M-4H]4- |
| 47 | 22 | 1308 | GlsMlsAdsGlsTdsTdsMdsTdsMdsMds GdsMdsGlsGdsTdsGl | 1334. 2 | [M-4H]4- |
| 48 | 23 | 1308 | GlsGlsMlsAdsGdsTdsTdsCdsTdsCds CdsGdsCdsGdsGlsTlsGl | 1884. 5 | [M-3H]3- |
| 49 | 24 | 1308 | GmsGdsMmsAdsGmsTdsTdsMdsTdsMds MdsGdsMdsGmsGdsTdsGm | 1213.3 | [M-5H]5- |
| 50 | 24 | 1308 | GmsGdsMmsAdsGmsTdsTdsMdsTdsMds MdsGdsMdsGmsGmsTdsGm | 1028. 5 | [M-6H]6- |
| 51 | 24 | 1308 | GlsGdsMlsAdsGlsTdsTdsMdsTdsMds MdsGdsMdsGlsGdsTdsGl | 1136.2 | [M-5H]5- |
| 52 | 24 | 1308 | GlsGdsMlsAdsGlsTdsTdsMdsTdsMds MdsGdsMdsGlsGlsTdsGl | 1141.8 | [M-5H]5- |
| 53 | 25 | 1309 | MgsAgsGgsTdsTdsCdsTdsCdsCdsGds CdsGgsGgsTg | 1621.0 | [M-3H]3- |
| 54 | 26 | 1309 | GmsCdsAmsGmsTdsTdsCdsTdsCdsCdsGdsMm sGmsGdsTm | 1356. 7 | [M-4H]4- |
| 55 | 3 | 1309 | GlsMlsAlsGdsTdsTdsCdsTdsCdsCds GdsCdsGlsGlsTl | 1654. 3 | [M-3H]3- |
| 56 | 3 | 1309 | GlsMlsAdsGdsTdsTdsCdsTdsCdsCds GdsCdsGlsGlsTl | 4936.4 | [M-H]- |
| 57 | 3 | 1309 | GlsMlsAlsGdsTdsTdsCdsTdsCdsCds GdsCdsGdsGlsTl | 1645.4 | [M-3H]3- |
| 58 | 3 | 1309 | GgsMgsAgsGdsTdsTdsCdsTdsCdsCds GdsCdsGgsGgsTg | 1736. 8 | [M-3H]3- |
| 59 | 3 | 1309 | GgsMgsAdsGdsTdsTdsCdsTdsCdsCds GdsCdsGgsGgsTg | 1285. 1 | [M-4H]4- |
| 60 | 3 | 1309 | GlsMlsAgsGdsTdsTdsCdsTdsCdsCds GdsCdsGgsGlsTl | 1261. 1 | [M-4H]4- |
| 61 | 3 | 1309 | GlsMgsAlsGdsTdsTdsCdsTdsCdsCds GdsCdsGlsGgsTl | 1261. 1 | [M-4H]4- |
| 62 | 3 | 1309 | GgsMlsAlsGdsTdsTdsCdsTdsCdsCds GdsCdsGlsGlsTg | 1261. 1 | [M-4H]4- |
| 63 | 3 | 1309 | GlsMlsAdsGlsTdsTdsCdsTdsCdsCds GdsCdsGlsGlsTl | 1240.6 | [M-4H]4- |
| 64 | 27 | 1309 | GmsMmsAdsGmsTdsTdsCdsTdsCdsCds GdsMmsGdsGmsTm | 1360. 1 | [M-4H]4- |
| 65 | 28 | 1309 | GgsMgsAgsGdsTdsTdsMdsTdsMdsMds GdsMdsGgsGgsTg | 1316. 1 | [M-4H]4- |
| 66 | 29 | 1309 | GmsMmsAmsGdsTdsTdsMdsTdsMdsMds GdsCosGmsGmsTm | 1374.8 | [M-4H]4- |
| 67 | 29 | 1309 | GmsMmsAmsGosTdsTdsMdsTdsMdsMds GdsCosGmsGmsTm | 1381.9 | [M-4H]4- |
| 68 | 29 | 1309 | GmsMmsAmsGdsTdsTdsMdsTdsMdsMds GosCosGmsGmsTm | 1381.9 | [M-4H]4- |

**[Table 1-4]**

| **(Table 1 continued)** | | | | | |
|---|---|---|---|---|---|
| 69 | 30 | 1309 | GlsMlsAlsGdsTdsTdsMdsTdsMdsMds GdsMdsGlsGlsTl | 1254.6 | [M-4H]4- |
| 70 | 30 | 1309 | GmsMmsAmsGdsTdsTdsMdsTdsMdsMds GdsMdsGmsGmsTm | 1370.2 | [M-4H]4- |
| 71 | 30 | 1309 | GtsMtsAtsGdsTdsTdsMdsTdsMdsMds GdsMdsGtsGtsTt | 1395.9 | [M-4H]4- |
| 72 | 30 | 1309 | GusMusAusGdsTdsTdsMdsTdsMdsMds GdsMdsGusGusTu | 1338. 8 | [M-4H]4- |
| 73 | 30 | 1309 | GpsMpsApsGdsTdsTdsMdsTdsMdsMds GdsMdsGpsGpsTp | 1381.3 | [M-4H]4- |
| 74 | 30 | 1309 | GmsMmsAmsGosTdsTdsMdsTdsMdsMds GdsMdsGmsGmsTm | 1377.9 | [M-4H]4- |
| 75 | 31 | 1309 | GmsMmsAmsGosUosTdsMdsTdsMdsMds GdsCosGmsGmsTm | 1385.9 | [M-4H]4- |
| 76 | 32 | 1309 | GmsMmsAmsGosUosTdsMdsTdsMdsMds GdsMdsGmsGmsTm | 1381.9 | [M-4H]4- |
| 77 | 33 | 1309 | GgsGgsCdsAgsGdsTdsTdsCdsTdsCds CdsGdsCdsGgsGgsTg | 1384.9 | [M-4H]4- |
| 78 | 33 | 1309 | GlsGlsCdsAdsGlsTdsTdsCdsTdsCds CdsGlsCdsGdsGlsTl | 1323. 6 | [M-4H]4- |
| 79 | 34 | 1309 | GlsGlsMlsAdsGdsTdsTdsCdsTdsCds CdsGdsCdsGlsGlsTl | 1769.2 | [M-3H]3- |
| 80 | 34 | 1309 | GlsGdsMlsAdsGdsTdsTdsCdsTdsCds CdsGdsCdsGlsGdsTl | 1750.7 | [M-3H]3- |
| 81 | 34 | 1309 | GgsGgsMgsAdsGdsTdsTdsCdsTdsCds CdsGdsCdsGgsGgsTg | 1388. 6 | [M-4H]4- |
| 82 | 34 | 1309 | GmsGmsMmsAdsGdsTdsTdsCdsTdsCds CdsGdsCdsGmsGmsTm | 1442.3 | [M-4H]4- |
| 83 | 35 | 1309 | GgsGgsMgsAdsGdsTdsTdsMdsTdsMds MdsGdsMdsGgsGgsTg | 1402.4 | [M-4H]4- |
| 84 | 35 | 1309 | GlsGlsMlsAdsGdsTdsTdsMdsTdsMds MdsGdsMdsGlsGlsTl | 1340.9 | [M-4H]4- |
| 85 | 36 | 1309 | TmsGmsGmsCdsAdsGdsTdsTdsCdsTds CdsCdsGdsCdsGmsGmsTm | 2025.9 | [M-3H]3- |
| 86 | 36 | 1309 | TgsGgsGgsCdsAdsGdsTdsTdsCdsTds CdsCdsGdsCdsGgsGgsTg | 1953. 3 | [M-3H]3- |
| 87 | 37 | 1310 | GlsMlsAlsGdsTdsTdsCdsTdsCdsCds GdsCdsGlsGl | 1538.2 | [M-3H]3- |
| 88 | 37 | 1310 | GgsMgsAgsGdsTdsTdsCdsTdsCdsCds GdsCdsGgsGg | 1204. 8 | [M-4H]4- |
| 89 | 38 | 1310 | GlsGlsCdsAdsGdsTdsTdsCdsTdsCds CdsGdsMlsGlsGl | 1653. 1 | [M-3H]3- |
| 90 | 39 | 1310 | GlsGlsMlsAdsGdsTdsTdsCdsTdsCds CdsGdsCdsGlsGl | 1653.4 | [M-3H]3- |
| 91 | 39 | 1310 | GgsGgsMgsAdsGdsTdsTdsCdsTdsCds CdsGdsCdsGgsGg | 1291. 1 | [M-4H]4- |

**[Table 1-5]**

| **(Table 1 continued)** | | | | | |
|---|---|---|---|---|---|
| 92 | 40 | 1310 | GgsGgsMgsAdsGdsTdsTdsCdsTdsCds CdsGdsMgsGgsGg | 1312.0 | [M-4H]4- |
| 93 | 41 | 1310 | GlsGlsMlsAdsGdsTdsTdsMdsTdsMds MdsGdsMdsGlsGl | 1253.9 | [M-4H]4- |
| 94 | 42 | 1472 | MlsGlsTlsCdsGdsGdsAdsAdsGdsGds TdsGlsGlsGl | 4774.58 | [M-H]- |
| 95 | 43 | 1472 | GlsMlsGlsTdsCdsGdsGdsAdsAdsGds GdsTdsGlsGlsGl | 1705. 7 | [M-3H]3- |
| 96 | 44 | 1472 | AlsGlsMlsGdsTdsCdsGdsGdsAdsAds GdsGdsTdsGlsGlsGl | 1815. 1 | [M-3H]3- |
| 97 | 45 | 1472 | MgsAgsGgsCdsGdsTdsCdsGdsGdsAds AdsGdsGdsTgsGgsGdsGg | 1499.9 | [M-4H]4- |
| 98 | 46 | 1473 | AlsGlsMlsGdsTdsCdsGdsGdsAdsAds GdsGdsTlsGlsGl | 1700.3 | [M-3H]3- |
| 99 | 46 | 1473 | AgsGgsMgsGdsTdsCdsGdsGdsAdsAds GdsGdsTgsGgsGg | 1336. 7 | [M-4H]4- |
| 100 | 47 | 1473 | AlsGlsMlsGdsTdsMdsGdsGdsAdsAds GdsGdsTlsGlsGl | 1278. 6 | [M-4H]4- |
| 101 | 48 | 1473 | MlsAlsGlsCdsGdsTdsCdsGdsGdsAds AdsGdsGdsTlsGlsGl | 1801.8 | [M-3H]3- |
| 102 | 48 | 1473 | MmsAmsGmsCdsGdsTdsCdsGdsGdsAds AdsGdsGdsTmsGmsGm | 1956. 1 | [M-3H]3- |
| 103 | 48 | 1473 | MgsAgsGgsCdsGdsTdsCdsGdsGdsAds AdsGdsGdsTgsGgsGg | 1883. 9 | [M-3H]3- |
| 104 | 48 | 1473 | MgsAgsGgsCdsGdsTdsCdsGdsGdsAds AdsGdsGgsTgsGdsGg | 1413.0 | [M-4H]4- |
| 105 | 49 | 1473 | MgsAgsGgsMdsGdsTdsMdsGdsGdsAds AdsGdsGdsTgsGgsGg | 1419.9 | [M-4H]4- |
| 106 | 50 | 1473 | AgsMgsAdsGgsCdsGdsTdsCdsGdsGds AdsAdsGdsGdsTgsGgsGg | 1495. 5 | [M-4H]4- |
| 107 | 50 | 1473 | AgsMgsAgsGdsCdsGdsTdsCdsGdsGds AdsAdsGdsGdsTgsGgsGg | 1495. 1 | [M-4H]4- |
| 108 | 51 | 1474 | MlsAlsGlsCdsGdsTdsCdsGdsGdsAds AdsGdsGdsTlsGl | 1678. 1 | [M-3H]3- |
| 109 | 52 | 1474 | AlsMlsAlsGdsCdsGdsTdsCdsGdsGds AdsAdsGdsGlsTlsGl | 1797. 1 | [M-3H]3- |
| 110 | 52 | 1474 | AlsMlsAlsGdsCdsGdsTdsCdsGdsGds AdsAdsGlsGlsTlsGl | 1354. 3 | [M-4H]4- |
| 111 | 53 | 1475 | GlsAlsMlsAlsGdsCdsGdsTdsCdsGds GdsAdsAdsGlsGlsTl | 1354.4 | [M-4H]4- |
| 112 | 54 | 1476 | GlsAlsMlsAdsGdsCdsGdsTdsCdsGds GdsAdsAlsGlsGl | 5073. 96 | [M-H]- |
| 113 | 55 | 1476 | AlsGlsAlsCdsAdsGdsCdsGdsTdsCds GdsGdsAdsAlsGlsGl | 1795. 1 | [M-3H]3- |
| 114 | 56 | 1480 | MgsMgsTgsAdsGdsAdsCdsAdsGdsCds GdsTdsMgsGgsGg | 1313. 8 | [M-4H]4- |

**[Table 1-6]**

| **(Table 1 continued)** | | | | | |
|---|---|---|---|---|---|
| 115 | 57 | 1480 | GlsCdsMlsTlsAdsGdsAdsCdsAdsGds CdsGdsTlsMlsGdsGl | 1334.9 | [M-4H]4- |
| 116 | 58 | 1480 | GlsMlsCdsTlsAdsGdsAdsCdsAdsGds CdsGdsTlsCdsGlsGl | 1331. 3 | [M-4H]4- |
| 117 | 59 | 1480 | GlsMlsCdsTlsAdsGdsAdsCdsAdsGds CdsGdsTdsMlsGlsGl | 1335. 1 | [M-4H]4- |
| 118 | 59 | 1480 | GlsMlsCdsTdsAdsGdsAlsCdsAdsGds CdsGdsTdsMlsGlsGl | 1335. 2 | [M-4H]4- |
| 119 | 60 | 1480 | GlsMlsMlsTdsAdsGdsAdsCdsAdsGdsCdsGl sTdsCdsGlsGl | 1334.9 | [M-4H]4- |
| 120 | 60 | 1480 | GlsMlsMlsTdsAdsGdsAdsCdsAdsGls CdsGdsTdsCdsGlsGl | 1335. 2 | [M-4H]4- |
| 121 | 4 | 1480 | GgsMgsMgsTdsAdsGdsAdsCdsAdsGds CdsGdsTdsMgsGgsGg | 1399. 7 | [M-4H]4- |
| 122 | 4 | 1480 | GlsMlsMlsTlsAdsGdsAdsCdsAdsGds CdsGdsTdsMlsGlsGl | 1345.4 | [M-4H]4- |
| 123 | 4 | 1480 | GmsMmsMmsTdsAdsGdsAdsCdsAdsGds CdsGdsTdsMmsGmsGm | 1454.0 | [M-4H]4- |
| 124 | 61 | 1480 | GlsMlsMlsTdsAdsGdsAdsMdsAdsGds MdsGdsTdsMlsGlsGl | 1345. 3 | [M-4H]4- |
| 125 | 61 | 1480 | GtsMtsMtsTdsAdsGdsAdsMdsAdsGds MdsGdsTdsMtsGtsGt | 1189.2 | [M-5H]5- |
| 126 | 61 | 1480 | GusMusMusTdsAdsGdsAdsMdsAdsGds MdsGdsTdsMusGusGu | 1143.5 | [M-5H]5- |
| 127 | 61 | 1480 | GmsMmsMmsTdsAdsGdsAdsMdsAdsGds MdsGdsTdsMmsGmsGm | 1168.8 | [M-5H]5- |
| 128 | 62 | 1480 | GmsMmsMmsTdsAdsGdsAdsMdsAdsGds MdsGdsUosMmsGmsGm | 1172.0 | [M-5H]5- |
| 129 | 62 | 1480 | GmsMmsMmsTdsAdsGdsAdsMdsAdsGds MdsGosUosMmsGmsGm | 1178.0 | [M-5H]5- |
| 130 | 63 | 1480 | GmsMmsMmsUosAdsGdsAdsMdsAdsGds MdsGdsTdsMmsGmsGm | 1172.0 | [M-5H]5- |
| 131 | 63 | 1480 | GmsMmsMmsUosAosGdsAdsMdsAdsGds MdsGdsTdsMmsGmsGm | 1178.0 | [M-5H]5- |
| 132 | 64 | 1480 | GmsMmsMmsUosAdsGdsAdsMdsAdsGds MdsGdsUosMmsGmsGm | 1175. 2 | [M-5H]5- |
| 133 | 65 | 214 | MlsTlsMlsAdsGdsCdsTdsGdsGdsCds GdsTlsGlsAl | 4683. 56 | [M-H]- |
| 134 | 66 | 1323 | MlsMlsAlsGdsGdsAdsAdsAdsGdsAds AdsTlsGlsGl | 4748.41 | [M-H]- |
| 135 | 67 | 1458 | GlsGlsGlsAdsGdsAdsCdsAdsTdsTds CdsAlsGlsMl | 4701. 26 | [M-H]- |
| 136 | 68 | 1495 | MlsTlsAlsAdsTdsCdsCdsAdsGdsGds TdsTlsTlsGl | 4641. 35 | [M-H]- |

### Example 6

### In vitro DUX4 knockdown activity test (Lipofection method)

C2C12 cells were seeded at 1.25 × 10⁴ cells/cm² on a transfection reagent in which a DUX4 modified oligonucleotide and Lipofectamine RNAi Reagent were mixed, and were cultured overnight in a CO₂ incubator. The next day, the cells were transfected with a reporter plasmid in which a DUX4 sequence was cloned into multiple cloning sites of a psiCHECK-2 vector (Promega) using Lipofectamine 2000 Reagent, and were cultured in a CO₂ incubator for about 24 hours. After that, using a Dual-Glo Luciferase Assay System, intracellular Firefly luciferase and Renilla luciferase luminescence values were detected with a plate reader. In order to correct influence of transfection efficiency and the number of the cells from a luminescence value due to Renilla luciferase activity, a ratio to a luminescence value of Firefly luciferase activity was calculated. An inhibition rate was calculated as a percentage from a reduction rate of Renilla/Firefly when a modified oligonucleotide was added, and an IC₅₀ value was calculated from concentrations at two points sandwiching 50% and an inhibition rate at that time (Table 2). As compared to compounds (Compound Nos. 1 - 132) that are complementary to positions 232 - 248, 1306 - 1325 or 1472 - 1495 of DUX4 mature mRNA, Compound Nos 133 (complementary to positions 214 - 227 of SEQ ID NO: 1 in the sequence listing), 134 (complementary to positions 1323 - 1336 of SEQ ID NO: 1 in the sequence listing), 135 (complementary to positions 1458 - 1471 of SEQ ID NO: 1 in the sequence listing), and 136 (complementary to positions 1495 - 1508 of SEQ ID NO: 1 in the sequence listing) were found to have significantly lower inhibition rates.

### Example 7

### In Vitro DUX4 Knockdown Activity Test (Gymnosis Method)

C2C12 cells were seeded at 6 × 10³ cells/cm² in a DUX4 modified oligonucleotide solution and cultured in a CO₂ incubator for 2 nights. Two days later, a culture medium containing a DUX4 modified oligonucleotide solution was removed from the cells, and the cells were washed with a fresh culture medium. After that, the cells were transfected with a reporter plasmid in which a DUX4 sequence was cloned into multiple cloning sites of a psiCHECK-2 vector (Promega) using Lipofectamine 2000 Reagent, and were cultured in a CO₂ incubator for about 24 hours. After that, using a Dual-Glo Luciferase Assay System, intracellular Firefly luciferase and Renilla luciferase luminescence values were detected with a plate reader. In order to correct influence of transfection efficiency and the number of the cells from a luminescence value due to Renilla luciferase activity, a ratio to a luminescence value of Firefly luciferase activity was calculated. An inhibition rate was calculated as a percentage from a reduction rate of Renilla/Firefly when a modified oligonucleotide was added, and an IC₅₀ value was calculated from concentrations at two points sandwiching 50% and an inhibition rate at that time. The results are shown in Table 2 below. As compared to compounds (Compound Nos. 1 - 132) containing a nucleobase sequence complementary to an equal length portion in a region of positions 232 - 248, 1306 - 1325 or 1472 - 1495 of DUX4 mature mRNA, Compound Nos 133 (complementary to positions 214 - 227 of SEQ ID NO: 1 in the sequence listing), 134 (complementary to positions 1323 - 1336 of SEQ ID NO: 1 in the sequence listing), 135 (complementary to positions 1458 - 1471 of SEQ ID NO: 1 in the sequence listing), and 136 (complementary to positions 1495 - 1508 of SEQ ID NO: 1 in the sequence listing) were found to have significantly lower inhibition rates.

### [Table 2-1]

**Table 2**

| Compound No. | I C ₅₀ [uM] | |
|---|---|---|
| | Lipofection Method | Gymnosis Method |
| 1 | 0.004 | 0. 108 |
| 2 | 0.008 | 0. 084 |
| 3 | 0.003 | 0. 142 |
| 4 | 0.098 | 0.829 |
| 5 | 0.027 | 0. 341 |
| 6 | 0.007 | 0. 128 |
| 7 | 0.009 | 0. 378 |
| 8 | 0.022 | 0.495 |
| 9 | 0.018 | 0. 340 |
| 10 | 0. 006 | 0. 182 |
| 11 | 0.008 | 0.114 |
| 12 | 0.020 | 0.423 |
| 13 | 0.015 | 0.242 |
| 14 | 0.002 | 0.079 |
| 15 | 0.010 | 0. 128 |
| 16 | 0. 008 | 0.091 |
| 17 | 0.006 | 0.079 |
| 18 | 0.016 | 0. 394 |
| 19 | 0.006 | 0. 158 |
| 20 | 0.018 | 0.724 |
| 21 | 0.006 | 0.964 |
| 22 | 0. 004 | 0.280 |
| 23 | 0.007 | 0. 365 |
| 24 | 0.018 | 0. 188 |
| 25 | 0.009 | 0.447 |
| 26 | 0.043 | 0.259 |
| 27 | 0.037 | 0.201 |
| 28 | 0. 003 | 0.080 |
| 29 | 0.010 | 0. 556 |
| 30 | 0.006 | 0. 559 |
| 31 | 0.006 | 0.117 |
| 32 | 0.007 | 0.404 |
| 33 | 0.007 | 0.272 |
| 34 | 0. 008 | 0.073 |

**[Table 2-2]**

| **(Table 2 continued)** | | |
|---|---|---|
| 35 | 0.009 | 0. 105 |
| 36 | 0.006 | 0. 390 |
| 37 | 0.003 | 0.133 |
| 38 | 0.005 | 0. 125 |
| 39 | 0.006 | 0. 143 |
| 40 | 0.002 | 0.051 |
| 41 | 0. 007 | 0. 184 |
| 42 | 0.008 | 0. 166 |
| 43 | 0.008 | 0. 148 |
| 44 | 0.009 | 0.174 |
| 45 | 0.023 | 0.699 |
| 46 | 0.003 | 0. 109 |
| 47 | 0.005 | 0.181 |
| 48 | 0. 005 | 0. 166 |
| 49 | 0.009 | 0.261 |
| 50 | 0.003 | 0.206 |
| 51 | 0.005 | 0.419 |
| 52 | 0.006 | 0.414 |
| 53 | 0.016 | 0. 164 |
| 54 | 0. 010 | 0.402 |
| 55 | 0.006 | 0.052 |
| 56 | <0.03 | 0. 577 |
| 57 | 0.012 | 0.435 |
| 58 | 0.002 | 0.058 |
| 59 | 0.002 | 0. 052 |
| 60 | 0.005 | 0.100 |
| 61 | 0.002 | 0.048 |
| 62 | 0.003 | 0.038 |
| 63 | 0.004 | 0.090 |
| 64 | 0.019 | 0. 426 |
| 65 | 0.002 | 0. 128 |
| 66 | 0.002 | 0.066 |
| 67 | 0.003 | 0. 188 |
| 68 | 0.013 | 0.850 |
| 69 | 0.002 | 0.083 |
| 70 | 0.005 | 0. 111 |

**[Table 2-3]**

| **(Table 2 continued)** | | |
|---|---|---|
| 71 | 0.002 | 0.037 |
| 72 | 0.002 | 0.072 |
| 73 | 0.005 | 0.292 |
| 74 | 0.002 | 0.079 |
| 75 | 0.005 | 0. 237 |
| 76 | 0.003 | 0.073 |
| 77 | 0.004 | 0.091 |
| 78 | 0.010 | 0.132 |
| 79 | 0.010 | 0.099 |
| 80 | 0.009 | 0. 373 |
| 81 | 0. 003 | 0.115 |
| 82 | 0.011 | 0. 529 |
| 83 | 0.003 | 0.113 |
| 84 | 0.007 | 0.207 |
| 85 | 0.017 | 0.400 |
| 86 | 0.005 | 0. 106 |
| 87 | 0. 005 | 0.097 |
| 88 | 0.007 | 0. 154 |
| 89 | 0.019 | 0.434 |
| 90 | 0.008 | 0.077 |
| 91 | 0.009 | 0. 148 |
| 92 | 0.015 | 0. 763 |
| 93 | 0.007 | 0. 135 |
| 94 | <0.03 | 0. 793 |
| 95 | 0.021 | 0.496 |
| 96 | 0.036 | 0. 306 |
| 97 | 0.009 | 0. 546 |
| 98 | 0.010 | 0. 363 |
| 99 | 0.008 | 0.624 |
| 100 | 0.014 | 0. 669 |
| 101 | 0.029 | 0.443 |
| 102 | 0.014 | 0.657 |
| 103 | 0.011 | 0. 307 |
| 104 | 0.007 | 0.255 |
| 105 | 0.005 | 0.122 |
| 106 | 0. 006 | 0.291 |

**[Table 2-4]**

| **(Table 2 continued)** | | |
|---|---|---|
| 107 | 0.004 | 0. 321 |
| 108 | 0.076 | 0.877 |
| 109 | 0.075 | 0. 730 |
| 110 | 0.020 | 0.266 |
| 111 | 0.017 | 0. 223 |
| 112 | 0.050 | 0.616 |
| 113 | 0.016 | 0. 569 |
| 114 | 0.013 | 0.259 |
| 115 | 0.011 | 0. 531 |
| 116 | 0.003 | 0.112 |
| 117 | 0.003 | 0. 092 |
| 118 | 0.015 | 0.461 |
| 119 | 0.004 | 0.467 |
| 120 | 0.004 | 0.456 |
| 121 | 0.008 | 0.239 |
| 122 | 0.006 | 0. 289 |
| 123 | 0.013 | 0. 154 |
| 124 | 0.003 | 0.338 |
| 125 | 0.010 | 0.272 |
| 126 | 0.004 | 0. 159 |
| 127 | 0.003 | 0.093 |
| 128 | 0.006 | 0. 256 |
| 129 | 0.008 | 0.088 |
| 130 | 0.003 | 0.079 |
| 131 | 0.005 | 0.245 |
| 132 | 0.006 | 0.294 |
| 133 | >0. 3 | >3 |
| 134 | >0. 3 | >3 |
| 135 | 0. 140 | >3 |
| 136 | >0. 3 | >3 |

### Example 8

### Synthesis of Modified Oligonucleotide Compound and In Vitro DUX4 Knockdown Activity Test (Gymnosis Method)

Table 3 shows newly synthesized modified oligonucleotide compounds and results of in vitro DUX4 knockdown activity tests performed on the compounds in the same manner as in Example 7.

In the notation of the compounds, each nucleotide is represented by three letters. However, a 3'-terminal nucleotide is represented by two letters since there is no internucleoside linkage.
1) The first letter is capitalized and indicates the following nucleobases:
   A = adenine, T = thymine, G = guanine, C = cytosine, U = uracil, M = 5-methylcytosine;
2) the second letter indicates the following sugar moieties:
   1 = LNA, m = ALNA[Ms], e = 2'-MOE, o = 2'-OMe, d = 2'-deoxyribose,
3) the third letter indicates the following internucleoside linkages:
   s = phosphorothioate, p = phosphodiester.

A target position indicates a 5' target site of DUX4 mature mRNA of a modified oligonucleotide (a position of SEQ ID NO: 1 in the sequence listing corresponding to a 3' end of a modified oligonucleotide).

As compared to compounds (Compound Nos. 137 - 247) containing a nucleobase sequence complementary to an equal length portion in a region of positions 126 - 147, 232 - 248, 1306 - 1325 or 1472 - 1495 of DUX4 mature mRNA, Compound Nos 248 (complementary to positions 112 - 127 of SEQ ID NO: 1 in the sequence listing), 249 (complementary to positions 162 - 177 of SEQ ID NO: 1 in the sequence listing), 250 (complementary to positions 264 - 279 of SEQ ID NO: 1 in the sequence listing), and 251 (complementary to positions 1273 - 1288 of SEQ ID NO: 1 in the sequence listing) were found to have significantly lower inhibition rates.

### [Table 3-1]

**Table 3**

| Compund No. | Sequ ence No. | Target Position | Sequence | m/z | Ion Species | IC50(uM) Gymnosis Method |
|---|---|---|---|---|---|---|
| 137 | 75 | 128 | GmsTmsGmsGdsCdsGdsAdsTdsGdsCdsCdsCdsGdsGmsGmsTm | 1940.3 | [M-3H]3- | 0.112 |
| 138 | 79 | 1304 | GmsMmsAdsGdsTdsTdsCdsTdsCdsCdsGdsCdsGdsGdsTdsGdsTdsGdsGmsAm | 1725.0 | [M-4H]4- | <0.3 |
| 139 | 81 | 1307 | AmsTmsGdsGdsCdsAdsGdsTdsTdsCdsTdsCdsCdsGdsCdsGdsGdsTdsGmsTm | 2287.8 | [M-3H]3- | <0.3 |
| 140 | 80 | 1306 | GmsGmsCdsAdsGdsTdsTdsCdsTdsCdsCdsGdsCdsGdsGdsTdsGdsTmsGm | 2185.0 | [M-3H]3- | <0.3 |
| 141 | 82 | 1307 | TmsGmsGdsCdsAdsGdsTdsTdsCdsTdsCdsCdsGdsCdsGdsGdsTdsGmsTm | 2177.9 | [M-3H]3- | <0.3 |
| 142 | 95 | 1307 | GmsGmsCdsAdsGdsTdsTdsCdsTdsCdsCdsGdsCdsGdsGdsTdsGmsTm | 2070.5 | [M-3H]3- | <0.3 |
| 143 | 83 | 1308 | TmsGmsGdsCdsAdsGdsTdsTdsCdsTdsCdsCdsGdsCdsGdsGdsTmsGm | 2070.8 | [M-3H]3- | <0.3 |
| 144 | 16 | 1307 | GmsMmsAmsGdsTdsTdsCdsTdsCdsCdsGdsCdsGdsGdsTmsGmsTm | 2030.0 | [M-3H]3- | 0.180 |
| 145 | 23 | 1308 | GmsGmsMmsAdsGdsTdsTdsCdsTdsCdsCdsGdsCdsGdsGmsTmsGm | 2038.1 | [M-3H]3- | 0.180 |
| 146 | 84 | 1476 | GmsMmsCdsTdsAdsGdsAdsCdsAdsGdsCdsGdsTdsCdsGdsGdsAdsAdsGmsGm | 2308.5 | [M-3H]3- | <0.3 |
| 147 | 87 | 1479 | TmsTmsTdsGdsCdsCdsTdsAdsGdsAdsCdsAdsGdsCdsGdsTdsCdsGdsGmsAm | 2284.2 | [M-3H]3- | <0.3 |
| 148 | 90 | 1480 | GmsTmsTdsTdsGdsCdsCdsTdsAdsGdsAdsCdsAdsGdsCdsGdsTdsCdsGmsGm | 2290.1 | [M-3H]3- | <0.3 |
| 149 | 88 | 1479 | TmsGmsCdsCdsTdsAdsGdsAdsCdsAdsGdsCdsGdsTdsCdsGdsGmsAm | 2071.2 | [M-3H]3- | <0.3 |
| 150 | 91 | 1480 | TmsTmsGdsCdsCdsTdsAdsGdsAdsCdsAdsGdsCdsGdsTdsCdsGmsGm | 2068.4 | [M-3H]3- | <0.3 |
| 151 | 89 | 1479 | GmsMmsMmsTdsAdsGdsAdsCdsAdsGdsCdsGdsTdsCdsGmsGmsAm | 2044.0 | [M-3H]3- | 0.237 |
| 152 | 92 | 1480 | TmsGmsMmsCdsTdsAdsGdsAdsCdsAdsGdsCdsGdsTdsMmsGmsGm | 2042.6 | [M-3H]3- | <0.3 |
| 153 | 4 | 1480 | GmsMmpMmsTdsAdsGdsAdsCdsAdsGdsCdsGdsTdsMmpGmsGm | 1928.3 | [M-3H]3- | 0.077 |
| 154 | 4 | 1480 | GmpMmpMmsTdsAdsGdsAdsCdsAdsGdsCdsGdsTdsMmpGmpGm | 1917.5 | [M-3H]3- | <0.3 |
| 155 | 60 | 1480 | GmsMmsMmsTmsAmsGdsAdsCdsAdsGdsCdsGdsTdsCdsGdsGd | 1898.8 | [M-3H]3- | <0.3 |
| 156 | 102 | 1480 | GmsMmsMmsUosAdsGdsAdsCdsAdsGdsCdsGdsTdsMmsGmsGm | 1944.3 | [M-3H]3- | 0.023 |
| 157 | 4 | 1480 | GmsMmsMmsTdsAosGdsAdsCdsAdsGdsCdsGdsTdsMmsGmsGm | 1949.0 | [M-3H]3- | 0.029 |
| 158 | 4 | 1480 | GmsMmsMmsTdsAdsGosAdsCdsAdsGdsCdsGdsTdsMmsGmsGm | 1949.0 | [M-3H]3- | 0.070 |
| 159 | 4 | 1480 | GmsMmsMmsTdsAdsGdsAosCdsAdsGdsCdsGdsTdsMmsGmsGm | 1948.9 | [M-3H]3- | <0.3 |
| 160 | 4 | 1480 | GmsMmsMmsTdsAdsGdsAdsCosAdsGdsCdsGdsTdsMmsGmsGm | 1949.3 | [M-3H]3- | 0.565 |
| 161 | 4 | 1480 | GmsMmsMmsTdsAdsGdsAdsCdsAosGdsCdsGdsTdsMmsGmsGm | 1949.0 | [M-3H]3- | 0.024 |
| 162 | 4 | 1480 | GmsMmsMmsTdsAdsGdsAdsCdsAdsGosCdsGdsTdsMmsGmsGm | 1948.6 | [M-3H]3- | <0.3 |
| 163 | 4 | 1480 | GmsMmsMmsTdsAdsGdsAdsCdsAdsGdsCosGdsTdsMmsGmsGm | 1949.0 | [M-3H]3- | 0.097 |
| 164 | 4 | 1480 | GmsMmsMmsTdsAdsGdsAdsCdsAdsGdsCdsGosTdsMmsGmsGm | 1949.3 | [M-3H]3- | <0.3 |
| 165 | 103 | 1480 | GmsMmsMmsTdsAdsGdsAdsCdsAdsGdsCdsGdsUosMmsGmsGm | 1943.8 | [M-3H]3- | 0.125 |
| 166 | 102 | 1480 | GmsMmsMmsUosAosGdsAdsCdsAdsGdsCdsGdsTdsMmsGmsGm | 1954.1 | [M-3H]3- | 0.057 |
| 167 | 105 | 1480 | GmsMmsMmsUosAdsGdsAdsCdsAdsGdsCdsGdsUosMmsGmsGm | 1950.0 | [M-3H]3- | 0.056 |
| 168 | 103 | 1480 | GmsMmsMmsTdsAdsGdsAdsCdsAdsGdsCdsGosUosMmsGmsGm | 1954.4 | [M-3H]3- | 0.078 |
| 169 | 93 | 1480 | GlsMlsMlsTdsAosGdsAdsCdsAdsGdsCdsGdsTdsMlsGlsGl | 1794.8 | [M-3H]3- | 0.049 |
| 170 | 104 | 1480 | GlsMlsMlsUosAosGdsAdsCdsAdsGdsCdsGdsTdsMlsGlsGl | 1800.2 | [M-3H]3- | 0.025 |
| 171 | 93 | 1480 | GlsMlpMlsTdsAdsGdsAdsCdsAdsGdsCdsGdsTdsMlpGlsGl | 1774.1 | [M-3H]3- | 0.012 |
| 172 | 93 | 1480 | GlpMlpMlsTdsAdsGdsAdsCdsAdsGdsCdsGdsTdsMlpGlpGl | 1763.4 | [M-3H]3- | <0.3 |
| 173 | 93 | 1480 | GmsMmsMmsTdsAdsGdsAdsCdsAdsGdsCdsGdsTdsMlsGlsGl | 1862.3 | [M-3H]3- | 0.056 |
| 174 | 93 | 1480 | GlsMlsMlsTdsAdsGdsAdsCdsAdsGdsCdsGdsTdsMmsGmsGm | 1861.9 | [M-3H]3- | 0.019 |
| 175 | 4 | 1480 | GmsMmsMmsTesAdsGdsAdsCdsAdsGdsCdsGdsTdsMmsGmsGm | 1963.8 | [M-3H]3- | 0.090 |
| 176 | 4 | 1480 | GmsMmsMmsTdsAdsGdsAdsCdsAdsGdsCdsGdsTesMmsGmsGm | 1963.7 | [M-3H]3- | 0.019 |
| 177 | 4 | 1480 | GmsMmsMmsTesAesGdsAdsCdsAdsGdsCdsGdsTdsMmsGmsGm | 1988.4 | [M-3H]3- | <0.3 |
| 178 | 4 | 1480 | GmsMmsMmsTesAdsGdsAdsCdsAdsGdsCdsGdsTesMmsGmsGm | 1988.9 | [M-3H]3- | <0.3 |
| 179 | 4 | 1480 | GmsMmsMmsTdsAdsGdsAdsCdsAdsGdsCdsGesTesMmsGmsGm | 1987.8 | [M-3H]3- | <0.3 |
| 180 | 93 | 1480 | GesMesMmsTmsAdsGdsAdsCdsAdsGdsCdsGdsTmsMmsGesGe | 1968.2 | [M-3H]3- | <0.3 |
| 181 | 93 | 1480 | GmsMesMmsTesAdsGdsAdsCdsAdsGdsCdsGdsTesMmsGesGm | 1967.8 | [M-3H]3- | <0.3 |
| 182 | 93 | 1480 | GesMesMesTdsAdsGdsAdsCdsAdsGdsCdsGdsTdsMmsGmsGm | 1907.5 | [M-3H]3- | <0.3 |
| 183 | 93 | 1480 | GesMesMesTesAdsGdsAdsCdsAdsGdsCdsGdsTdsMmsGmsGm | 1932.8 | [M-3H]3- | <0.3 |
| 184 | 93 | 1480 | GesMesMesTesAesGdsAdsCdsAdsGdsCdsGdsTdsMmsGmsGm | 1956.7 | [M-3H]3- | <0.3 |
| 185 | 93 | 1480 | GmsMmsMmsTdsAdsGdsAdsCdsAdsGdsCdsGdsTdsMesGesGe | 1908.5 | [M-3H]3- | <0.3 |
| 186 | 93 | 1480 | GmsMmsMmsTdsAdsGdsAdsCdsAdsGdsCdsGdsTesMesGesGe | 1932.8 | [M-3H]3- | <0.3 |
| 187 | 93 | 1480 | GmsMmsMmsTdsAdsGdsAdsCdsAdsGdsCdsGesTesMesGesGe | 1957.8 | [M-3H]3- | <0.3 |
| 188 | 93 | 1480 | GlsMlsMlsTdsAesGdsAdsCdsAdsGdsCdsGdsTdsMlsGlsGl | 1809.6 | [M-3H]3- | 0.064 |
| 189 | 93 | 1480 | GlsMlsMlsTesAesGdsAdsCdsAdsGdsCdsGdsTdsMlsGlsGl | 1833.8 | [M-3H]3- | 0.161 |
| 190 | 93 | 1480 | GesMesMesTdsAdsGdsAdsCdsAdsGdsCdsGdsTdsMlsGlsGl | 1831.4 | [M-3H]3- | <0.3 |
| 191 | 93 | 1480 | GlsMlsMlsTdsAdsGdsAdsCdsAdsGdsCdsGdsTdsMesGesGe | 1831.0 | [M-3H]3- | 0.476 |
| 192 | 93 | 1480 | GlsMesMlsTesAdsGdsAdsCdsAdsGdsCdsGdsTesMlsGesGl | 1865.0 | [M-3H]3- | <0.3 |
| 193 | 93 | 1480 | GesMlsMesTlsAdsGdsAdsCdsAdsGdsCdsGdsTlsMesGlsGe | 1865.4 | [M-3H]3- | <0.3 |
| 194 | 93 | 1480 | GesMesMlsTlsAdsGdsAdsCdsAdsGdsCdsGdsTlsMlsGesGe | 1865.0 | [M-3H]3- | <0.3 |
| 195 | 93 | 1480 | GlsMlsMesTesAdsGdsAdsCdsAdsGdsCdsGdsTesMesGlsGl | 1865.3 | [M-3H]3- | <0.3 |

**[Table 3-2]**

| (Table 3 continued) | | | | | | |
|---|---|---|---|---|---|---|
| Compund No. | Sequ ence No. | Target Position | Sequence | m/z | Ion Species | IC50(uM) Gymnosis Method |
| 196 | 85 | 1477 | GmsMmsCdsTdsAdsGdsAdsCdsAdsGdsCdsGdsTdsCdsGdsGdsAdsAmsGm | 2194.2 | [M-3H]3- | <0.3 |
| 197 | 86 | 1478 | TmsGmsCdsCdsTdsAdsGdsAdsCdsAdsGdsCdsGdsTdsCdsGdsGdsAmsAm | 2181.4 | [M-3H]3- | <0.3 |
| 198 | 71 | 127 | GmsGmsTdsGdsGdsCdsGdsAdsTdsGdsCdsCdsCdsGdsGdsGdsTmsAm | 2096.4 | [M-3H]3- | 0.964 |
| 199 | 72 | 127 | GmsTmsGmsGdsCdsGdsAdsTdsGdsCdsCdsCdsGdsGdsGmsTmsAm | 2050.1 | [M-3H]3- | 0.786 |
| 200 | 76 | 128 | GmsGmsTmsGdsGdsCdsGdsAdsTdsGdsCdsCdsCdsGdsGmsGmsTm | 2055.7 | [M-3H]3- | 0.451 |
| 201 | 69 | 126 | GmsGmsMmsGdsAdsTdsGdsCdsCdsCdsGdsGdsGdsTmsAmsMm | 1940.1 | [M-3H]3- | 0.866 |
| 202 | 73 | 127 | TmsGmsGmsCdsGdsAdsTdsGdsCdsCdsCdsGdsGdsGmsTmsAm | 1936.3 | [M-3H]3- | 0.269 |
| 203 | 75 | 128 | GmsTmsGmsGosCdsGdsAdsTdsGdsCdsCdsCdsGdsGmsGmsTm | 1950.9 | [M-3H]3- | 0.300 |
| 204 | 75 | 128 | GmsTmsGmsGdsCosGdsAdsTdsGdsCdsCdsCdsGdsGmsGmsTm | 1950.9 | [M-3H]3- | 0.298 |
| 205 | 75 | 128 | GmsTmsGmsGdsCdsGosAdsTdsGdsCdsCdsCdsGdsGmsGmsTm | 1951.4 | [M-3H]3- | 0.370 |
| 206 | 75 | 128 | GmsTmsGmsGdsCdsGdsAosTdsGdsCdsCdsCdsGdsGmsGmsTm | 1951.6 | [M-3H]3- | 0.709 |
| 207 | 106 | 128 | GmsTmsGmsGdsCdsGdsAdsUosGdsCdsCdsCdsGdsGmsGmsTm | 1946.8 | [M-3H]3- | 0.326 |
| 208 | 75 | 128 | GmsTmsGmsGdsCdsGdsAdsTdsGosCdsCdsCdsGdsGmsGmsTm | 1951.4 | [M-3H]3- | 0.200 |
| 209 | 75 | 128 | GmsTmsGmsGdsCdsGdsAdsTdsGdsCosCdsCdsGdsGmsGmsTm | 1950.5 | [M-3H]3- | 0.611 |
| 210 | 75 | 128 | GmsTmsGmsGdsCdsGdsAdsTdsGdsCdsCosCdsGdsGmsGmsTm | 1463.0 | [M-4H]4- | 0.467 |
| 211 | 75 | 128 | GmsTmsGmsGdsCdsGdsAdsTdsGdsCdsCdsCosGdsGmsGmsTm | 1950.9 | [M-3H]3- | 0.345 |
| 212 | 75 | 128 | GmsTmsGmsGdsCdsGdsAdsTdsGdsCdsCdsCdsGosGmsGmsTm | 1951.2 | [M-3H]3- | 0.516 |
| 213 | 3 | 1309 | GmsMmsAmsGosTdsTdsCdsTdsCdsCdsGdsCdsGmsGmsTm | 1818.9 | [M-3H]3- | 0.074 |
| 214 | 107 | 1309 | GmsMmsAmsGdsUosTdsCdsTdsCdsCdsGdsCdsGmsGmsTm | 1814.6 | [M-3H]3- | 0.092 |
| 215 | 108 | 1309 | GmsMmsAmsGdsTdsUosCdsTdsCdsCdsGdsCdsGmsGmsTm | 1814.2 | [M-3H]3- | 0.237 |
| 216 | 109 | 1309 | GmsMmsAmsGdsTdsTdsCdsUosCdsCdsGdsCdsGmsGmsTm | 1814.2 | [M-3H]3- | 0.215 |
| 217 | 3 | 1309 | GmsMmsAmsGdsTdsTdsCdsTdsCosCdsGdsCdsGmsGmsTm | 1818.3 | [M-3H]3- | 0.225 |
| 218 | 3 | 1309 | GmsMmsAmsGdsTdsTdsCdsTdsCdsCosGdsCdsGmsGmsTm | 1818.4 | [M-3H]3- | 0.164 |
| 219 | 3 | 1309 | GmsMmsAmsGdsTdsTdsCdsTdsCdsCdsGosCdsGmsGmsTm | 1819.3 | [M-3H]3- | 0.082 |
| 220 | 3 | 1309 | GmsMmsAmsGdsTdsTdsCdsTdsCdsCdsGdsCosGmsGmsTm | 1818.8 | [M-3H]3- | 0.123 |
| 221 | 75 | 128 | GmsTmsGmsGdsCdsGdsAdsTdsGdsCdsCdsCdsGesGesGesTe | 1934.6 | [M-3H]3- | 0.279 |
| 222 | 96 | 128 | GmsTmsGmsGdsCdsGdsAdsTdsGdsCdsCdsMesGesGesGesTe | 1472.7 | [M-4H]4- | 0.285 |
| 223 | 75 | 128 | GmsTmsGmsGdsCdsGdsAdsTdsGdsCdsCdsCdsGmsGesGesTe | 1944.9 | [M-3H]3- | 0.527 |
| 224 | 3 | 1309 | GmsMmsAmsGdsTdsTdsCdsTdsCdsCdsGdsCdsGesGesTe | 1777.3 | [M-3H]3- | 0.737 |
| 225 | 27 | 1309 | GmsMmsAmsGdsTdsTdsCdsTdsCdsCdsGdsMesGesGesTe | 1807.2 | [M-3H]3- | 0.741 |
| 226 | 27 | 1309 | GmsMmsAmsGdsTdsTdsCdsTdsCdsCdsGdsMmsGesGesTe | 1818.1 | [M-3H]3- | 0.252 |
| 227 | 21 | 1308 | GmsMmsAmsGdsTdsTdsCdsTdsCdsCdsGdsCdsGmsGesTesGe | 1927.9 | [M-3H]3- | 0.205 |
| 228 | 16 | 1307 | GmsMmsAmsGdsTdsTdsCdsTdsCdsCdsGdsCdsGmsGmsTmsGesTe | 2069.3 | [M-3H]3- | 0.300 |
| 229 | 79 | 1304 | GmsMmsAmsGdsTdsTdsCdsTdsCdsCdsGdsCdsGmsGmsTmsGesTesGesGesAe | 1870.3 | [M-4H]4- | 0.570 |
| 230 | 78 | 232 | GmsAmsGmsAdsTdsTdsCdsCdsCdsGdsCdsMmsGmsGmsTesGe | 1975.8 | [M-3H]3- | 0.471 |
| 231 | 78 | 232 | GmsAmsGmsAdsTdsTdsCdsCdsCdsGdsCdsMmsGmsGesTesGe | 1965.6 | [M-3H]3- | 0.491 |
| 232 | 75 | 128 | GmsTmpGmsGdsCdsGdsAdsTdsGdsCdsCdsCdsGdsGmpGmsTm | 1930.1 | [M-3H]3- | 0.554 |
| 233 | 75 | 128 | GmpTmpGmsGdsCdsGdsAdsTdsGdsCdsCdsCdsGdsGmpGmpTm | 1439.4 | [M-4H]4- | 0.596 |
| 234 | 70 | 126 | GmsTmsGmsGdsCdsGdsAdsTdsGdsCdsCdsCdsGdsGdsGdsTmsAmsMm | 2156.7 | [M-3H]3- | 0.759 |
| 235 | 74 | 127 | MmsTmsGmsGdsTdsGdsGdsCdsGdsAdsTdsGdsCdsCdsCdsGdsGdsGmsTmsAm | 1784.3 | [M-4H]4- | 0.434 |
| 236 | 77 | 128 | TmsMmsTmsGdsGdsTdsGdsGdsCdsGdsAdsTdsGdsCdsCdsCdsGdsGmsGmsTm | 1781.7 | [M-4H]4- | 0.509 |
| 237 | 74 | 127 | MmsTmsGmsGdsTdsGosGdsCdsGdsAdsTdsGdsCdsCdsCdsGdsGdsGmsTmsAm | 1791.1 | [M-4H]4- | 0.262 |
| 238 | 74 | 127 | MmsTmsGmsGdsTdsGdsGosCdsGdsAdsTdsGdsCdsCdsCdsGdsGdsGmsTmsAm | 1791.1 | [M-4H]4- | 0.281 |
| 239 | 74 | 127 | MmsTmsGmsGdsTdsGdsGdsCosGdsAdsTdsGdsCdsCdsCdsGdsGdsGmsTmsAm | 1791.2 | [M-4H]4- | 0.377 |
| 240 | 74 | 127 | MmsTmsGmsGdsTdsGdsGdsCdsGosAdsTdsGdsCdsCdsCdsGdsGdsGmsTmsAm | 1791.2 | [M-4H]4- | 0.229 |
| 241 | 75 | 128 | GmsTmsGmsGdsCosGdsAdsTdsGdsCdsCdsCdsGesGesGesTe | 1943.8 | [M-3H]3- | 0.861 |
| 242 | 21 | 1308 | GmsMmsAmsGdsTdsTdsCdsTdsCdsCdsGdsCdsGdsGesTesGe | 1893.1 | [M-3H]3- | 0.529 |
| 243 | 21 | 1308 | GmsMmsAmsGdsTdsTdsCdsTdsCdsCdsGdsCdsGesGesTesGe | 1917.3 | [M-3H]3- | 0.379 |
| 244 | 74 | 127 | MmsTmsGmsGdsTdsGdsGdsCdsGdsAdsTdsGdsCdsCdsCdsGosGdsGmsTmsAm | 1791.2 | [M-4H]4- | 0.822 |
| 245 | 74 | 127 | MmsTmsGmsGdsTdsGdsGdsCdsGdsAdsTdsGdsCdsCdsCdsGdsGosGmsTmsAm | 1791.8 | [M-4H]4- | 0.812 |
| 246 | 94 | 1308 | GmsMmsAmsGdsTdsTdsCdsTdsCdsCdsGdsMesGesGesTesGe | 1947.0 | [M-3H]3- | 0.866 |
| 247 | 51 | 1474 | MlsAlsGlsCdsGdsTdsCdsGdsGdsAdsAdsGdsGlsTlsGl | 1686.8 | [M-3H]3- | >0.538 |
| 248 | 98 | 112 | AmsMmsGmsGdsGdsTdsTdsCdsCdsGdsCdsTdsCdsAmsAmsAm | 1436.6 | [M-4H]4- | >0.3 |
| 249 | 99 | 162 | MmsGmsGmsAdsAdsTdsGdsCdsCdsGdsAdsTdsGdsGmsMmsMm | 1454.4 | [M-4H]4- | >0.3 |
| 250 | 100 | 264 | TmsTmsMmsTdsGdsGdsCdsGdsGdsGdsCdsCdsGdsMmsGmsTm | 1450.1 | [M-4H]4- | >0.3 |
| 251 | 101 | 1273 | TmsAmsGmsAdsCdsCdsCdsCdsGdsCdsGdsTdsCdsMmsTmsAm | 1420.5 | [M-4H]4- | >0.3 |

### Example 9

### In vivo DUX4 knockdown activity test

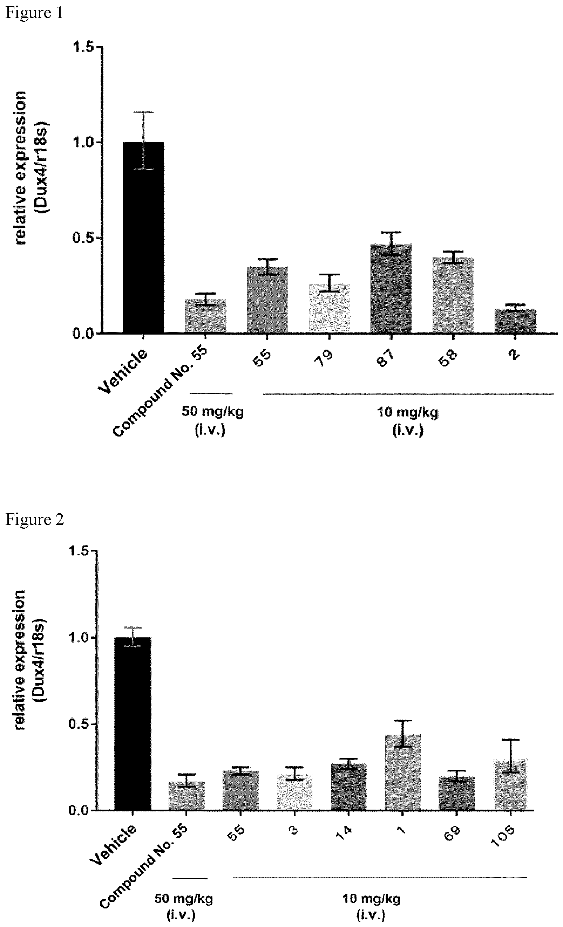

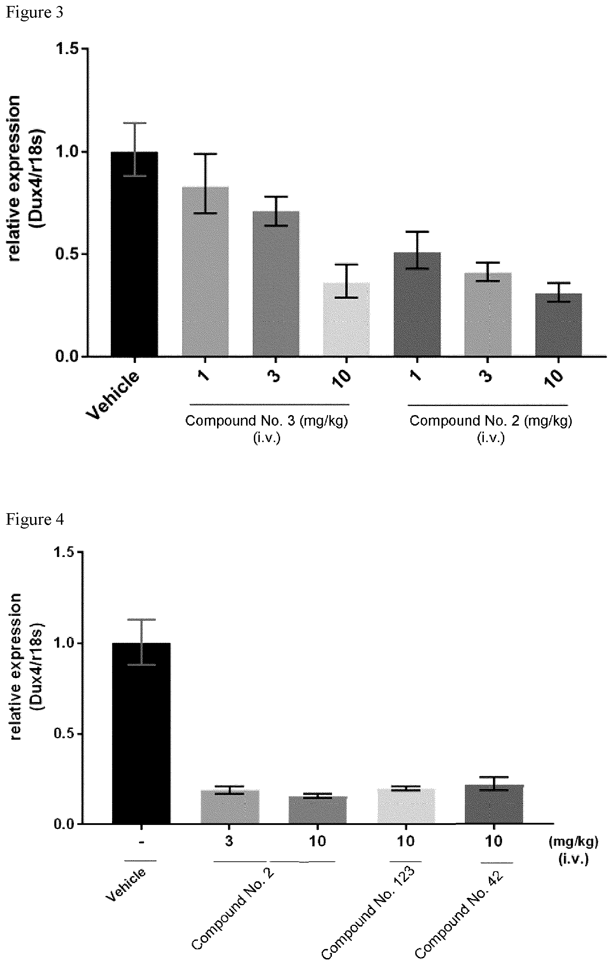

An adeno-associated virus vector AAV-DUX4 (SignaGen Laboratories, Cat. # SL100862) incorporating DUX4 mature mRNA of SEQ ID NO: 1 in the sequence listing was prepared. The AAV-DUX4 was intramuscularly administered at 1E+10 VG / 50 uL to the anterior tibialis muscle of 8-week-old C57BL/6J mice (male, Charles River Japan) under isoflurane (Pfizer Inc.) anesthesia. Three days later, modified oligonucleotides targeting DUX4 were prepared in physiological saline at 1, 3, 10 and 50 mg/(5 mL)/kg, and were administered to 8-week-old C57BL/6J mice (male, Charles River Japan) via the tail vein. 72 hours later, whole blood was collected from the abdominal vena cava under cervical dislocation or isoflurane (Pfizer Inc.) anesthesia, and the mice were sacrificed. After that, the tibialis anterior muscle was collected, immersed in RNAlater Soln (invitrogen), and frozen at -80 °C. A homogenization buffer of Maxwell RSC simplyRNA Tissue Kit (Promega) was added to the tissue, and the tissue was crushed using a multi-beads shocker, and RNA was purified according to the protocol described in the kit. 400 ng of RNA was reverse transcribed and quantitative PCR was performed using the obtained cDNA. Knockdown activity of a modified oligonucleotide was expressed as a quantitative ratio of DUX4 to 18S rRNA relative to a vehicle group. The results for 1, 3, 10 and 50 mg/(5 mL)/kg are shown in Figs. 1 - 4. Compound No. 1 (sequence complementary to positions 233 - 248 of DUX4 mature mRNA), Compound No. 2 (sequence complementary to positions 1309 - 1323 of DUX4 mature mRNA), Compound No. 3 (sequence complementary to positions 1480 - 1495 of DUX4 mature mRNA), Compound No. 13 (sequence complementary to positions 234 - 247 of DUX4 mature mRNA), Compound No. 41 (sequence complementary to positions 1308 - 1323 of DUX4 mature mRNA), Compound No. 54 (sequence complementary to positions 1309 - 1323 of DUX4 mature mRNA), Compound No. 57 (sequence complementary to positions 1309 - 1323 of DUX4 mature mRNA), Compound No. 68 (sequence complementary to positions 1309 - 1323 of DUX4 mature mRNA), Compound No. 78 (sequence complementary to positions 1309 - 1324 of DUX4 mature mRNA), Compound No. 88 (sequence complementary to positions 1310 - 1323 of DUX4 mature mRNA), Compound No. 104 (sequence complementary to positions 1473 - 1488 of DUX4 mature mRNA), Compound No. 122 (sequence complementary to positions 1480 - 1495 of DUX4 mature mRNA) were able to suppress expression of the DUX4 gene in muscle even when administered to a living body.

### Example 10

### Safety of modified oligonucleotide

When Compound Nos. 3, 42 and 123 were intravenously administered to 6-week-old ICR mice (male, Charles River Japan) at a maximum dose of 100 mg/kg, liver toxicity (increased ALT and AST in blood and histopathological abnormalities), renal toxicity (increased UN and creatinine in blood, and histopathological abnormalities), changes in general symptoms, death, and the like were not observed.

### Example 11

### In vivo Tg mouse DUX4 knockdown activity test

9-week-old male FLExDUX4-heteto/HSA-MCM-hetero: TG (DUX4-Tg) and FLExDUX4-wild/HSA-MCM-hetero: TG (MCM, control) were used (male, introduced to Charles River Japan from The Jackson Laboratory). A modified oligonucleotide targeting DUX4 was prepared in saline such that an administration liquid of each dose was 5 mL/kg, and was administered weekly via the tail vein. One week after 4-week administration, whole blood was collected from the abdominal vena cava under isoflurane anesthesia, and the mouse was euthanized. EDTA plasma was separated and was subjected to creatine kinase (CK) measurement. A muscle of a lower limb was collected, a wet weight was measured, and the muscle was subjected to gene expression analysis.

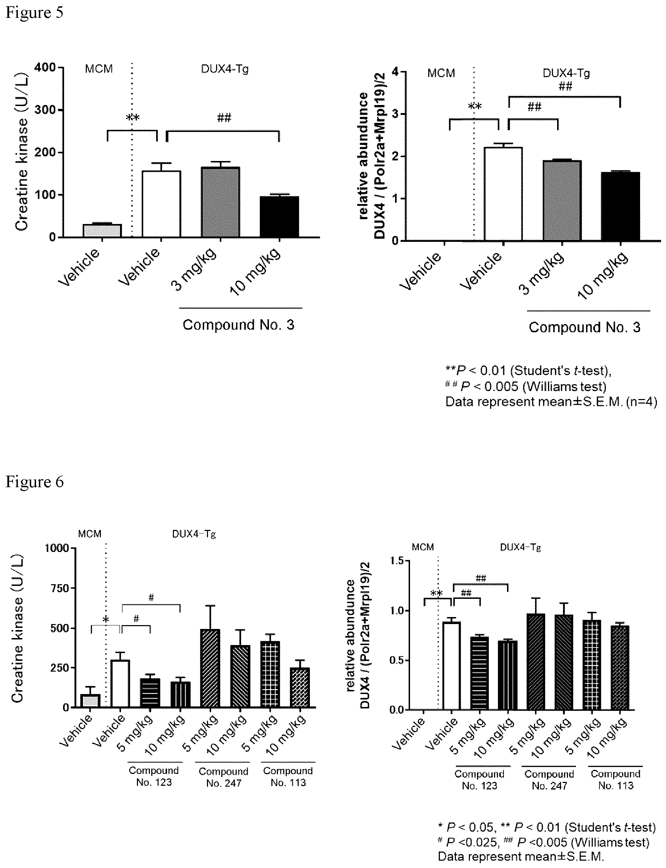

As shown in Figs. 5 and 6, Compound No. 3 and Compound No. 123 suppressed DUX4 mRNA expression. Further, a CK level in blood as a marker for myopathy was lowered. On the other hand, for Compound No 113 and Compound No 247, there was no clear effect on the DUX4 mRNA expression and the CK level in blood.

### Example 12

### Mouse continuous administration toxicity test

A modified oligonucleotide targeting DUX4 was prepared in saline at 100 mg/(5 mL)/kg, and was administered to a 6-week old ICR mouse (male, Charles River Japan) via the tail vein for four consecutive days. 72 hours after the last administration, blood was collected from the posterior vena cava under isoflurane anesthesia and was subjected to clinical biochemical testing. Further, after the mouse was euthanized by exsanguination, autopsy was performed, and the liver and the kidney were subjected to histopathological examination. For Compound No 123, after administration, there were no death and changes in general conditions, food consumption and body weight, and hepatotoxicity (increases in ALT and AST in serum, and histopathological abnormalities) and nephrotoxicity (increases in UN and creatinine in serum, and histopathological abnormalities) were not observed. On the other hand, for Compound No 113 and Compound No 247, after administration, although there were no death and changes in general conditions, food consumption and body weight, there were clear hepatotoxicity (increases in ALT, AST, GLDH, ALP, bilirubin and bile acid in serum, and histopathological abnormalities: degenerative necrosis of hepatocytes, and hepatocyte hypertrophy) and nephrotoxicity (increase in creatinine in serum) for both compounds. Further, concentrations of Compound No 123 in the liver and the kidney were respectively 323 and 251 µg/g, and there were no hepatotoxicity and nephrotoxicity, despite that the concentrations of Compound No 123 in the tissues were comparable to or higher than concentrations of Compound No 247 of 111 and 185 µg/g in the liver and the kidney and concentrations of Compound No 113 of 39.3 and 235 µg/g in the liver and the kidney.

**[Table 4]**

| Mouse continuous administration test blood biochemical examination | | | | | |
|---|---|---|---|---|---|
| Examination Item | Control Group | Compound No. 123 | Control Group | Compound No. 247 | Compound No. 113 |
| AST (U/L) | 53.8 | 60.2 | 38.0 | 1187.0 | 1355.8 |
| ALT (U/L) | 30.4 | 38.6 | 17.6 | 2300.2 | 1282.8 |
| GLDH (U/L) | 16.0 | 36.0 | 7.0 | 813.2 | 768.8 |
| ALP (U/L) | 340.2 | 345.2 | 204.2 | 1063.8 | 640.6 |
| T-Bil (mg/dL) | 0.086 | 0.052 | 0.106 | 1.208 | 0.298 |
| D-Bil (mg/dL) | 0.024 | 0.012 | 0.044 | 1.086 | 0.238 |
| I-Bil (mg/dL) | 0.062 | 0.040 | 0.062 | 0.122 | 0.060 |
| TBA (µmol/L) | 0.80 | 1.60 | 0.90 | 28.02 | 63.32 |
| UN (mg/dL) | 20.34 | 18.62 | 18.32 | 23.02 | 23.20 |
| Cre (mg/dL) | 0.056 | 0.054 | 0.062 | 0.126 | 0.152 |

**[Table 5]**

| Mouse Continuous Administration Test Pathological Findings | | | | | |
|---|---|---|---|---|---|
| Pathological Findings | Control Group | Compound No. 123 | Control Group | Compound No. 247 | Compound No. 113 |
| Degenerative necrosis of hepatocytes | 0/5 | 0/5 | 0/5 | 3/5 | 4/5 |
| Hepatocyte hypertrophy | 0/5 | 0/5 | 0/5 | 4/5 | 5/5 |

### Reference Example

Schemes of methods for synthesizing ALNA [Ms]-containing nucleotides, ALNA [mU]-containing nucleotides, ALNA [ipU]-containing nucleotides, ALNA [Trz]-containing nucleotides, ALNA [Oxz]-containing nucleotides are shown below. The starting compounds (1a, 1d, and 1g) can be synthesized using a method described in WO 2017/047816.

### Synthesis of ALNA [Ms]-T

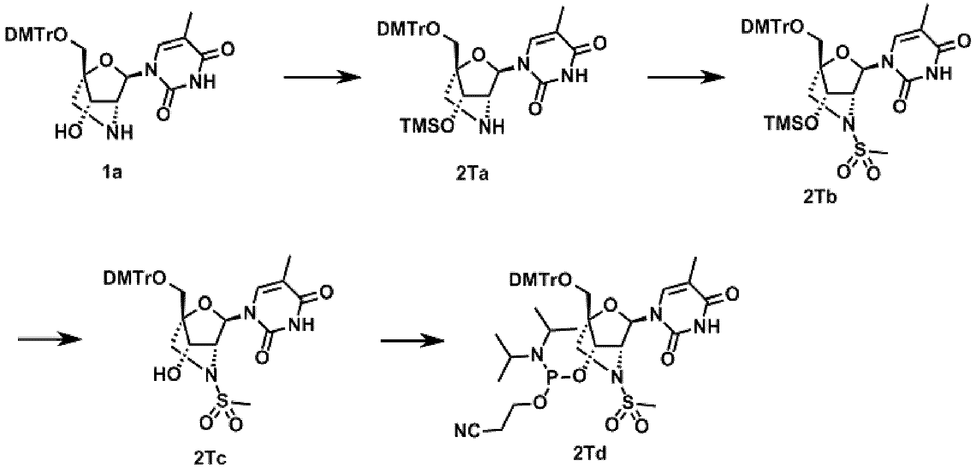

### Synthesis of ALNA [Ms]-mC

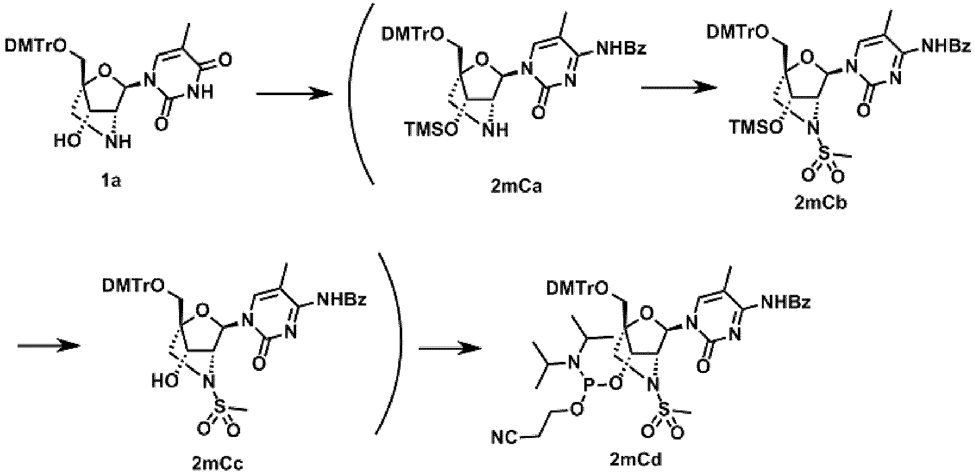

### Synthesis of ALNA [Ms]-G

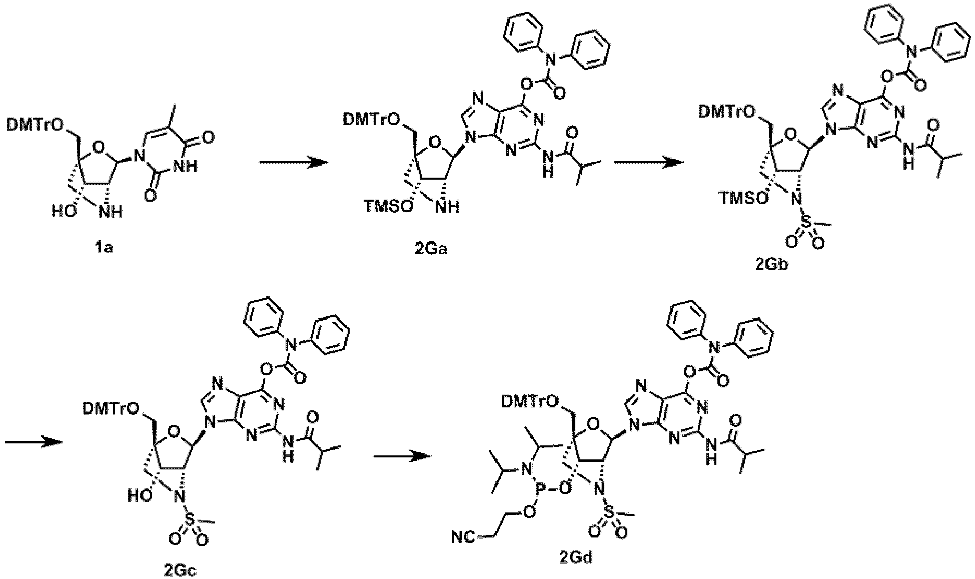

### Synthesis of ALNA [Ms]-A

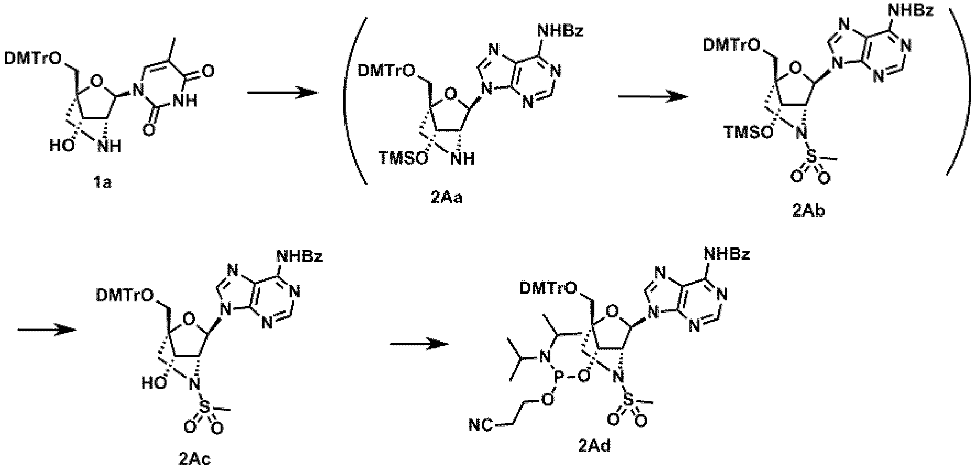

### Synthesis of ALNA [mU]-T

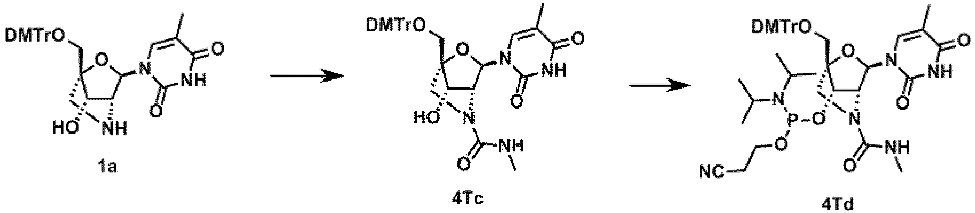

### Synthesis of ALNA [mU]-mC

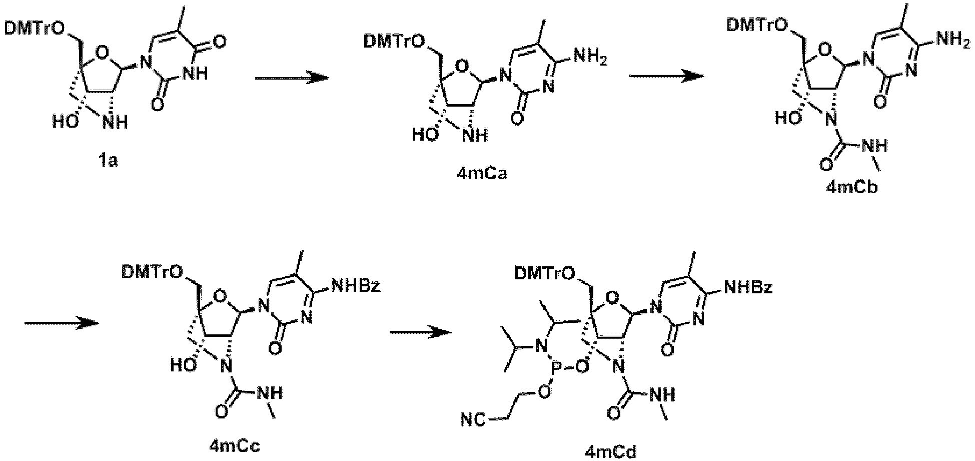

### Synthesis of ALNA [mU]-G

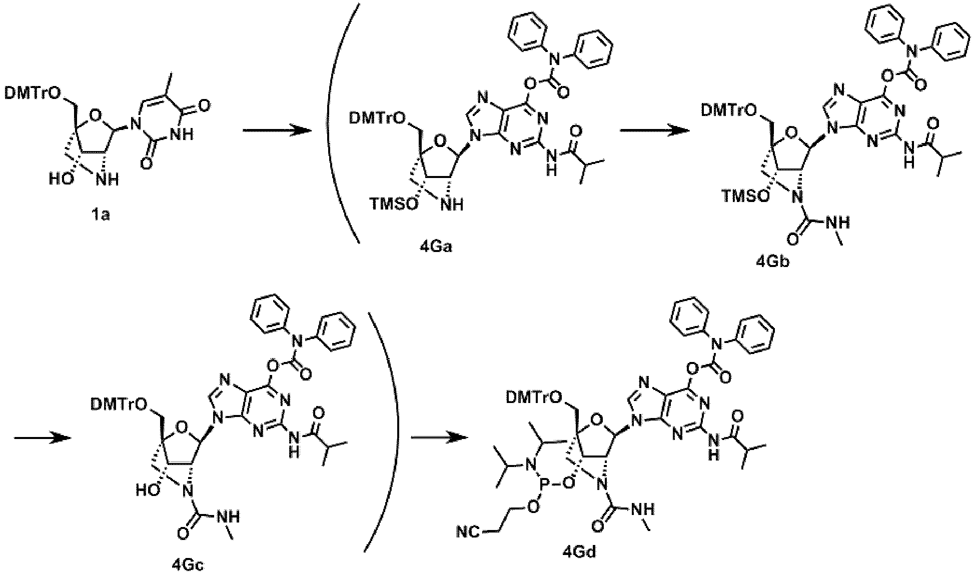

### Synthesis of ALNA [mU]-A

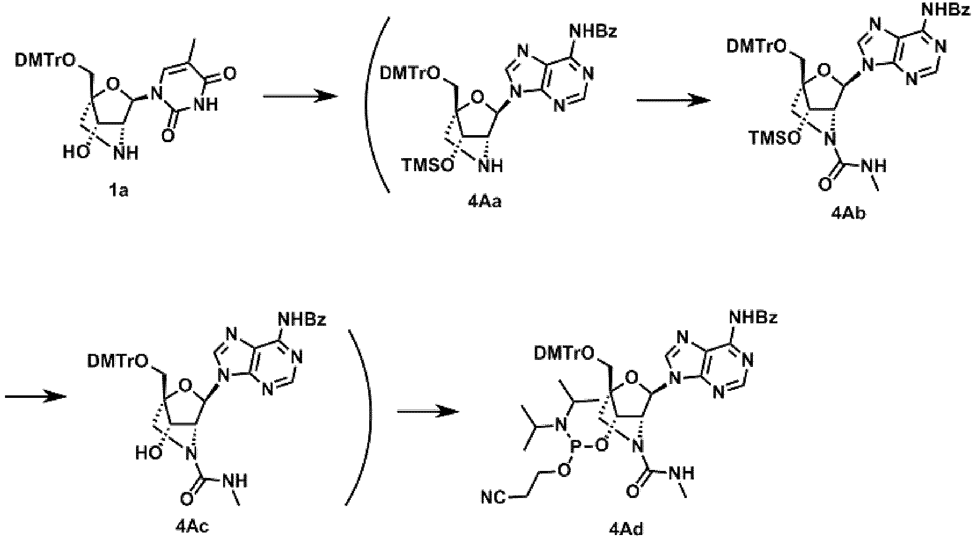

### Synthesis of ALNA [ipU]-T

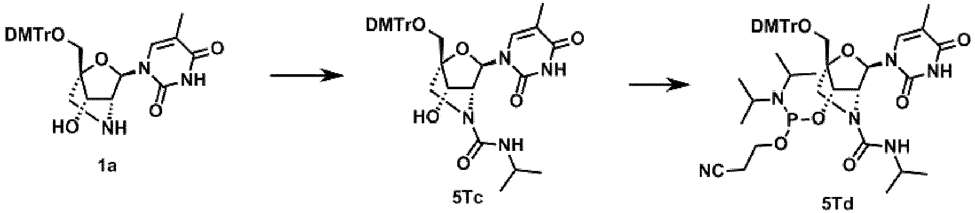

### Synthesis of ALNA [ipU]-mC

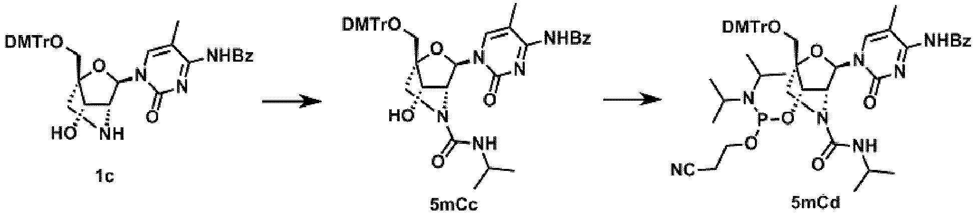

### Synthesis ALNA [ipU]-G

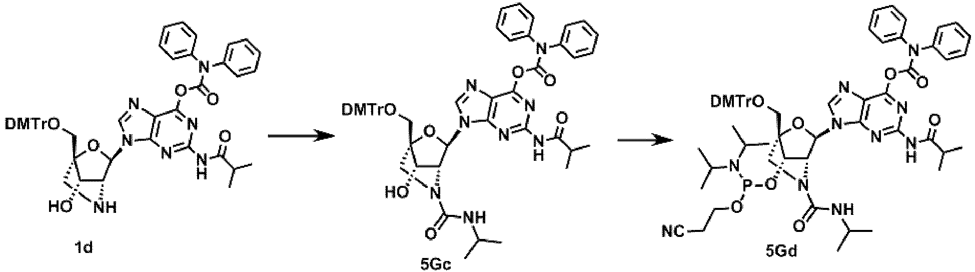

### Synthesis of ALNA [ipU]-A

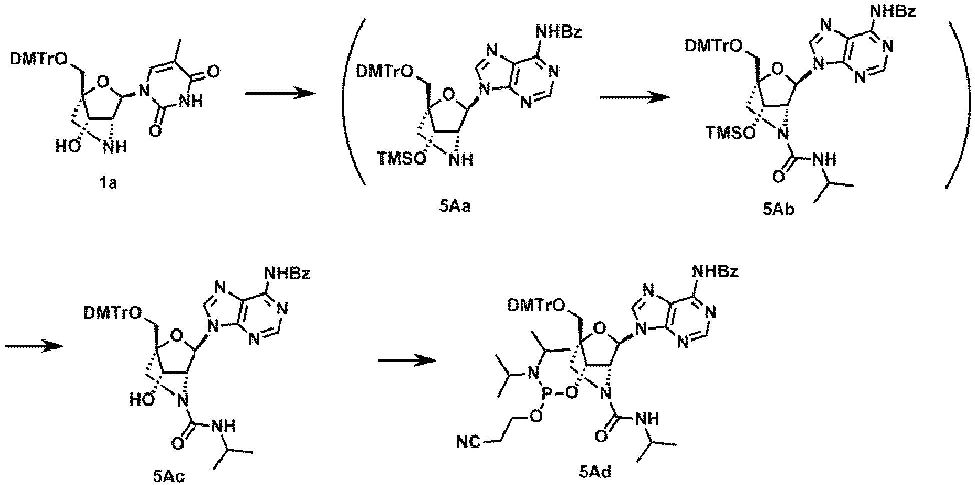

### Synthesis of ALNA [Trz]-T

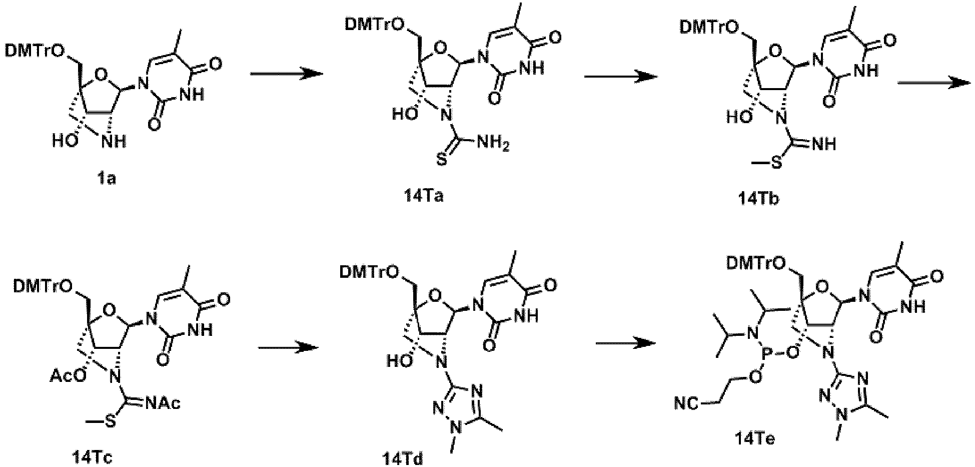

### Synthesis of ALNA [Trz]-mC

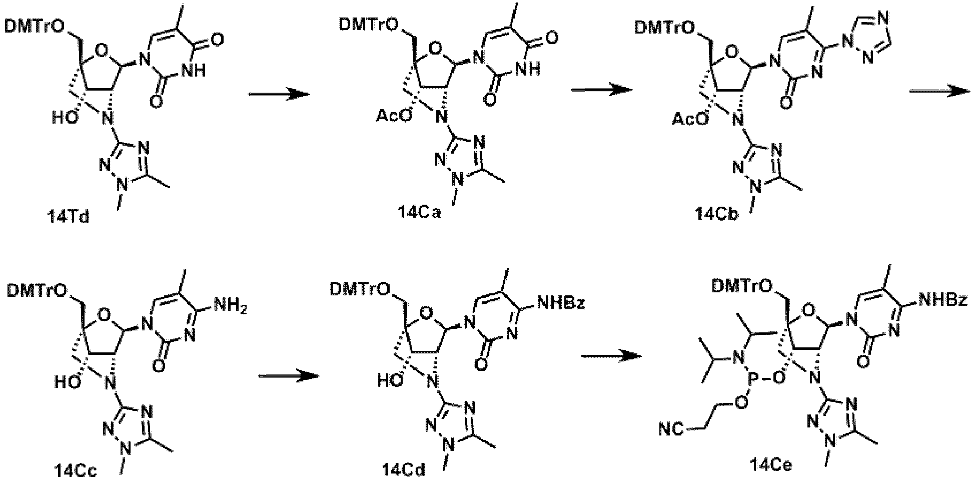

### Synthesis ALNA [Trz]-G

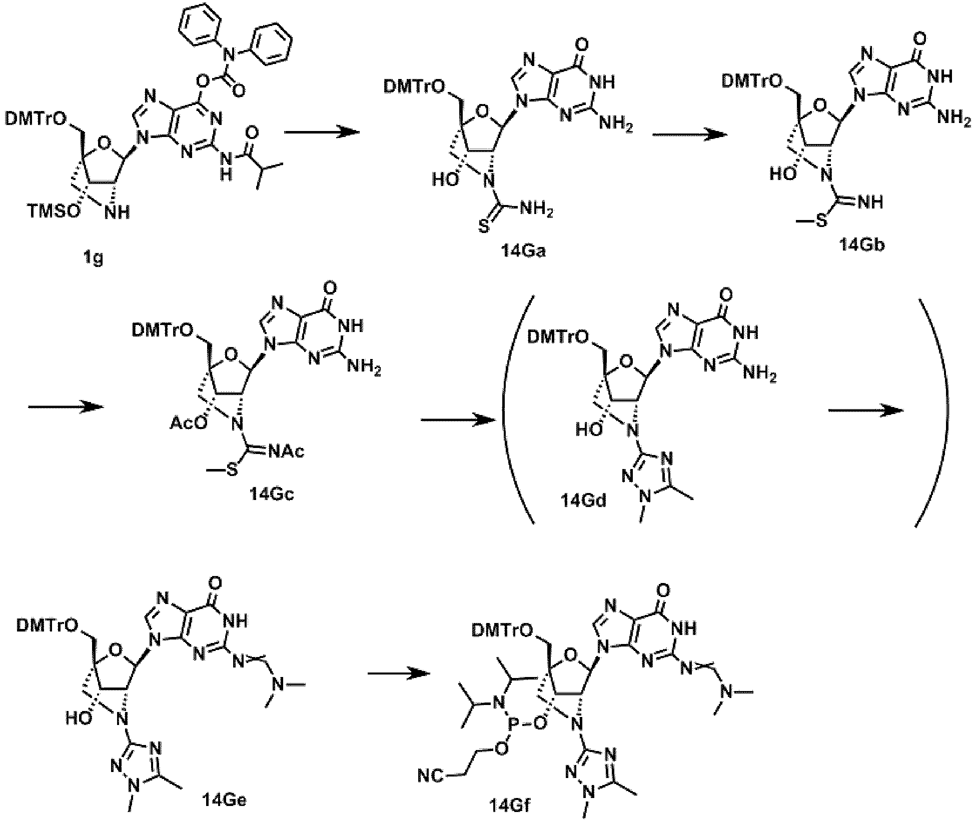

### Synthesis of ALNA [Trz]-A

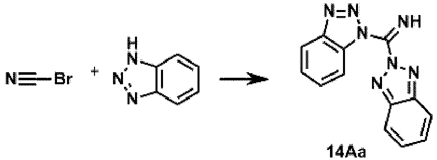

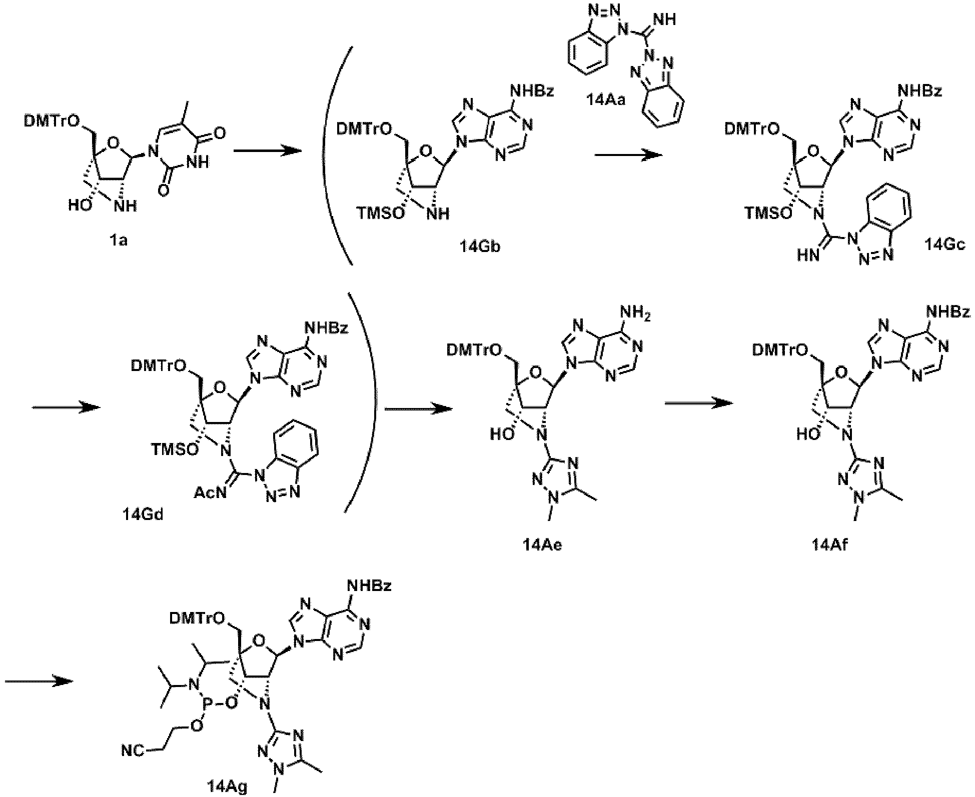

### Synthesis of ALNA [Oxz]-T

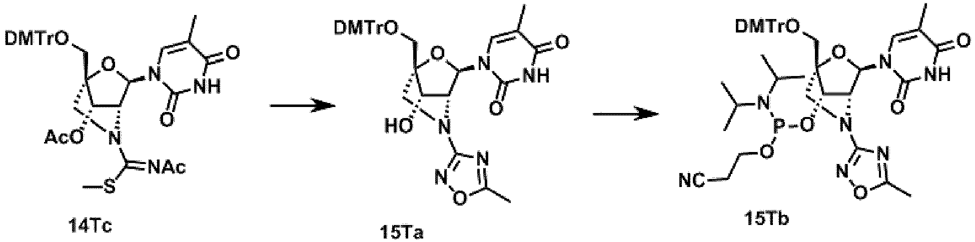

### Synthesis of ALNA [Oxz]-mC

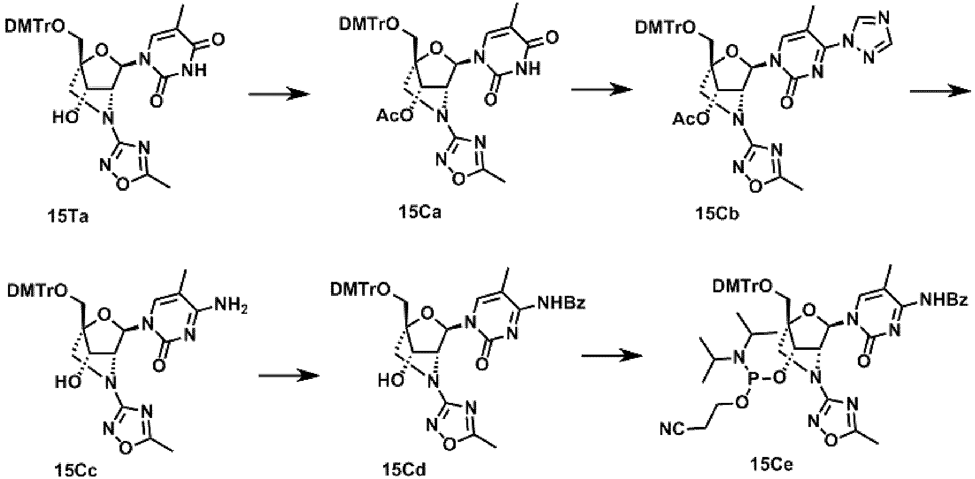

### [Industrial Applicability]

A modified oligonucleotide of the present invention can be used as a compound useful for therapeutically treating, preventing or delaying progress of a DUX4-related disease.

### [Sequence Listing Free Text]

SEQ ID NO: 1 in the sequence listing shows a base sequence of DUX4 mature mRNA.
SEQ ID NOs. 2 - 4 and 7 - 109 in the sequence listing show base sequences of modified oligonucleotides.
SEQ ID NOs: 5 - 6 in the sequence listing respectively show base sequences of DUX4-FL2 and DUX4-s as splicing variants of SEQ ID NO: 1.

## Claims

1. A modified oligonucleotide consisting 12 - 30 residues, comprising:
a nucleobase sequence that includes at least 8 contiguous nucleobase sequences and is complementary to an equal length portion at positions 126 - 147, 232 - 248, 1306 - 1325 or 1480 - 1495 from a 5' end of a nucleobase sequence of a mature mRNA of DUX4 of SEQ ID NO: 1,
wherein the nucleobase sequence of the modified oligonucleotide has at least 90% complementarity to the equal length portion in the nucleobase sequence of the mature mRNA of DUX4 of SEQ ID NO: 1, and when the at least 8 contiguous nucleobase sequences include a nucleobase sequence that is complementary to the equal length portion at the positions 1480 - 1495 from the 5' end of the nucleobase sequence of SEQ ID NO: 1, the modified oligonucleotide consists of a nucleobase sequence having at a 3' end a complementary base of a base of the position 1480 from the 5' end of the nucleobase of SEQ ID NO: 1.

2. The modified oligonucleotide according to claim 1, wherein one or more modified nucleotides of the modified oligonucleotide each include a modified sugar.

3. The modified oligonucleotide according to claim 2, wherein the modified sugar is selected from a group consisting of a bicyclic sugar, a 2'-O-methoxyethyl modified sugar, and a 2'-O-methyl modified sugar.

4. The modified oligonucleotide according to claim 3, wherein the bicyclic sugar is selected from a group consisting of LNA, GuNA, ALNA [Ms], ALNA [mU], ALNA [ipU], ALNA [Oxz], and ALNA [Trz].

5. A modified oligonucleotide consisting of 12-30 residues, comprising:
a nucleobase sequence that includes at least 8 contiguous nucleobase sequences and is complementary to an equal length portion at positions 1472 - 1495 from a 5' end of a nucleobase sequence of a mature mRNA of DUX4 of SEQ ID NO: 1,
wherein the nucleobase sequence of the modified oligonucleotide has at least 90% complementarity to the equal length portion in the nucleobase sequence of the mature mRNA of DUX4 of SEQ ID NO: 1, and the modified oligonucleotide includes at least one nucleoside that includes a modified sugar selected from the group consisting of GuNA, ALNA [Ms], ALNA [mU], ALNA [ipU], ALNA [Oxz], and ALNA [Trz].

6. The modified oligonucleotide according to claim 5, further comprising:
a 2'-O-methoxyethyl modified sugar and/or a 2'-O-methyl modified sugar.

7. The modified oligonucleotide according to any one of claims 1 - 6, wherein at least one modified nucleotide of the modified oligonucleotide includes a modified nucleobase.

8. The modified oligonucleotide according to claim 7, wherein the modified nucleobase is a 5-methylcytosine.

9. The modified oligonucleotide according to any one of claims 1 - 8, wherein at least one internucleoside linkage is a modified internucleoside linkage.

10. The modified oligonucleotide according to claim 9, wherein the modified internucleoside linkage is a phosphorothioate internucleoside linkage.

11. The modified oligonucleotide according to any one of claims 1 - 10, comprising:
1) a gap segment;
2) a 5' wing segment; and
3) a 3' wing segment,
wherein the gap segment is positioned between the 5' wing segment and the 3' wing segment, all nucleosides of the 5' wing segment and the 3' wing segment each include at least one modified sugar, and the gap segment includes only nucleosides that contain no modified sugar, or includes one or two nucleosides that each contain a modified sugar, and includes other nucleosides that contain no modified sugar.

12. The modified oligonucleotide according to any one of claims 1 - 11, consisting of:
a nucleobase sequence that is complementary to a nucleobase sequence of positions 128 - 143 from the 5' end of the nucleobase sequence, a nucleobase sequence of positions 232 - 247 from the 5 'end, a nucleobase sequence of positions 233 - 248 from the 5' end, a nucleobase sequence of positions 1309 - 1323 from the 5' end, or a nucleobase sequence of positions 1480 - 1495 from the 5' end of the mature mRNA of DUX4 of SEQ ID NO: 1.

13. The modified oligonucleotide according to any one of claims 1 - 12, consisting of:
a base sequence of gtggcgatgc ccgggt (SEQ ID NO: 75), gagattcccg cnggtg (SEQ ID NO: 78: n represents a 5-methylcytosine), ngagattcccgccggt (SEQ ID NO: 2: n represents a 5-methylcytosine), gnagttctccgcggt (SEQ ID NO: 3: n represents a 5-methylcytosine), or gnntagacagcgtngg (SEQ ID NO: 4: n represents a 5-methylcytosine).

14. The modified oligonucleotide according to claim 13 represented by the following formula,
GlsMlsMlsTdsAdsGdsAdsCdsAdsGdsCdsGdsTdsMlsGlsGl,
wherein nucleobases are represented by the following symbols: A = adenine, T = thymine, G = guanine, C = cytosine, and M = 5-methylcytosine; sugar moieties are represented by the following symbols: l = LNA, and d = 2'-deoxyribose; and internucleoside linkages are represented according to the following symbol: s = phosphorothioate.

15. The modified oligonucleotide according to claim 13 represented by the following formula,
GmsMmsMmsTdsAdsGdsAdsCdsAdsGdsCdsGdsTdsMmsGmsGm,
wherein nucleobases are represented by the following symbols: A = adenine, T = thymine, G = guanine, C = cytosine, and M = 5-methylcytosine; sugar moieties are represented by the following symbols: m = ALNA [Ms], and d = 2'-deoxyribose; and internucleoside linkages are represented by the following symbol: s = phosphorothioate.

16. The modified oligonucleotide according to claim 13 represented by the following formula,
GmsMmsAmsGdsTdsTdsCdsTdsCdsCdsGdsCdsGmsGmsTm,
wherein nucleobases are represented by the following symbols: A = adenine, T = thymine, G = guanine, C = cytosine, and M = 5-methylcytosine; sugar moieties are represented by the following symbols: m = ALNA [Ms], and d = 2'-deoxyribose; and internucleoside linkages are represented by the following symbol: s = phosphorothioate.

17. The modified oligonucleotide according to claim 13 represented by the following formula,
MlsGlsAlsGdsAdsTdsTdsCdsCdsCdsGdsCdsCdsGlsGlsTl,
wherein nucleobases are represented by the following symbols: A = adenine, T = thymine, G = guanine, C = cytosine, and M = 5-methylcytosine; sugar moieties are represented by the following symbols: l = LNA, and d = 2'-deoxyribose; and internucleoside linkages are represented by the following symbol: s = phosphorothioate.

18. The modified oligonucleotide according to claim 14 represented by the following formula or a salt thereof,

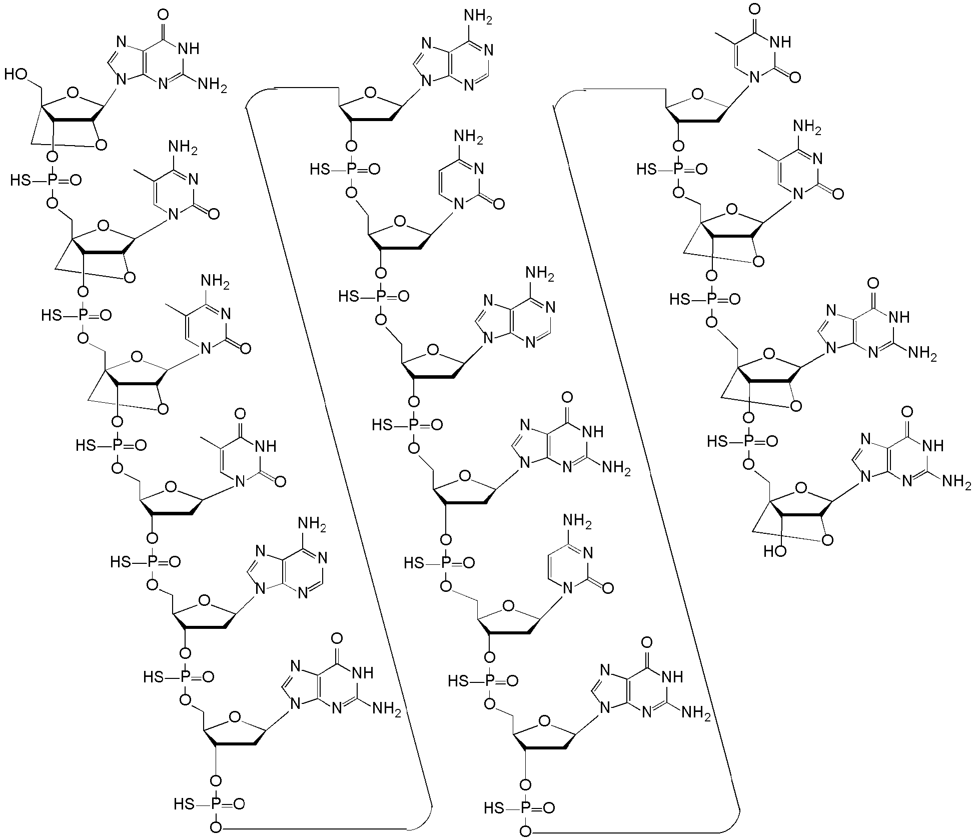

19. The modified oligonucleotide according to claim 15 represented by the following formula or a salt thereof,

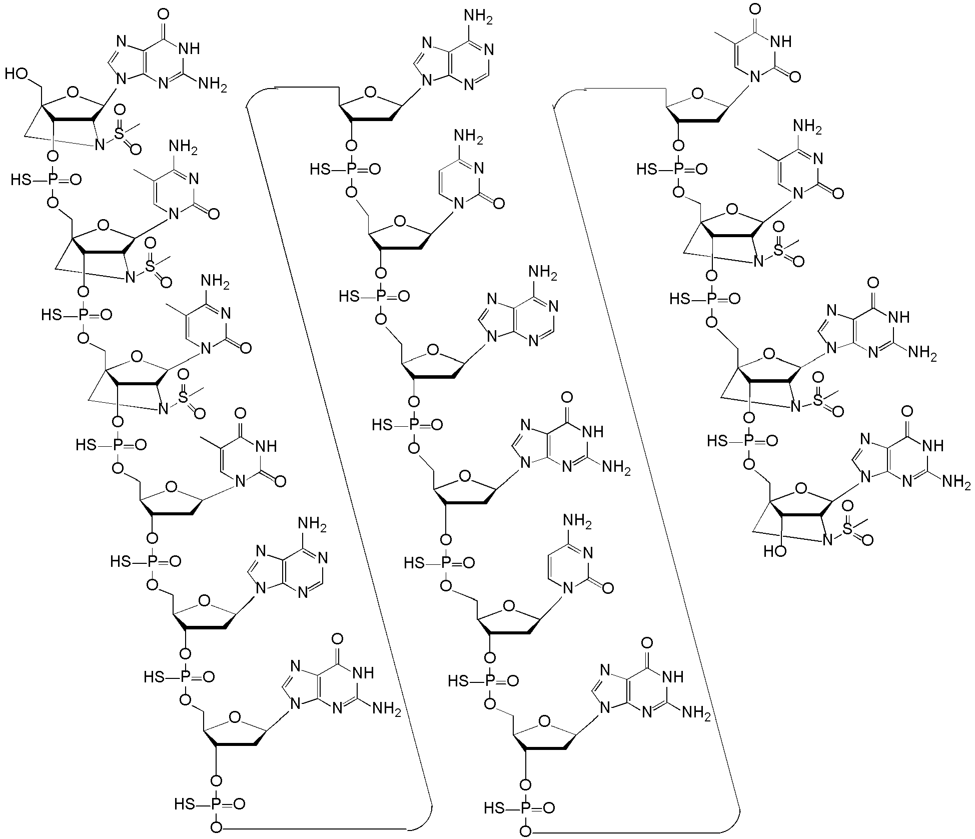

20. The modified oligonucleotide according to claim 16 represented by the following formula or a salt thereof,

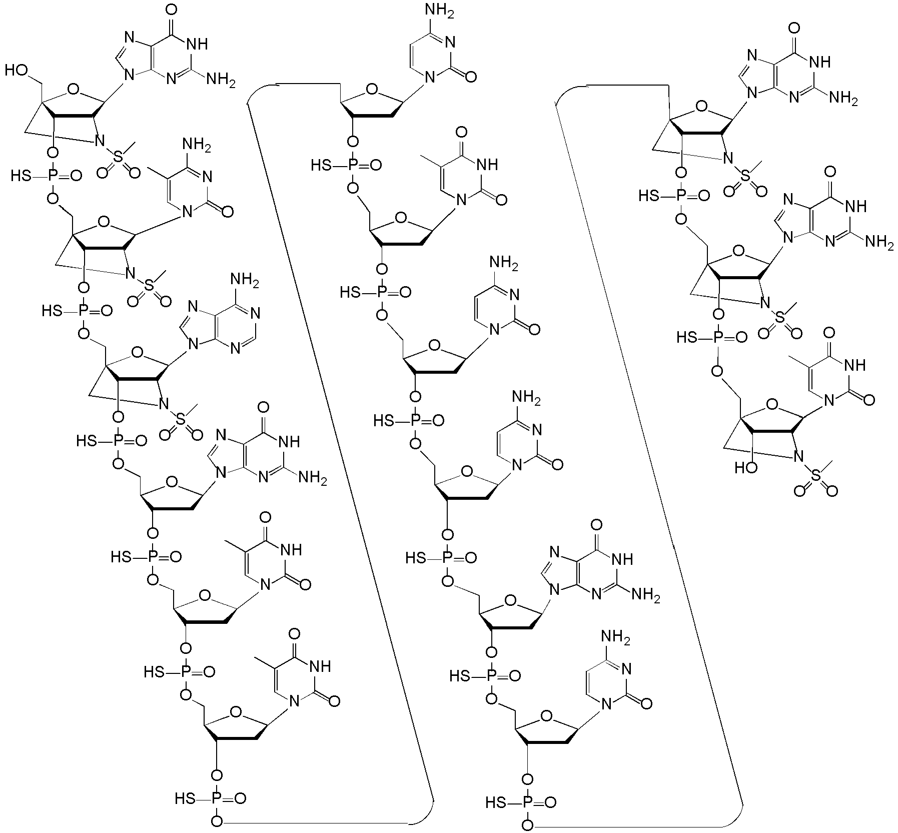

21. The modified oligonucleotide according to claim 17 represented by the following formula or a salt thereof,

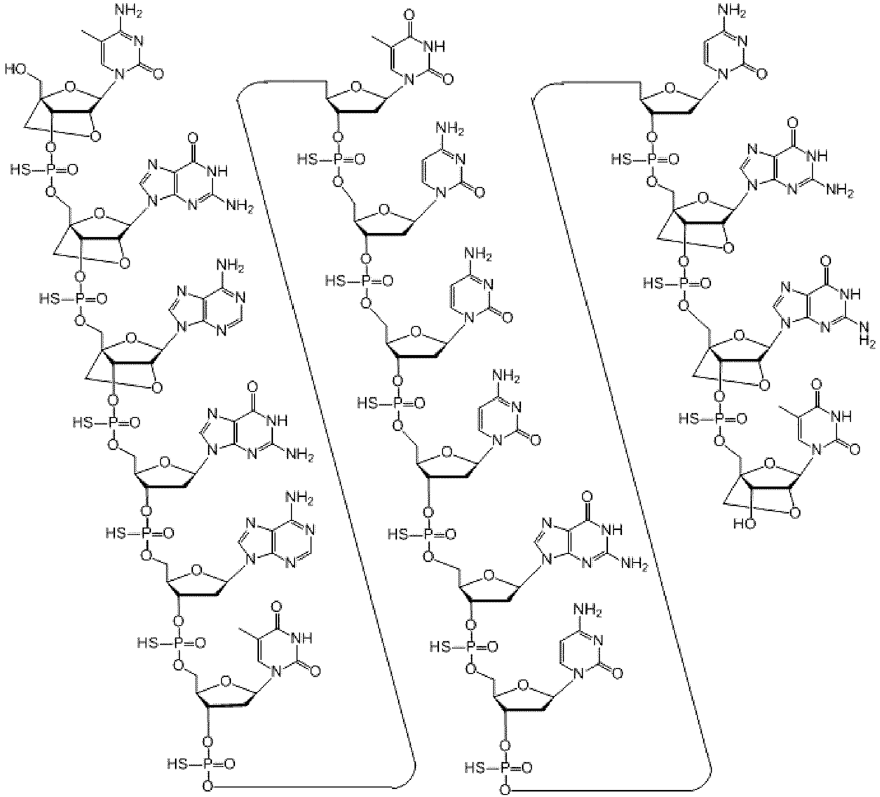

22. A pharmaceutical composition, comprising:
the modified oligonucleotide according to any one of claims 1 - 21 or a pharmaceutically acceptable salt thereof; and
a pharmaceutically acceptable carrier.

23. The pharmaceutical composition according to claim 22, for therapeutically treating, preventing, or delaying progress of a DUX4-related disease.

24. The pharmaceutical composition according to claim 23, wherein the DUX4-related disease is facioscapulohumeral muscular dystrophy.

25. A method for therapeutically treating, preventing or delaying progress of a DUX4-related disease in a subject, comprising: administering an effective amount of the modified oligonucleotide according to any one of claims 1 - 21 to a subject in need thereof.

26. Use of the modified oligonucleotide according to any one of claims 1 - 21, in manufacture of a medicament for therapeutically treating, preventing or delaying progress of a DUX4-related disease.

27. Use of the modified oligonucleotide according to any one of claims 1 - 21, for therapeutically treating, preventing or delaying progress of a DUX4-related disease.
